# EUROPEAN PATENT SPECIFICATION

(11) **EP 3 986 403 B1**
(45) Date of publication and mention of the grant of the patent: **16.09.2026**
(21) Application number: 20737778.9
(22) Date of filing: 19.06.2020
(51) Int. Cl.: A61K 31/437, A61K 31/706, A61P 35/02, A61K 31/404, A61L 2/00, A61L 2/16

(54) **AZACITIDINE IN COMBINATION WITH VENETOCLAX FOR TREATING LEUKEMIA OR MYELODYSPLASTIC SYNDROME**
AZACITIDINE ZUSAMMEN MIT VENETOCLAX ZUR BEHANDLUNG VON LEUKEMIA ODER MYELODYSPLASTISCHE SYNDROME
AZACITIDINE EN COMBINAISON AVEC VENETOCLAX POUR TRAITER LEUKEMIE OU LE SYNDROME MYELODYSPLASTIQUE

(30) Priority: 20.06.2019 US 201962864413 P
(43) Date of publication of application: 27.04.2022
(73) Proprietor: Celgene Corporation, Princeton, NJ 08543 (US)
(72) Inventor: FRATTINI, Mark, Summit, New Jersey 07901 (US); BEACH, Cl, Summit, New Jersey 07901 (US); ROSE, Shelonitda, Summit, New Jersey 07901 (US); DUNSHEE, Diana R., Summit, New Jersey 07901 (US); LOPES DE MENEZES, Daniel E., Summit, New Jersey 07901 (US); MACBETH, Kyle, Summit, New Jersey 07901 (US); DAI, Yumin, Summit, New Jersey 07901 (US); JANG, Jessica, Summit, New Jersey 07901 (US)
(74) Representative: Carpmaels & Ransford LLP
(86) International application number: PCT/US2020/038760
(87) International publication number: WO 2020/257665

(56) References cited:
- WO-A1-2017/157813
- WO-A1-2019/012328
- WO-A1-2020/127503
- US-A1- 2009 286 752
- POLLYEA DANIEL A ET AL: "Venetoclax with azacitidine disrupts energy metabolism and targets leukemia stem cells in patients with acute myeloid leukemia", NATURE MEDICINE, NATURE PUBLISHING GROUP US, NEW YORK, vol. 24, no. 12, 12 November 2018 (2018-11-12), pages 1859 - 1866, XP036653577, ISSN: 1078-8956, [retrieved on 20181112], DOI: 10.1038/S41591-018-0233-1
- DINARDO COURTNEY D ET AL: "Mutant Isocitrate Dehydrogenase (mIDH) Inhibitors, Enasidenib or Ivosidenib, in Combination with Azacitidine (AZA): Preliminary Results of a Phase 1b/2 Study in Patients with Newly Diagnosed Acute Myeloid Leukemia (AML)", vol. 130, no. Suppl. 1, 7 December 2017 (2017-12-07), pages 639, XP009522609, ISSN: 0006-4971, Retrieved from the Internet <URL:https://ashpublications.org/blood/article/130/Supplement%201/639/83406/Mutant-Isocitrate-Dehydrogenase-mIDH-Inhibitors>
- SWAMINATHAN MAHESH ET AL: "The Combination of Quizartinib with Azacitidine or Low Dose Cytarabine Is Highly Active in Patients (Pts) with FLT3-ITD Mutated Myeloid Leukemias: Interim Report of a Phase I/II Trial", vol. 130, no. Suppl. 1, 7 December 2017 (2017-12-07), pages 723, XP009522608, ISSN: 0006-4971, Retrieved from the Internet <URL:https://ashpublications.org/blood/article/130/Supplement%201/723/83424/The-Combination-of-Quizartinib-with-Azacitidine-or>
- ABOUDALLE IMAN ET AL: "Phase I-II Study of Crenolanib Combined with Standard Salvage Chemotherapy and Crenolanib Combined with 5-Azacitidine in Acute Myeloid Leukemia Patients with FLT3 Activating Mutations", vol. 132, no. Suppl. 1, 29 November 2018 (2018-11-29), pages 2715, XP009522607, ISSN: 0006-4971, Retrieved from the Internet <URL:https://doi.org/10.1182/blood-2018-99-117632?nosfx=y> DOI: 10.1182/BLOOD-2018-99-117632
- ESTEVE JORDI ET AL: "Multicenter, Open-Label, 3-Arm Study of Gilteritinib, Gilteritinib Plus Azacitidine, or Azacitidine Alone in Newly Diagnosed FLT3 Mutated (FLT3(mut+)) Acute Myeloid Leukemia (AML) Patients Ineligible for Intensive Induction Chemotherapy: Findings from the Safety Cohort", vol. 132, no. Suppl. 1, 29 November 2018 (2018-11-29), pages 2736, XP009522606, ISSN: 0006-4971, Retrieved from the Internet <URL:https://doi.org/10.1182/blood-2018-99-110976?nosfx=y> DOI: 10.1182/BLOOD-2018-99-110976

## Description

### RELATED APPLICATIONS

This application claims the benefits of priority of U.S. provisional application 62/864,413 filed June 20, 2019.

### FIELD

Provided are a (i) pharmaceutical composition comprising 5-azacytidine, and (ii) at least one additional therapeutic agent for use in a method of treating acute myeloid leukemia (AML) and/or myelodysplastic syndromes (MDS) in a human according to the claims.

### BACKGROUND

Cancer is a major worldwide public health problem with many types of cancer that have been described in the medical literature. Examples include cancer of the blood, bone, lung (*e.g*., non-small-cell lung cancer and small-cell lung cancer), colon, breast, prostate, ovary, brain, and intestine. The incidence of cancer continues to climb as the general population ages and as new forms of cancer develop. A continuing need exists for effective therapies to treat cancer patients.

Myelodysplastic syndromes (MDS) refers to a diverse group of hematopoietic stem cell disorders. The annual incidence of MDS is estimated to be 4.9 cases per 100,000 people worldwide, and approximately 10,000 people in the United States are diagnosed with MDS each year. MDS may be characterized by a cellular marrow with impaired morphology and maturation (dysmyelopoiesis), peripheral blood cytopenias, and a variable risk of progression to acute leukemia, resulting from ineffective blood cell production.

MDS are grouped together because of the presence of dysplastic changes in one or more of the hematopoietic lineages including dysplastic changes in the myeloid, erythroid, and megakaryocytic series. These changes result in cytopenias in one or more of the three lineages. Patients afflicted with MDS may develop complications related to anemia, neutropenia (infections), and/or thrombocytopenia (bleeding). About 10% to about 70% of patients with MDS may develop acute leukemia. In the early stages of MDS, the main cause of cytopenias is increased programmed cell death (apoptosis). As the disease progresses and converts into leukemia, a proliferation of leukemic cells overwhelms the healthy marrow. The disease course differs, with some cases behaving as an indolent disease and others behaving aggressively with a very short clinical course that converts into an acute form of leukemia. The majority of people with higher risk MDS eventually experience bone marrow failure. Up to 50% of MDS patients succumb to complications, such as infection or bleeding, before progressing to AML.

Acute myeloid leukemia (AML) is a type of cancer that affects the bone marrow and blood. AML is known by a variety of names, including acute myelogenous leukemia, acute myeloblastic leukemia, acute granulocytic leukemia, and acute nonlymphocytytic leukemia. The word "acute" in acute myelogenous leukemia reflects the disease's rapid progression. It is called acute myeloid leukemia because it affects a group of white blood cells called the myeloid cells, which normally develops into the various types of mature blood cells, such as red blood cells, white blood cells, and platelets. In other words, AML is a malignancy of the myeloid precursor cell line, characterized by the rapid proliferation of abnormal cells, which accumulate in the bone marrow and interfere with the production of normal cells.

AML is generally classified as de novo, or secondary when arising following exposure to prior cytotoxic chemotherapy, or after a history of prior myelodysplastic syndrome (MDS) or antecedent hematologic disorder (AHD). The pathogenesis of AML at the genetic level is also heterogeneous. Genetic alterations that cause AML include an internal tandem duplication in a tyrosine kinase gene, chromosomal rearrangements that alter the functioning of genes involved in leukemogenesis, and mutations resulting in activation of transcription factors, etc. Comprehensive profiling of genetic alterations in AML will enhance disease classification, risk stratification and prognosis, and ultimately, allow more precise therapeutic interventions. MV4-11 and MOLM-13 are AML cell lines that express FLT3 mutations. *See* Quentmeier et al., Leukemia, 17(1):120-4 (Jan. 2003). FLT3-ITD up-regulates MCL-1 to promote survival of stem cells in AML. *See* Yoshimoto et al., Blood, 114(24):5034-43 (Dec. 3, 2009). Document WO/2019/012328 relates to combination therapies of a cytarabine conjugate and one or more anti-neoplastic agents for inhibiting cancer cell growth.

Current strategies of AML treatment include inductive chemotherapy for remission induction and low-intensity therapy intended for survival prolongation. The remission-induction chemotherapy is a cytoreductive modality for achieving remission or at least effective reduction of tumor burden. The combination of cytarabine and anthracycline has been the mainstay of treatments to induce remission. A common induction regimen consists of cytarabine at doses of 100 to 200 mg/m²/day for 7 days and daunorubicin at doses of 45 to 90 mg/m²/day for 3 days, often referred to as the "7 + 3 protocol." If remission is achieved, additional cycles of chemotherapy or stem cell transplantation from a donor (allogeneic hematopoietic stem cell transplantation [HSCT]) are employed for consolidation. Although inductive chemotherapy has become the standard for younger fit patients, it remains a matter of debate in the elderly and unfit population. In elderly patients who have received IC, outcomes are less favorable primarily due to the increased rate of treatment-related death and poor prognostic factors leading to lower remission rates seen in the elderly population. Treatment options for patients considered ineligible or unfit due to age, performance status, and co-morbidities or those who choose not to receive IC current chemotherapy options include low-dose cytarabine, 5-azacytidine or decitabine.

Although induction chemotherapy produces morphologic complete remissions (CRs) in about 60% to 80% of younger adults and 40% to 50% of older adults with newly diagnosed AML, there is a substantial population of patients who will fail to attain CR (*i.e.,* refractory AML). Even for those who attain CR after induction treatment, a significant portion will eventually relapse, leading to only about 29% relapse-free survival at three years after treatment.

Thus, there is a need for more effective treatments for treating cancer, including but not limited to AML and/or MDS, and this disclosure addresses this need.

### SUMMARY

The present invention is defined by the claims. In the following paragraphs, whenever methods of treatment of the human or animal body are referred to, what is intended are compositions for use in such treatments. Provided herein are methods of treating AML and/or MDS using a (i) pharmaceutical composition comprising 5-azacytidine, and (ii) at least one additional therapeutic agent.

Certain embodiments herein provide that the additional therapeutic agent is gilteritinib, midostaurin, quizartinib, enasidenib, ivosidenib, or venetoclax. In the present invention the additional therapeutic agent is venetoclax.

Certain embodiments herein provide that the 5-azacytidine is administered as a composition that is a single unit dosage form. Certain embodiments herein provide compositions that are non-enteric-coated. Certain embodiments herein provide compositions that are immediate release oral compositions.

Provided in one aspect is a method of treating a human having acute myeloid leukemia or myelodysplastic syndrome, wherein the method comprises administering to the human (i) a pharmaceutical composition comprising 5-azacytidine administered orally, and (ii) at least one additional therapeutic agent.

In some embodiments, the at least one additional therapeutic agent comprises a FMS-like tyrosine kinase 3 (FLT3) inhibitor, an isocitrate dehydrogenase 2 (IDH2) inhibitor, an isocitrate dehydrogenase 2 (IDH1) inhibitor, and/or a B-cell lymphoma 2 (BCL2) inhibitor.

The at least one additional therapeutic agent is venetoclax.

In some embodiments, the 5-azacytidine and the at least one additional therapeutic agent are administered concomitantly. In some embodiments, the 5-azacytidine and the at least one additional therapeutic agent are administered sequentially wherein the 5-azacytidine is administered first.

In some embodiments, the 5-azacytidine and the at least one additional therapeutic agent are co-formulated as a single unit dosage form. In some embodiments, the 5-azacytidine and the at least one additional therapeutic agent are formulated as separate dosage forms.

In some embodiments, the at least one additional therapeutic agent is administered parenterally. In some embodiments, the at least one additional therapeutic agent is administered orally.

In some embodiments, the 5-azacytidine is administered at a dose of about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg , about 590 mg, or about 600 mg orally. In some embodiments, the 5-azacytidine is administered at a dose of about 200 mg. In some embodiments, the 5-azacytidine is administered at a dose of about 300 mg. In some embodiments, the 5-azacytidine is administered at a dose of 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 310 mg, 320 mg, 330 mg, 340 mg, 350 mg, 360 mg, 370 mg, 380 mg, 390 mg, 400 mg, 410 mg, 420 mg, 430 mg, 440 mg, 450 mg, 460 mg, 470 mg, 480 mg, 490 mg, 500 mg, 510 mg, 520 mg, 530 mg, 540 mg, 550 mg, 560 mg, 570 mg, 580 mg or 600 mg orally. In some embodiments, the 5-azacytidine is administered at a dose of 200 to 300 mg orally. In some embodiments, the 5-azacytidine is administered at a dose of 200 mg. In some embodiments, the 5-azacytidine is administered at a dose of 300 mg. In some embodiments, the 5-azacytidine is administered daily for the first seven, fourteen, or twenty-one days of a 28-day cycle. In some embodiments, the 5-azacytidine is administered to the human one or two times per day. In some embodiments, the 5-azacytidine is administered to the human once per day. In some embodiments, the 5-azacytidine is administered in the form of a capsule or a tablet. In some embodiments, the 5-azacytidine is administered in the form of a non-enteric-coated tablet. In some embodiments, the 5-azacytidine is administered in the form of an immediate release oral composition.

In some embodiments, the 5-azacytidine is administered at a dose of about 200 mg per day for 14 days in a 28-day cycle. In some embodiments, the 5-azacytidine is administered at a dose of about 300 mg per day for 14 days in a 28-day cycle. In some embodiments, the 5-azacytidine is administered at a dose of about 200 mg per day for 21 days in a 28-day cycle. In some embodiments, the 5-azacytidine is administered at a dose of about 300 mg per day for 21 days in a 28-day cycle. In some embodiments, the 5-azacytidine is administered at a dose of about 200 mg per day for 7 days in a 28-day cycle. In some embodiments, the 5-azacytidine is administered at a dose of about 300 mg per day for 7 days in a 28-day cycle. In some embodiments, the 5-azacytidine is administered at a dose of 200 mg per day for 14 days in a 28-day cycle. In some embodiments, the 5-azacytidine is administered at a dose of 300 mg per day for 14 days in a 28-day cycle. In some embodiments, the 5-azacytidine is administered at a dose of 200 mg per day for 21 days in a 28-day cycle. In some embodiments, the 5-azacytidine is administered at a dose of 300 mg per day for 21 days in a 28-day cycle. In some embodiments, the 5-azacytidine is administered at a dose of 200 mg per day for 7 days in a 28-day cycle. In some embodiments, the 5-azacytidine is administered at a dose of 300 mg per day for 7 days in a 28-day cycle.

In some embodiments, the 5-azacytidine is administered (a) daily for 1, 2, 3, 4, 5, 6, 7, 8, 9, 10, 11, 12, 13, 14, or greater than 14 days, optionally followed by a treatment dosing holiday of 1, 2, 3, 4, 5, 6, 7, 8, 9, 10, 11, 12, 13, 14, or greater than 14 days; (b) daily for 14 or more days, optionally followed by a treatment dosing holiday of 7 or more days; (c) for 21 or more days, optionally followed by a treatment dosing holiday of 7 or more days; (d) for 14 days, optionally followed by a treatment dosing holiday of 14 days; (e) for 21 or more days, followed by a treatment dosing holiday of 7 or more days; (f) for 14 days, followed by a treatment dosing holiday of 14 days. In some embodiments, at least one of steps (a), (b), (c), (d), (e), or (f) are repeated.

In some embodiments, the 5-azacytidine is administered (a) at a dose of about 300 mg daily for 14 days, followed by a treatment dosing holiday of 14 days; (b) at a dose of about 200 mg daily for 14 days, followed by a treatment dosing holiday of 14 days; (c) at a dose of about 300 mg daily for 21 days, followed by a treatment dosing holiday of 7 days; (d) at a dose of about 200 mg daily, followed by a treatment dosing holiday of 7 days; (e) at a dose of 300 mg daily for 14 days, followed by a treatment dosing holiday of 14 days; (f) at a dose of 200 mg daily for 14 days, followed by a treatment dosing holiday of 14 days; (g) at a dose of 300 mg daily for 21 days, followed by a treatment dosing holiday of 7 days; or (h) at a dose of 200 mg daily, followed by a treatment dosing holiday of 7 days . In some embodiments, at least one of steps (a), (b), (c), (d), (e), (f), (g), or (h) are repeated.

In some embodiments, the 5-azacytidine is administered using a treatment cycle comprising administration of 5-azacytidine per day for 7 days in a 28-day cycle In some embodiments, the 5-azacytidine is administered using a treatment cycle comprising administration of 5-azacytidine per day for 14 days in a 28-day cycle. In some embodiments, the 5-azacytidine is administered using a treatment cycle comprising administration of 5-azacytidine per day for 21 days in a 28-day cycle.

In some embodiments, the 5-azacytidine and the at least one additional therapeutic agent provides a synergistic effect to treat the acute myeloid leukemia or myelodysplastic syndrome.

In some embodiments, the acute myeloid leukemia is characterized as caused by a FMS-like tyrosine kinase-3 internal tandem duplication (FLT3-ITD) mutation. In some embodiments, the 5-azacytidine is administered before the at least one additional therapeutic agent. In some embodiments, the 5-azacytidine and at least one additional therapeutic agent augments myeloid cell leukemia 1 (MCL-1) degradation.

In some embodiments, the 5-azacytidine and at least one additional therapeutic agent increases median survival as compared to 5-azacytidine administered intravenously or subcutaneously and at least one additional therapeutic agent. In some embodiments, the 5-azacytidine and at least one additional therapeutic agent increases median survival as compared to 5-azacytidine administered intravenously or subcutaneously and at least one additional therapeutic agent by about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%.

In some embodiments, the acute myeloid leukemia is resistant to treatment with at least one additional therapeutic agent. In some embodiments, the acute myeloid leukemia is responsive to treatment with a FMS-like tyrosine kinase-3 (FLT3 inhibitor). In some embodiments, the acute myeloid leukemia is characterized as having an overexpression of MCL-1. In some embodiments, the 5-azacytidine primes the cancer cells for apoptosis mediated by the at least one additional therapeutic agent by downregulating the expression of MCL-1. In some embodiments, the 5-azacytidine and at least one additional therapeutic agent augments MCL-1 degradation. In some embodiments, the 5-azacytidine alters cell metabolism. In some embodiments, the 5-azacytidine causes cell cycle arrest. In some embodiments, the 5-azacytidine suppresses oxidative phosphorylation. In some embodiments, the 5-azacytidine increases expression of activating transcription factor 3 (ATF3). In some embodiments, the 5-azacytidine decreases expression of stearoyl-CoA desaturase (SCD).

In some embodiments, the method comprises: (a) administering the 5-azacytidine daily to the human for 1, 2, or 3 days; (b) administering the at least one additional therapeutic agent to the human for one or more days; and (c) optionally repeating steps (a) and (b).

In some embodiments, the method comprises: (a) administering the 5-azacytidine daily to the human for 1, 2, 3, 4, 5, 6, 7, 8, 9, 10, 11, 12, 13, or 14 days; (b) administering the at least one additional therapeutic agent to the human for one or more days; and (c) optionally repeating steps (a) and (b).

In some embodiments, the method comprises: (a) administering the 5-azacytidine daily to the human for 1, 2, 3, 4, 5, 6, 7, 8, 9, 10, 11, 12, 13, 14, 15, 16, 17, 18, 19, 20, or 21 days; (b) administering the at least one additional therapeutic agent to the human for one or more days; and (c) optionally repeating steps (a) and (b).

In some embodiments, the method comprises: (a) administering the 5-azacytidine daily to the human for 1, 2, 3, 4, 5, 6, 7, 8, 9, 10, 11, 12, 13, or 14 days of a 28-day cycle; (b) concurrently administering the at least one therapeutic agent daily to the human for 1, 2, 3, 4, 5, 6, 7, 8, 9, 10, 11, 12, 13, 14, 15, 16, 17, 18, 19, 20, 21, 22, 23, 24, 25, 26, 27, or 28 days of a 28-day cycle; and (c) optionally repeating steps (a) and (b).

In some embodiments, the method comprises: (a) administering the 5-azacytidine daily to the human for 1, 2, 3, 4, 5, 6, 7, 8, 9, 10, 11, 12, 13, 14, 15, 16, 17, 18, 19, 20, or 21 days of a 28-day cycle; (b) concurrently administering the at least one additional therapeutic agent daily to the human for 1, 2, 3, 4, 5, 6, 7, 8, 9, 10, 11, 12, 13, 14, 15, 16, 17, 18, 19, 20, 21, 22, 23, 24, 25, 26, 27, or 28 days of a 28-day cycle; and (c) optionally repeating steps (a) and (b).

In some embodiments, the method comprises the sequential steps of: (a) administering the 5-azacytidine to the human for 7 days of a 28-day cycle; (b) administering the at least one additional therapeutic agent to the human for 1 day of a 28-day cycle; (c) administering 5-azacytidine to the human for 6 days of a 28-day cycle; and (d) repeating steps (a) to (c) after 7 days of a resting period.

In some embodiments, the method comprises the sequential steps of: (a) administering 5-azacytidine daily to the human for 1, 2, 3, 4, 5, 6, 7, 8, 9, 10, 11, 12, 13, 14, 15, 16, 17, 18, 19, 20, or 21 days of a 28-day cycle; (b) administering the at least one additional therapeutic agent daily to the human for 1, 2, 3, 4, 5, 6, 7, 8, 9, 10, 11, 12, 13, 14, 15, 16, 17, 18, 19, 20, or 21 days of a 28-day cycle; (c) administering 5-azacytidine daily to the human for 1, 2, 3, 4, 5, 6, 7, 8, 9, 10, 11, 12, 13, 14, 15, 16, 17, 18, 19, 20, or 21 days of a 28-day cycle; and (d) optionally repeating steps (a) and (c) after 1, 2, 3, 4, 5, 6, 7, 8, 9, 10, 11, 12, 13, 14, 15, 16, 17, 18, 19, 20, or 21 days of a resting period.

In some embodiments, the human has acute myeloid leukemia. In some embodiments, the human has myelodysplastic syndrome. In some embodiments, the myelodysplastic syndrome is high and very high risk myelodysplastic syndromes as defined by the Revised International Prognostic Scoring System (IPSS-R).

### BRIEF DESCRIPTION OF THE DRAWINGS

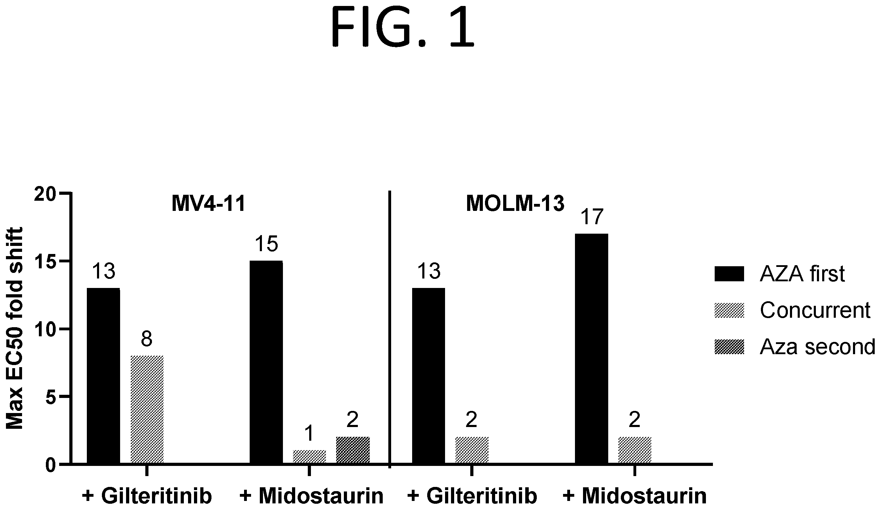

FIG. 1 represents the maximum EC₅₀ fold shift of 5-azacytidine in combination with gilteritinib, and 5-azacytidine in combination with midostaurin; both with cell lines MV4-11 and MOLM-13. The results from three different dosing schedules are shown: (i) 5-azacytidine administered first (black bar); (ii) two agents administered concurrently (light gray bar); and (iii) 5-azacytidine administered second (medium gray bar).

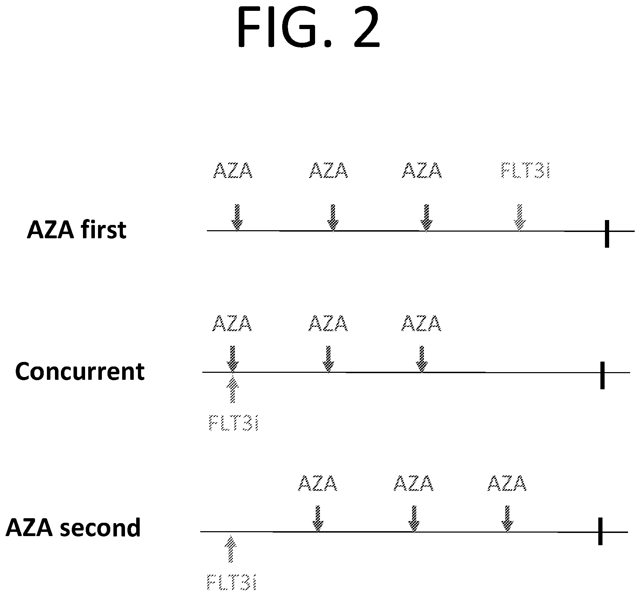

FIG. 2 represents the three different dosing schedules of (i) 5-azacytidine (AZA) administered first at intervals before the FLT3 inhibitor (FLT3i); (ii) the two agents (5-azacytidine and FLT3i) administered concurrently; and (iii) 5-azacytidine administered second at intervals after the FLT3i is administered. The FLT3 inhibitor may be any suitable FLT3 inhibitor, including midostaurin, or gilteritinib.

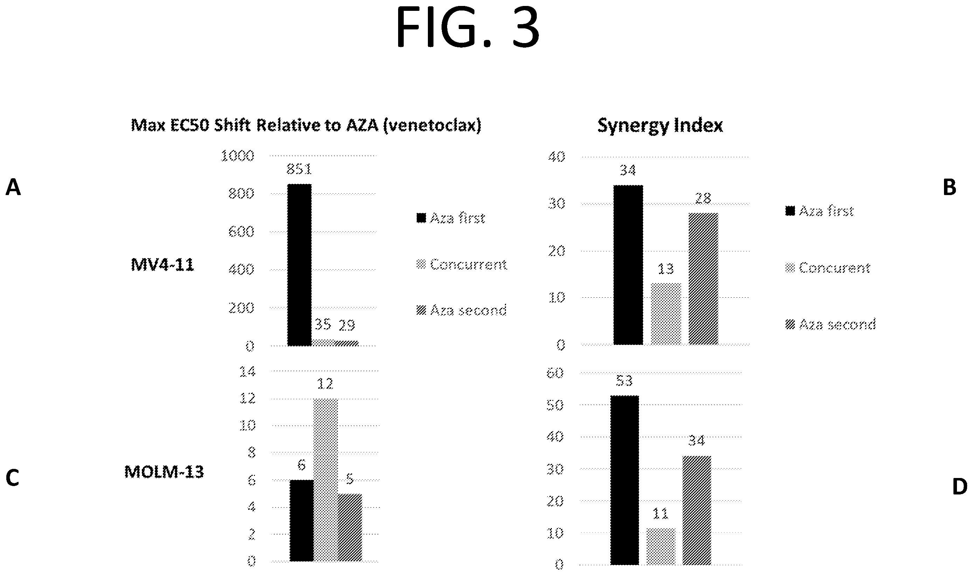

FIGS. 3A-D represent the maximum EC₅₀ fold shift of 5-azacytidine in combination with venetoclax with cell lines MV4-11 (FIG. 3A) and MOLM-13 (FIG. 3C). The results from three different dosing schedules are shown, as indicated in the legend: (i) 5-azacytidine administered first (black bar); (ii) the two agents administered concurrently (light gray bar); and (iii) 5-azacytidine administered second (medium gray bar). A synergy index is also shown for 5-azacytidine administered in combination with venetoclax with cell lines MV4-11 (FIG. 3B) and MOLM-13 (FIG. 3D) for the three different dosing schedules.

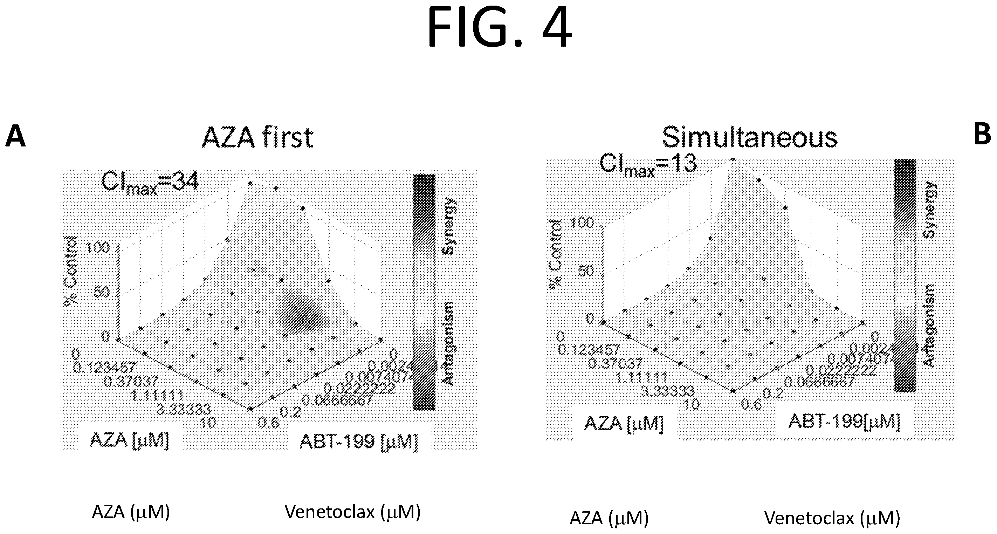

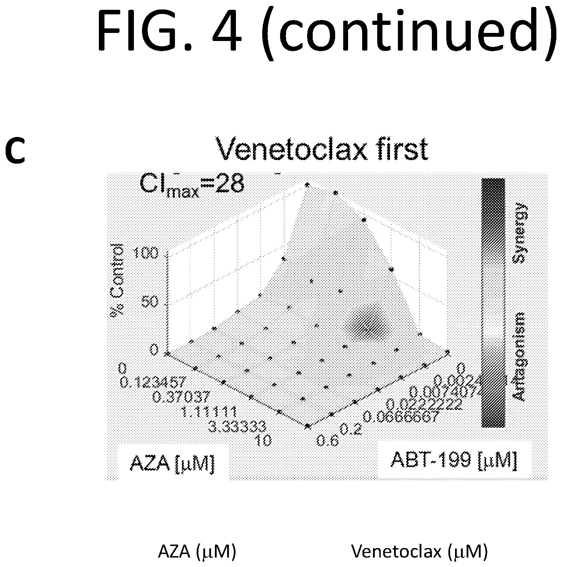

FIGS. 4A-C represent Response Surface Analyses showing synergistic effects of 5-azacytidine with venetoclax in MV4-11 cells when 5-azacytidine is administered first (FIG. 4A), the relatively lower synergy with simultaneous administration (FIG. 4B), and synergy with venetoclax administered first (FIG. 4C). Response surface methodology (RSM) is a well-known statistical method to explore the relationships between several explanatory variables and one or more response variables. RSM uses a sequence of designed experiments to obtain an optimal response, which in the present case is the synergistic effects of 5-azacytidine with venetoclax.

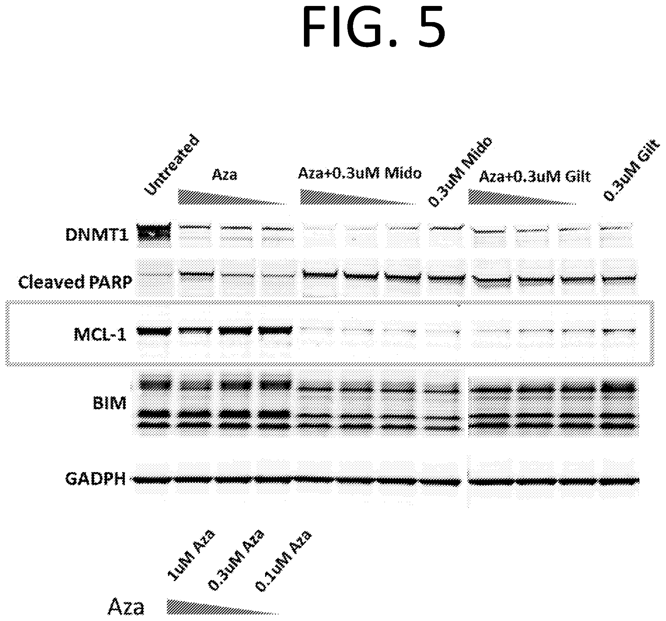

FIG. 5 depicts a western blot showing that (a) 5-azacytidine and midostaurin ("aza+0.3 µM Mido") and (b) 5-azacytidine and gilteritinib ("aza + 0.3 µM Gilt") augment MCL-1 degradation in MV4-11 cell lines.

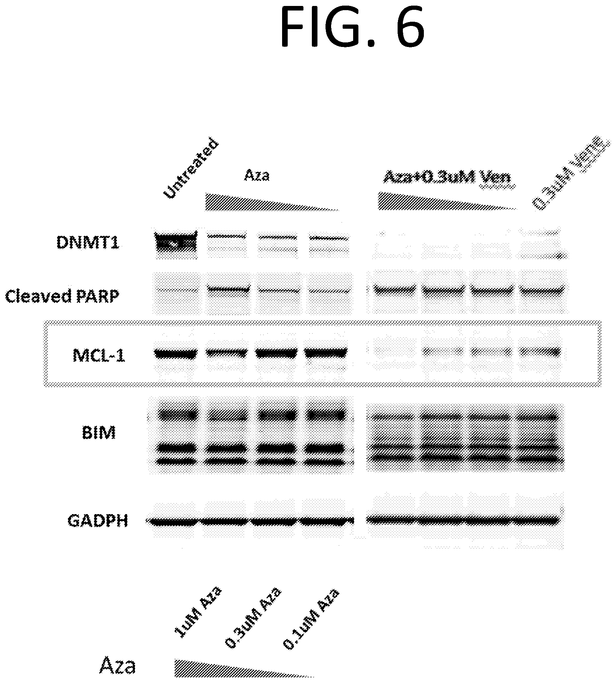

FIG. 6 depicts a western blot showing that 5-azacytidine and venetoclax treatment decreases MCL-1 levels in FLT3ITD MV4-11 cells.

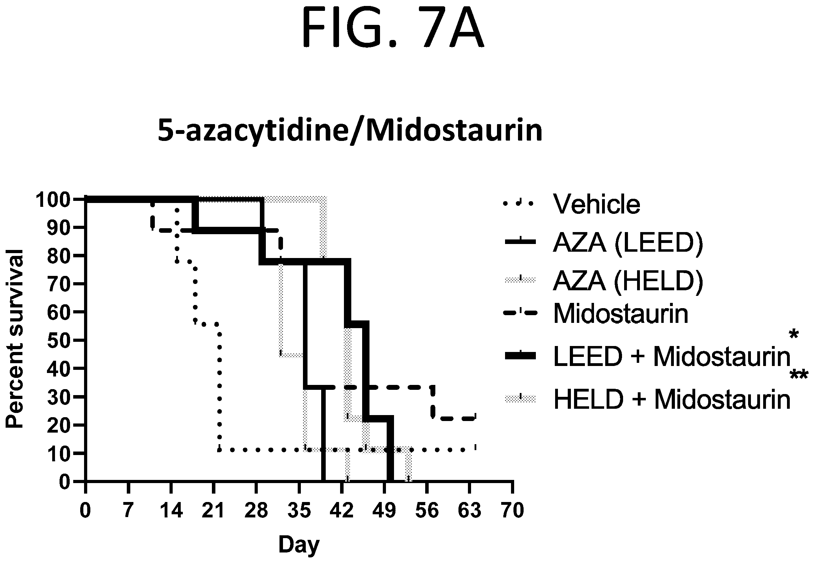

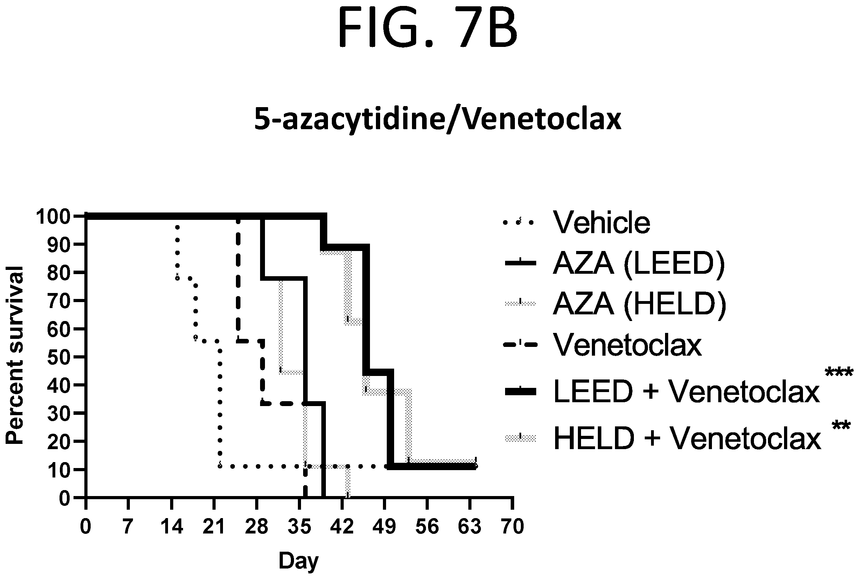

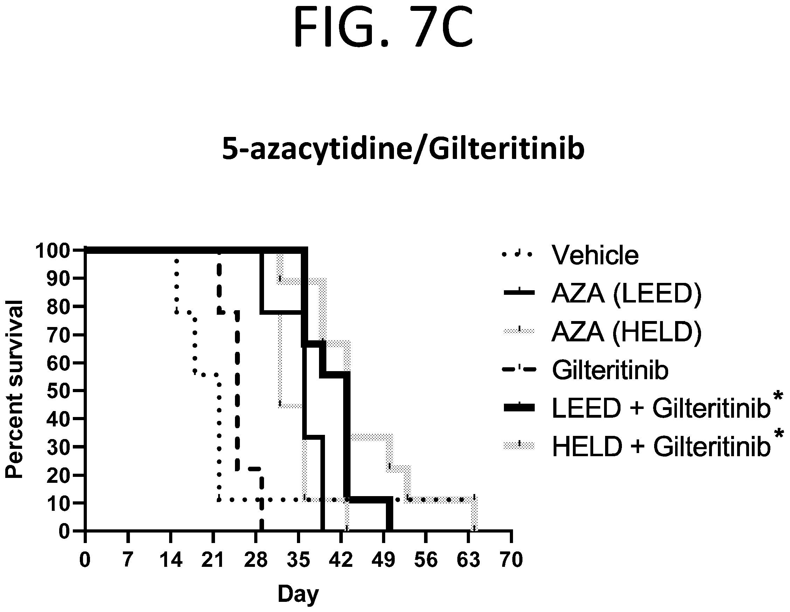

FIGS. 7A-C depicts *in vivo* assessments of 5-azacytidine combinations in a MOLM-13 xenograft model, with a graph of percent survival (y-axis) vs. day 0 to 70 (x-axis). FIG. 7A shows the results of the combination of 5-azacytidine and midostaurin, FIG. 7B shows the results of 5-azacytidine combined with venetoclax, and FIG. 7C shows the results of the combination of 5-azacytidine and gilteritinib. Dosing for the experiments shown in Figs. 7A-C was as follows: (i) 5-azacytidine (low exposure, extended duration, LEED): 1 mg/kg interperitoneally (IP), once daily for five days, three times (qdx 5x3); (ii) 5-azacytidine (high exposure, limited duration, HELD): 3 mg/kg interperitoneally (IP), once daily for five days (qdx5); (iii) Midaustaurin (100 mg/kg orally (PO), once daily for twenty-one days (qdx21)); (iv) Gilteritinib (4 mg/kg orally (PO), once daily for twenty-one days (qdx21)); and (v) Venetoclax (100 mg/kg orally (PO), once daily for twenty-one days (qdx21)). P-value (relative to best single agent) *P < 0.05; **P<0.001; ***P<0.0001.

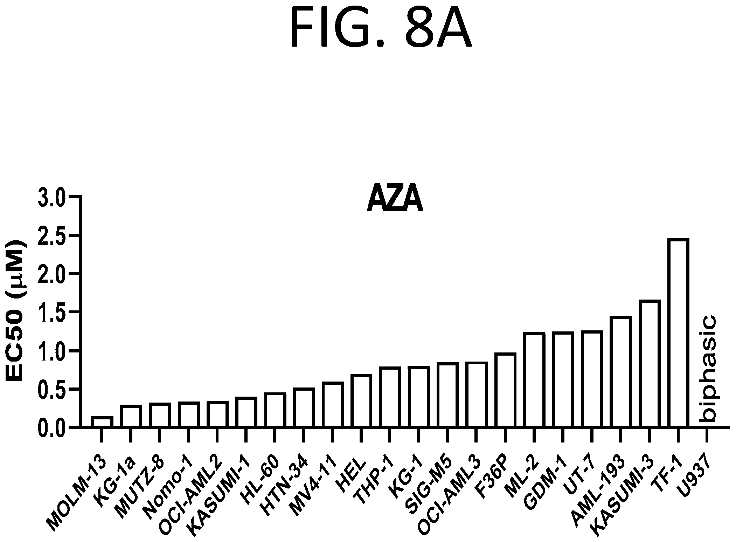

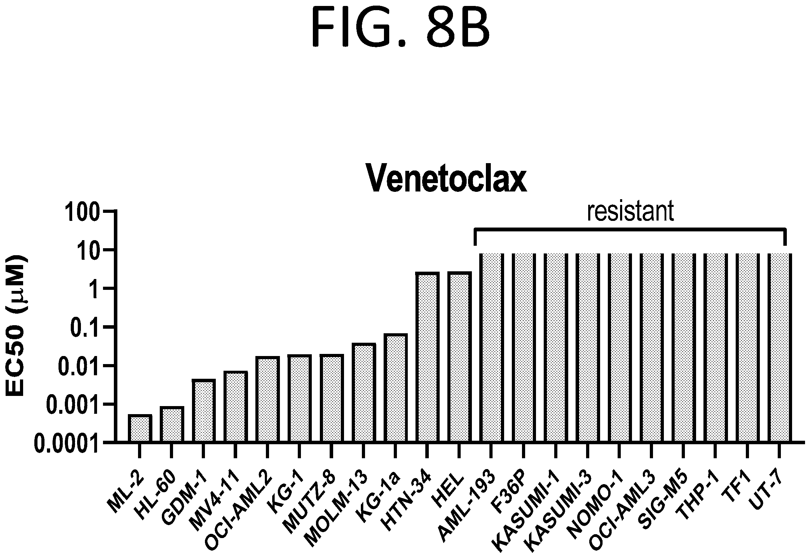

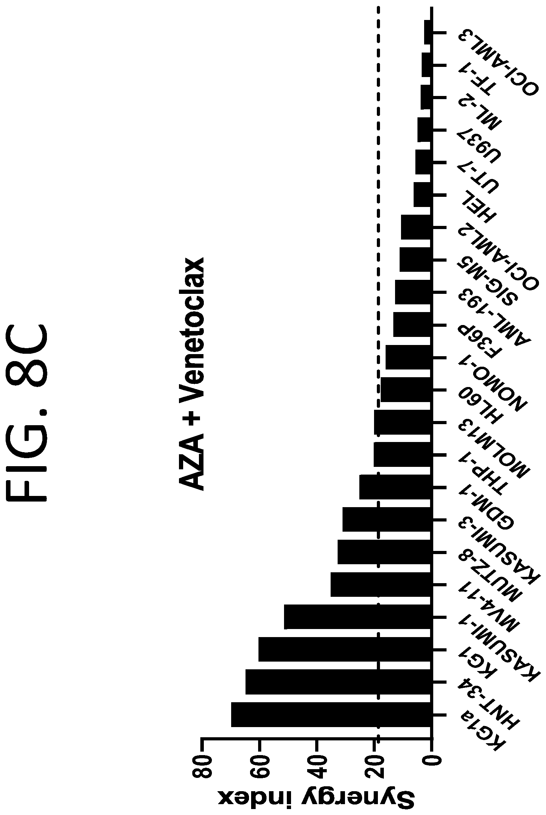

FIGS. 8A, 8B, and 8C depict the sensitivity of 22 AML cell lines to 5-azacytidine (AZA) and venetoclax as single agents (FIG. 8A and 8B, respectively) and the combination with 5-azacytidine and venetoclax (FIG. 8C).

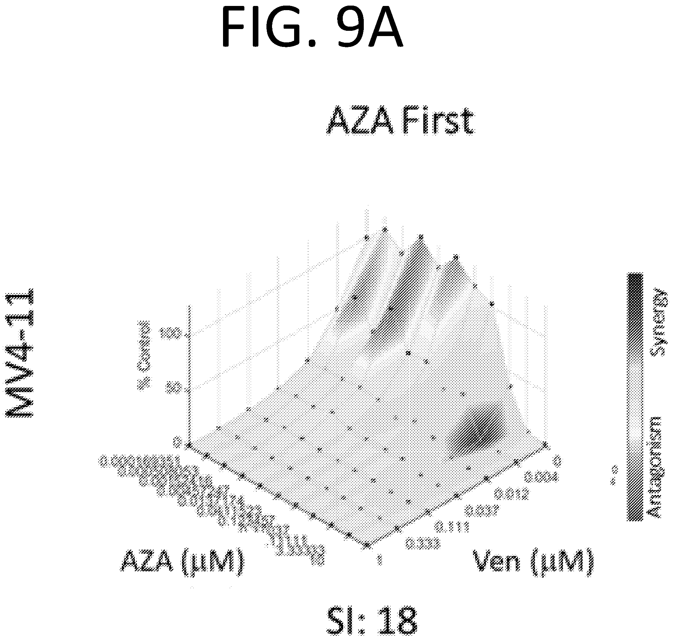

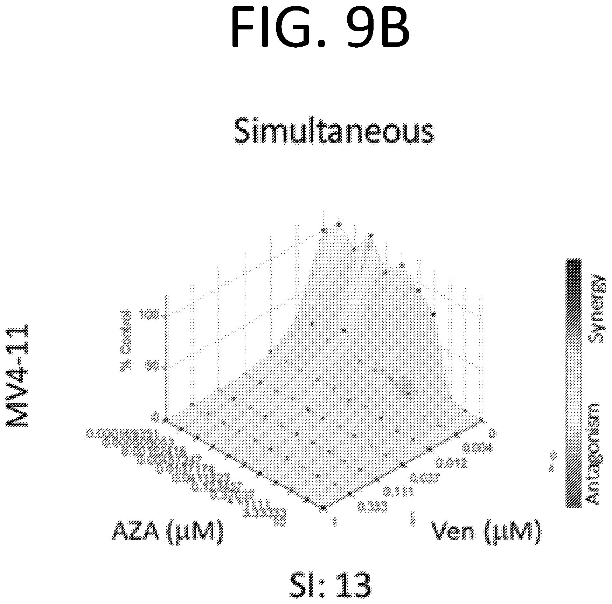

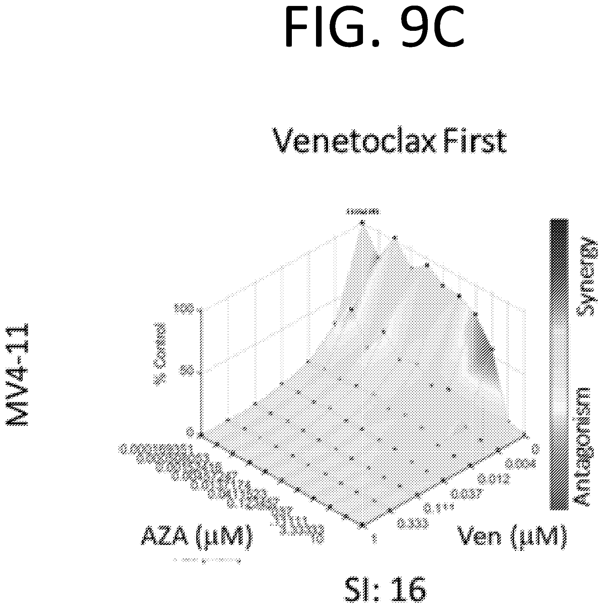

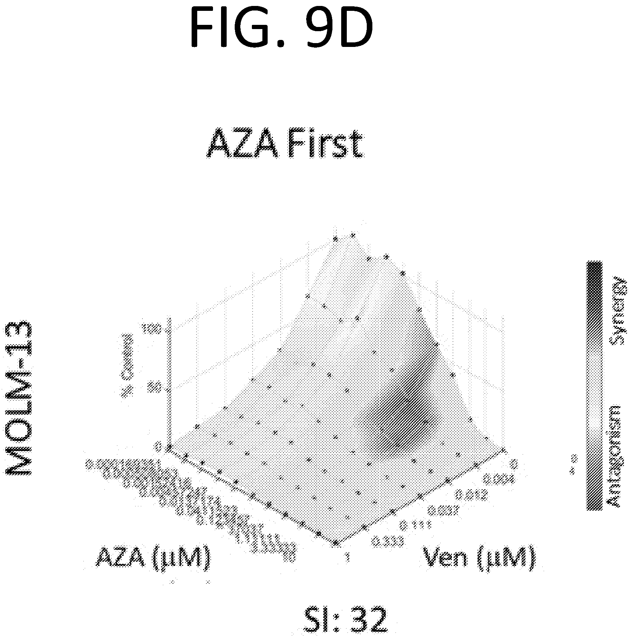

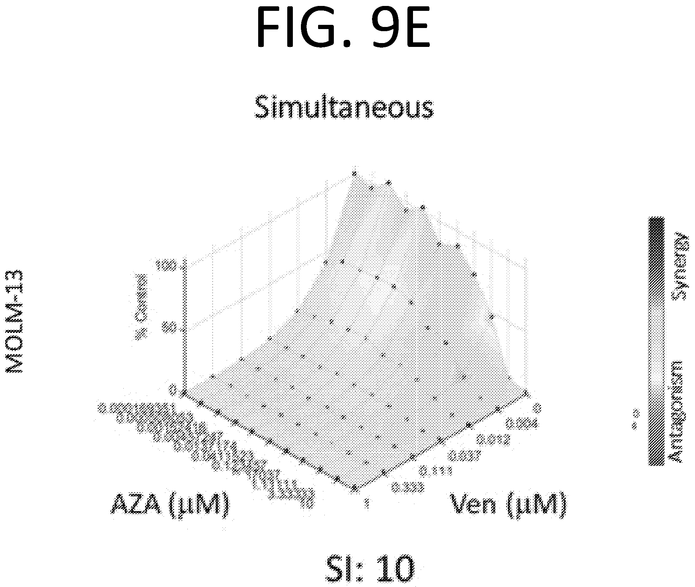

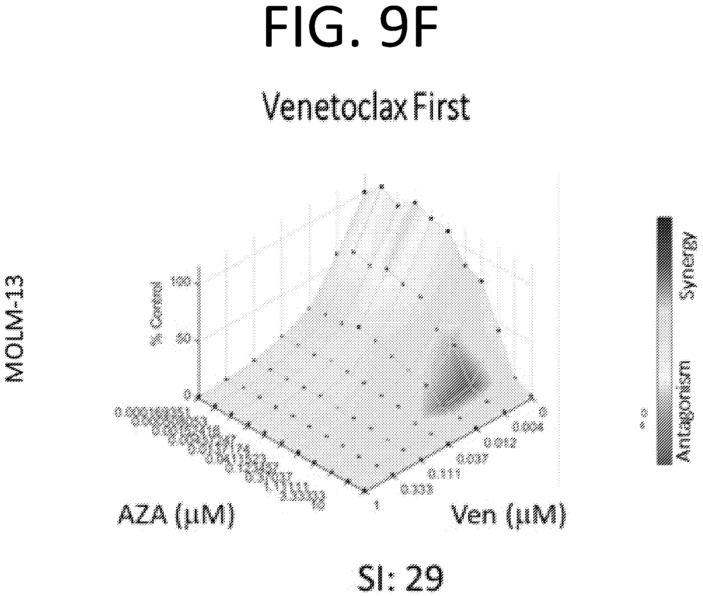

FIGS. 9A-F show the cell survival of MV4-11 cells (FIGS. 9A-C) and MOLM-13 cells (FIGS. 9D-F) and the corresponding synergy index (SI) seven days after the start of treatment with 5-azacytidine and venetoclax. The following schedules were tested: 5-azacytidine administration on Days 1, 2 and 3, followed by venetoclax administration on Day 4 (5-azacytidine (AZA) First) (FIGS. 9A and 9D); 5-azacytidine and venetoclax co-administration on Day 1, followed by 5-azacytidine administration on Days 2 and 3 (Simultaneous) (FIGS. 9B and 9E); and venetoclax administration on Day 1, followed by 5-azacytidine on Days 2, 3 and 4 (venetoclax first) (FIGS. 9C and 9F).

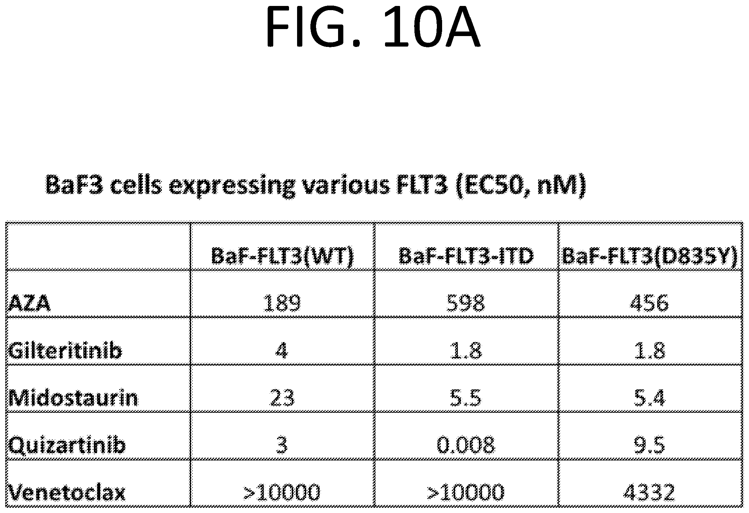

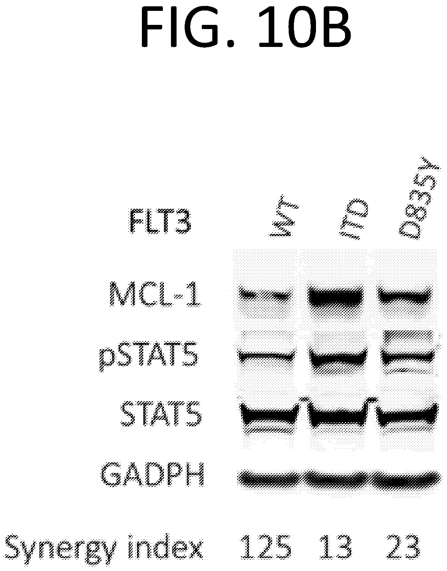

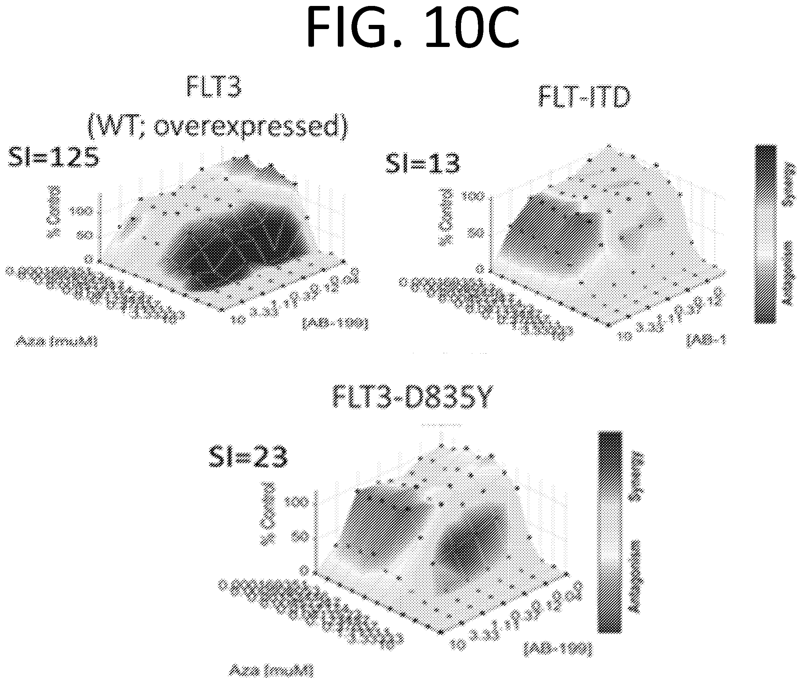

FIGS. 10A, 10B, and 10C depict the correlation of MCL-1 expression with the degree of the synergistic effect of the combination of 5-azacytidine andvenetoclax in a panel of engineered BaF3 cell lines expressing wild-type FLT3, FLT3-ITD or FLT3 (D835Y) mutations.

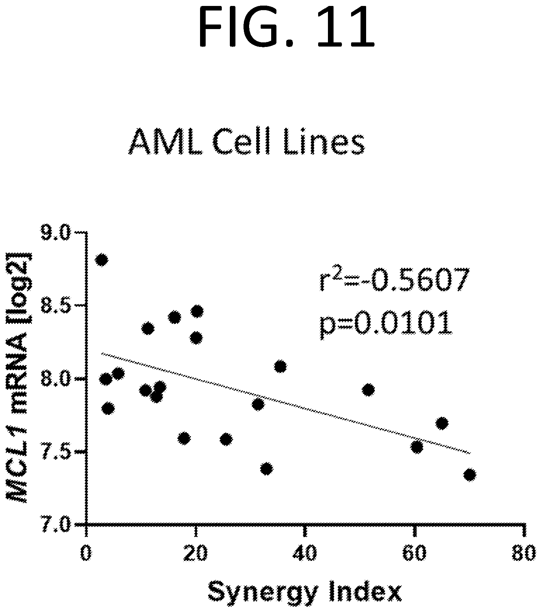

FIG. 11 depicts the correlation of *MCL1* RNA level, as measured by RNASeq, with the synergy index (r² = -0.5607, p = 0.0101) in a panel of 20 AML cell lines.

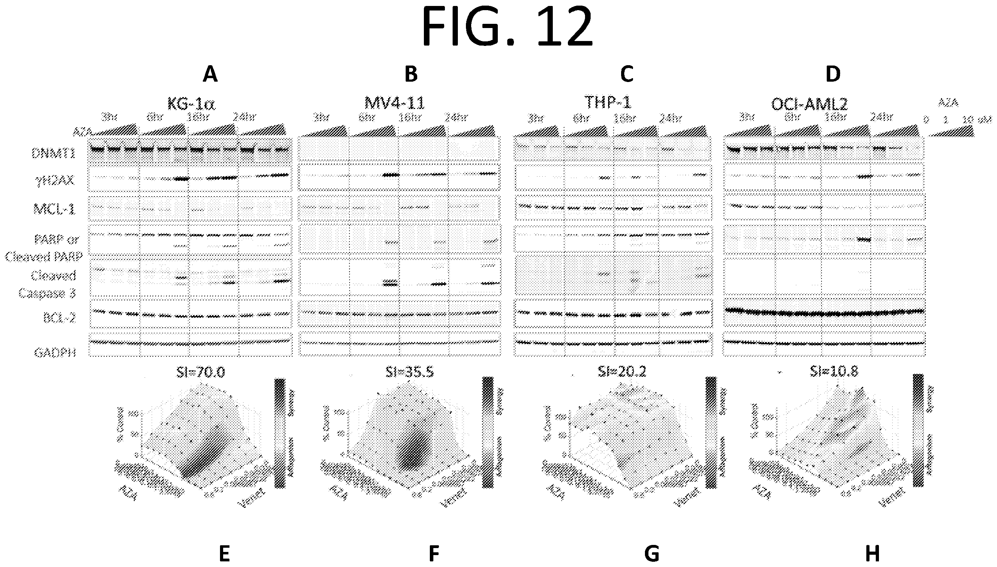

FIG. 12A-H depict the extent of 5-azacytidine-mediated MCL-1 degradation in four different AML cell lines: KG1α (FIG. 12A), MV4-11 (FIG. 12B), THP-1 (FIG. 12C), and OCI-AML2 (FIG. 12D). The results showed 5-azacytidine-venetoclax synergistic activity with KG1α (FIG. 12E) and MV4-11 (FIG. 12F) cell lines (synergy index (SI) of 70 and 35.5, respectively) and very little or no synergistic activity with THP-1 (FIG. 12G) and OCI-AML-2 (FIG. 12H) cell lines (SI of 20.2 and 10.8, respectively). For the KG1α (FIG. 12A) and MV4-11 (FIG. 12B) cell lines, where 5-azacytidine-venetoclax had the greatest synergistic effect (FIGS. 12E and 12F), 5-azacytidine led to MCL-1 degradation the fastest, starting 6 hours after treatment. In contrast, for THP-1 (FIG. 12G), where 5-azacytidine-venetoclax only provided minor synergistic activity showed 5-azacytidine-mediated MCL-1 degradation later, starting at 16 hours, with incomplete degradation by 24 hours (FIG. 12C). For OCI-AML2 (FIG. 12D), where 5-azacytidine-venetoclax had the lowest synergistic effect (FIG. 12H), 5-azacytidine treatment did not lead to any degradation of MCL-1.

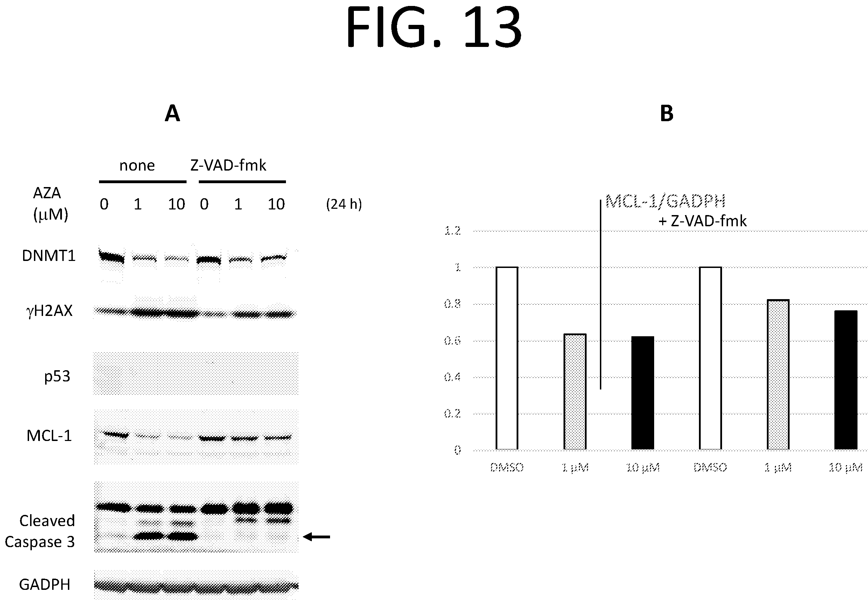

FIG. 13A depicts a western blot evaluating the degradation of caspase 3. Cells were treated with Z-VAD-FMK, a pan-caspase inhibitor, and the extent of MCL-1 degradation by 5-azacytidine was measured. FIG. 13B shows a bar graph of MCL-1 degradation by 5-azacytidine, where cells were treated with 20 µM Z-VAD-FMK for 1 hour before 5-azacytidine treatment for another 16 hours. Caspase inhibition partially ablated MCL-1 degradation by 5-azacytidine in MV4-11 cells, which suggested additional, caspase-independent mechanisms of MCL-1 degradation.

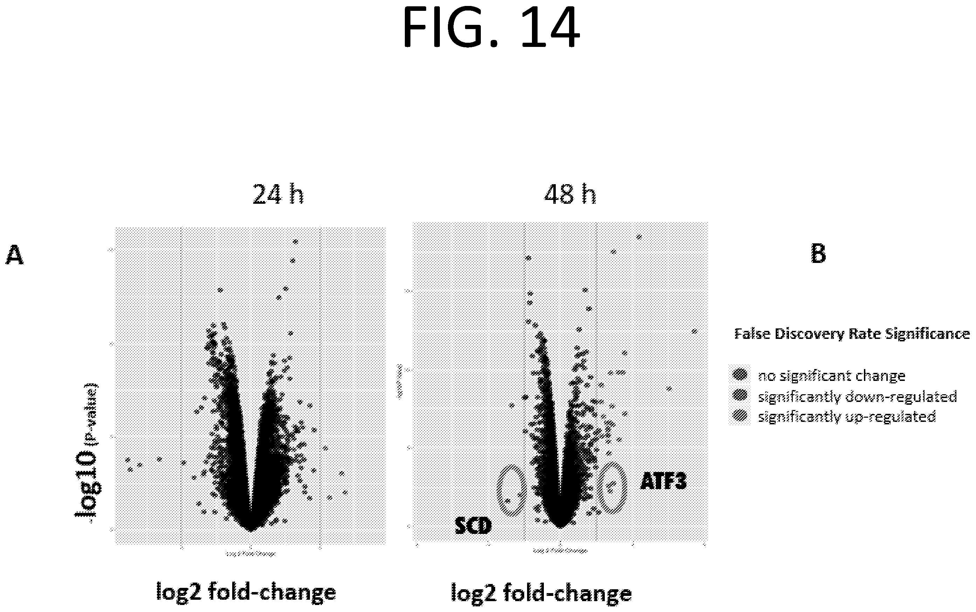

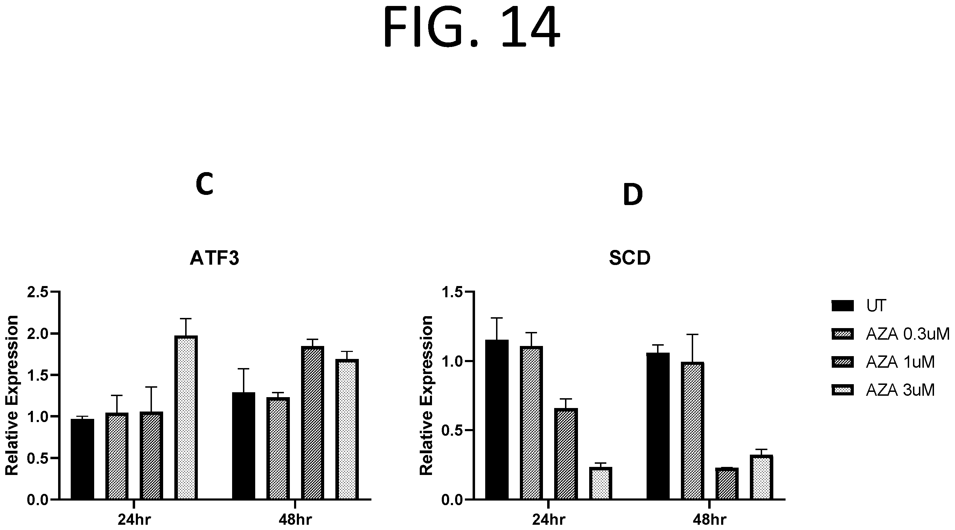

FIGS. 14A and B depict the results of an RNAseq performed on MV4-11 cells treated with PBS (vehicle), 1 µM 5-azacytidine for 24 hours (FIG. 14A), or with 1 µM 5-azacytidine for 48 hours (FIG. 14B). FIGS. 14A and B show volcano plots of significantly modified genes at 24 hours (FIG. 14A) and 48 hours (FIG. 14B), showing that 5-azacytidine induced 133 differentially expressed genes at 24 hours and 226 differentially expressed genes at 48 hours. Upon further analysis of the 5-azacytidine-induced differentially expressed genes, two genes were identified that have previously been shown to regulate *MCL1* expression: activating transcription factor 3 (ATF3) and stearoyl-CoA desaturase (SCD). ATF3 expression was increased two-fold 48 hours after 5-azacytidine treatment. The expression of SCD (Stearoyl-CoA desaturase), a regulator of lipid metabolism and MCL1, was decreased 2.5-fold by 5-azacytidine treatment at 48 hours. Alterations in ATF3 (FIG. 14C) and SCD (FIG. 14D) expression were validated in a separate experiment using real-time PCR. ATF3 expression was increased in a time- and concentration-dependent manner, as 0.3 µM 5-azacytidine treatment was not sufficient to induce ATF3 expression at either 24 or 48 hours (FIG. 14C). Similarly, SCD expression was decreased rapidly within 24 hours when treated with 3 µM 5-azacytidine, although it was not affected by low concentrations of 5-azacytidine at this timepoint (FIG. 14D).

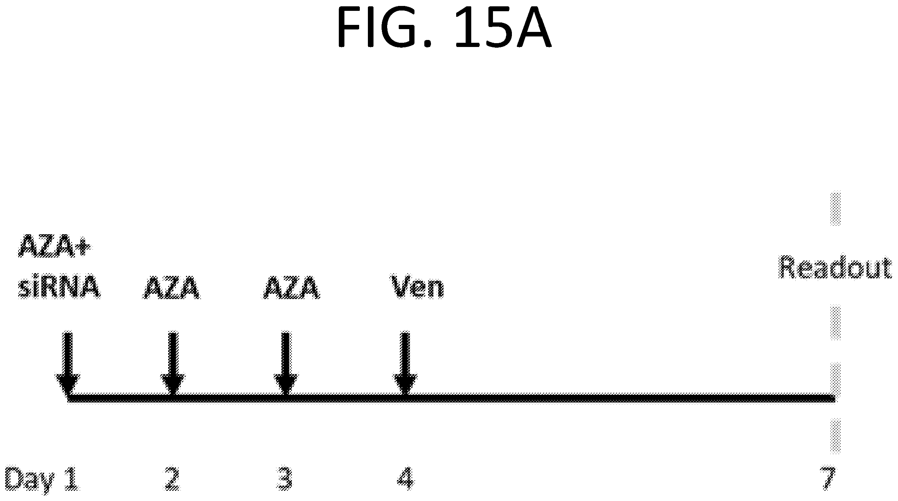

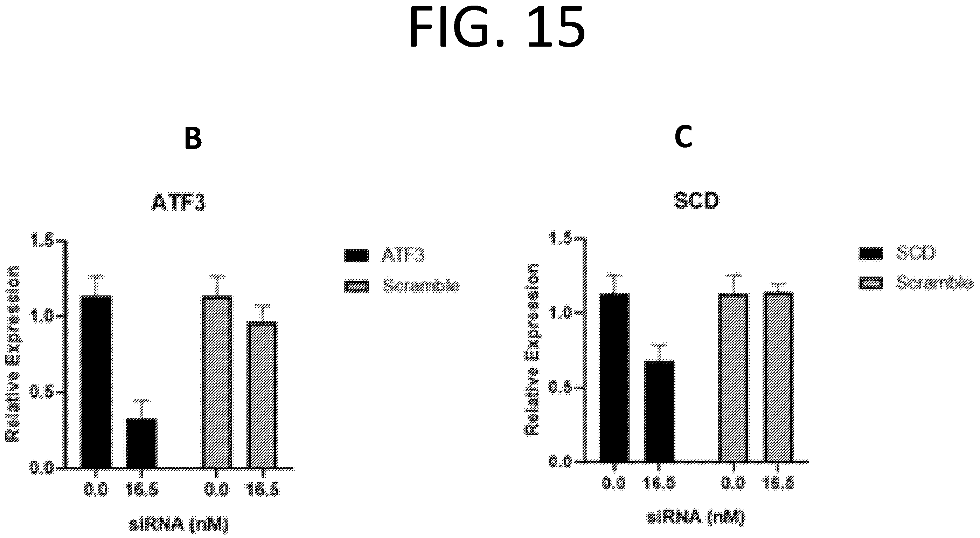

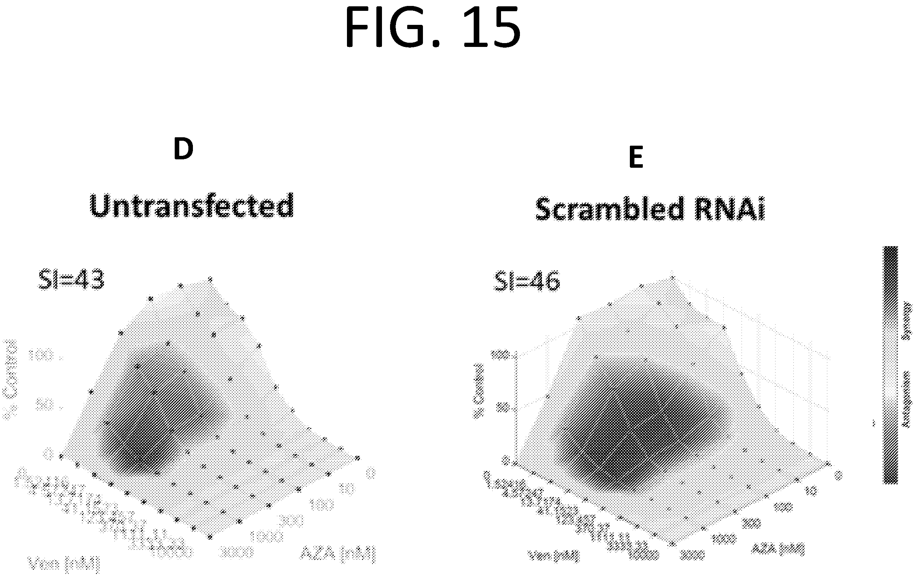

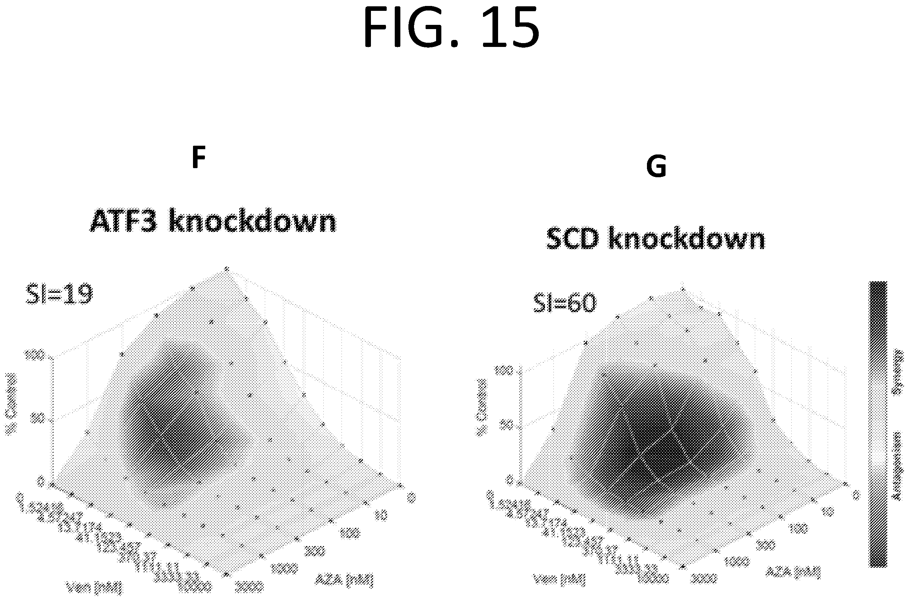

FIGS 15A-C shows the results of siRNA knockdown of ATF3 and/or SCD genes in MV4-11 cells to assess their function in synergy. MV4-11 cells were left untransfected or transfected with ATF3, SCD, or control (scrambled) siRNA. As a control, cells were transfected with siRNA and collected for RNA and qPCR 72 hours after transfection (FIG. 15A). In cells treated with scramble siRNA, no changes in ATF3 (FIG. 15B) or SCD (FIG. 15C) expression were seen. Following transfection, cells were treated with various concentrations of 5-azacytidine daily for Days 1-3. At Day 4, cells were dosed with venetoclax, followed by cell viability test using CellTiter-Glo^{®} 7 after treatment initiation. 5-azacytidine-venetoclax synergy was calculated using Combenefit and Highest Single Agent analysis (FIG. 15 D-G). FIG. 15D = untransfected cells; FIG. 15E = scrambled RNAi; FIG. 15F = ATF3 knockdown; and FIG. 15G = SCD knockdown.

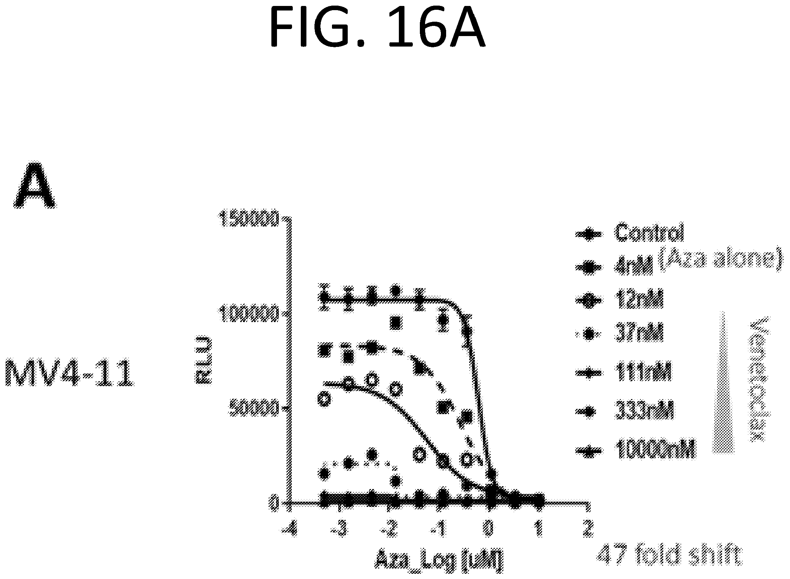

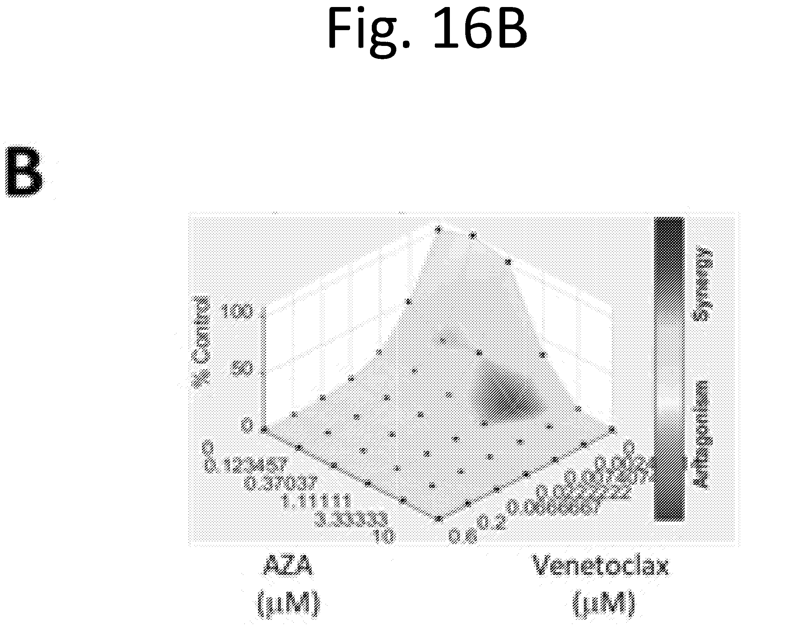

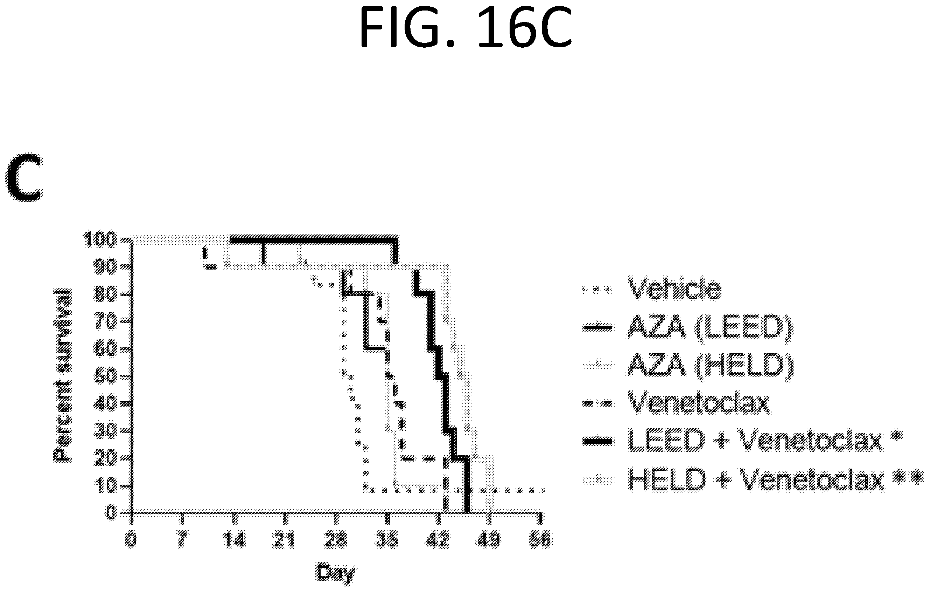

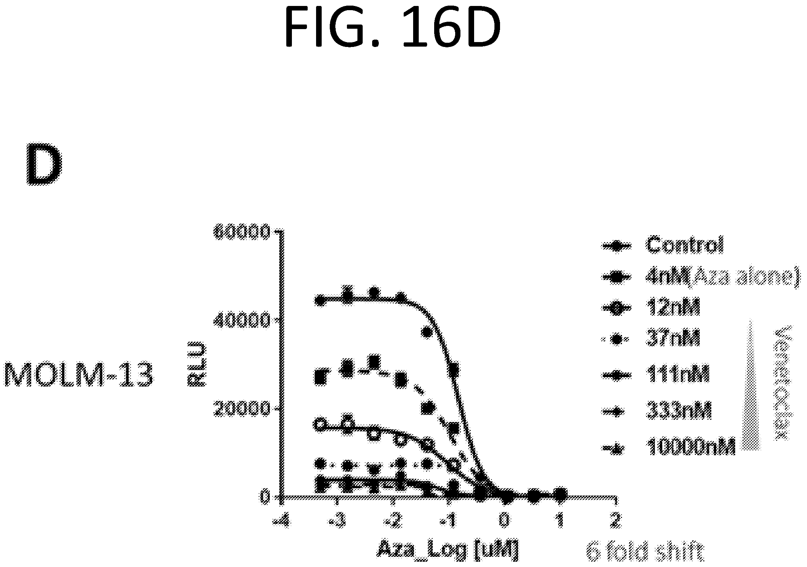

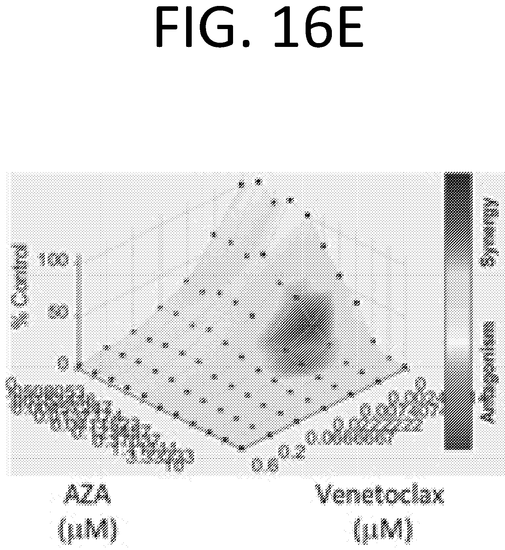

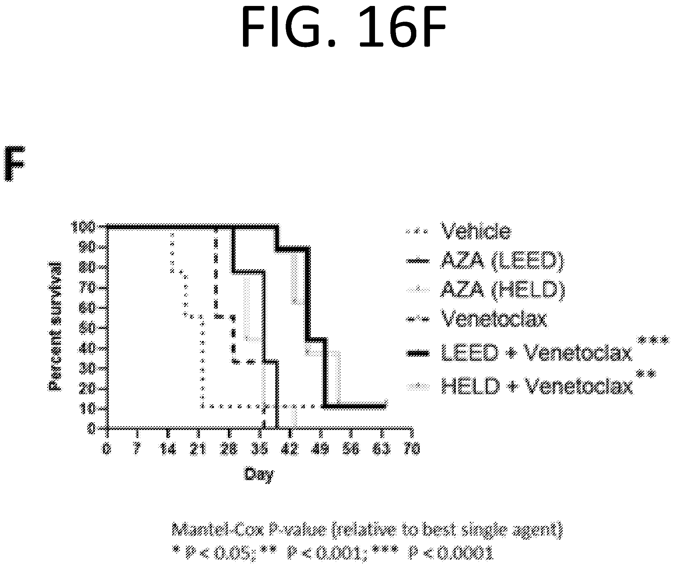

FIGS. 16A, 16B, 16C, 16D, 16E, and 16F depict the results of an evaluation as to whether 5-azacytidine and venetoclax have synergistic activity *in vivo* at doses and schedules corresponding to injectable 5-azacytidine (HELD) or oral 5-azacytidine (LEED). MV4-11 (FIGS. 16A-C) and MOLM-13 (FIGS. 16D-F), two cell lines that showed 5-azacytidine-venetoclax synergy, were to used to generate disseminated AML xenograft mice in immunodeficient animals. *In vitro,* venetoclax sensitized both cell lines to venetoclax (FIGS. 16A and 16D) and synergized with 5-azacytidine (FIGS. 16B and 16E). To model oral 5-azacytidine (LEED) regimens, mice were treated with 1 mg/kg 5-azacytidine for 15 days (low exposure, extended duration). Alternatively, to use the same cumulative dose but with an injectable 5-azacytidine (HELD) regimen, mice were treated with 3 mg/ml 5-azacytidine for 5 days (high exposure, limited duration).

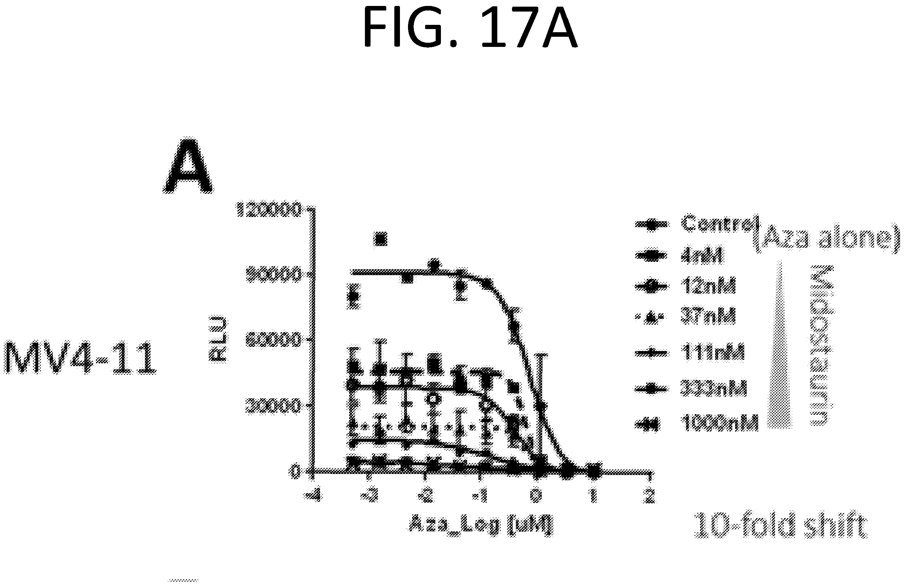

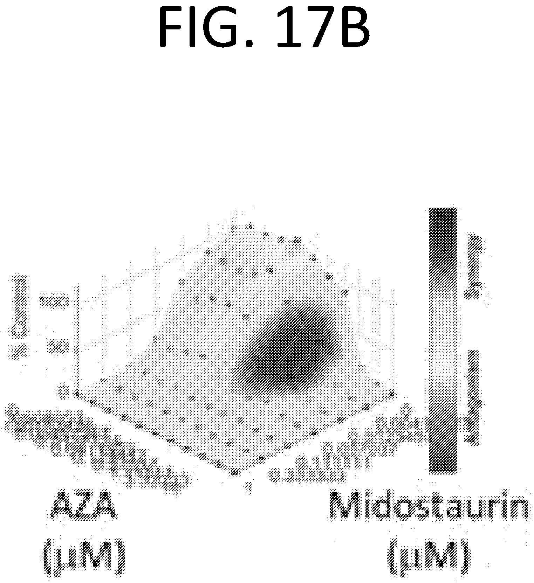

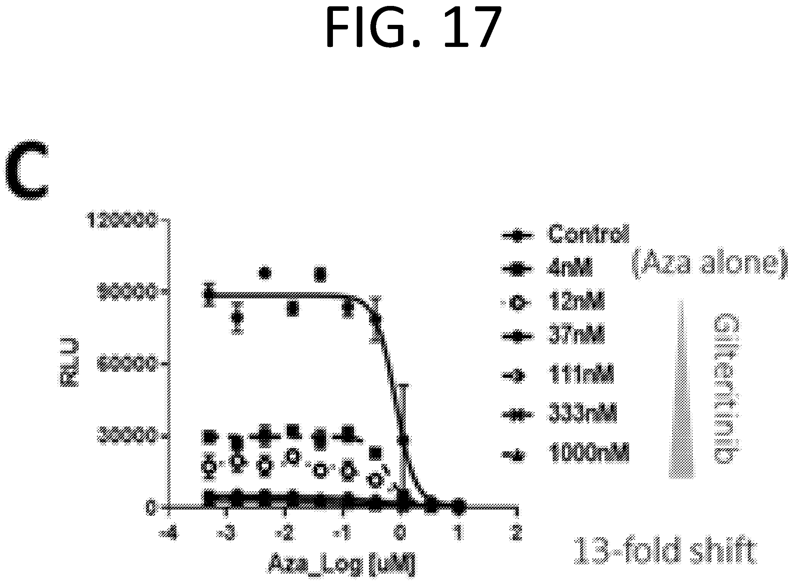

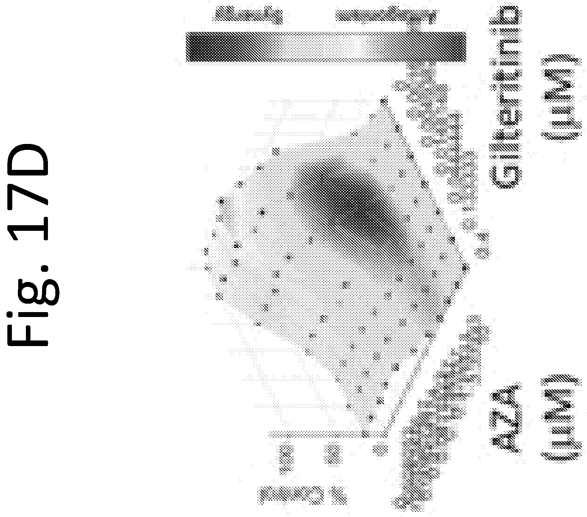

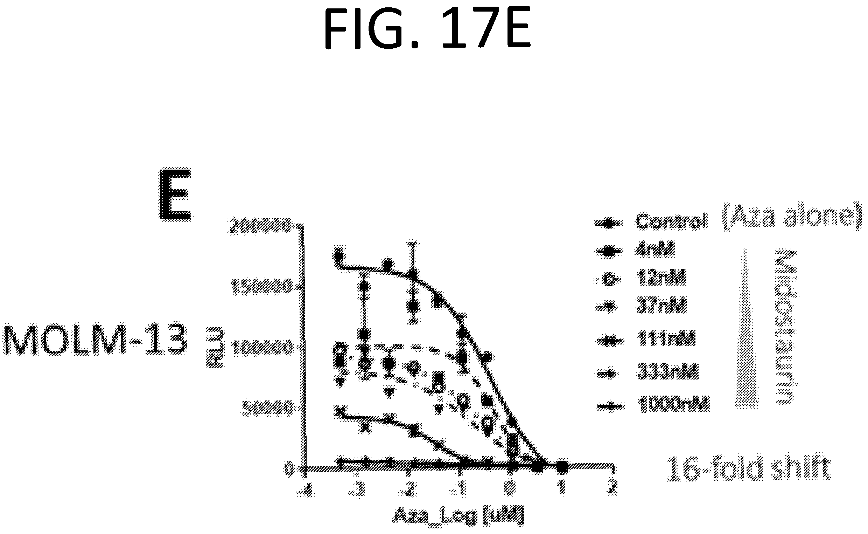

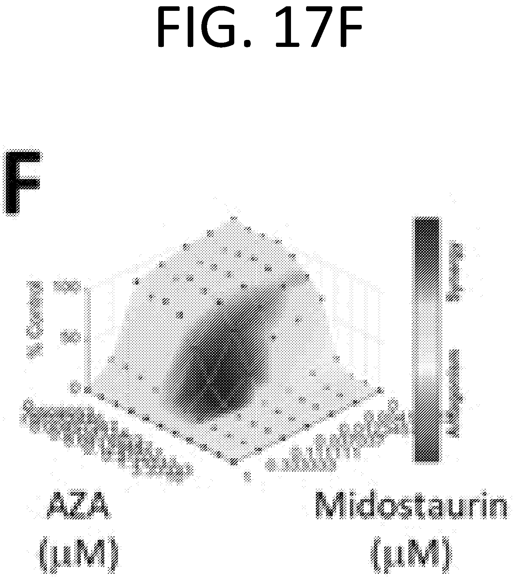

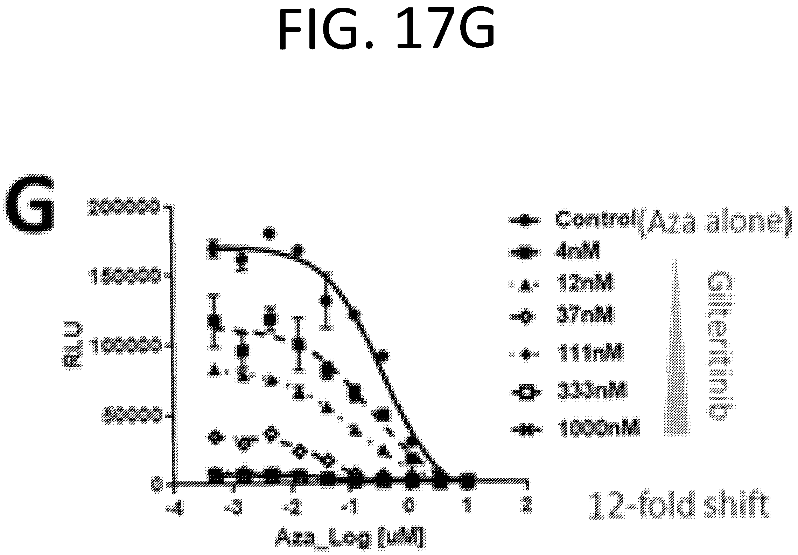

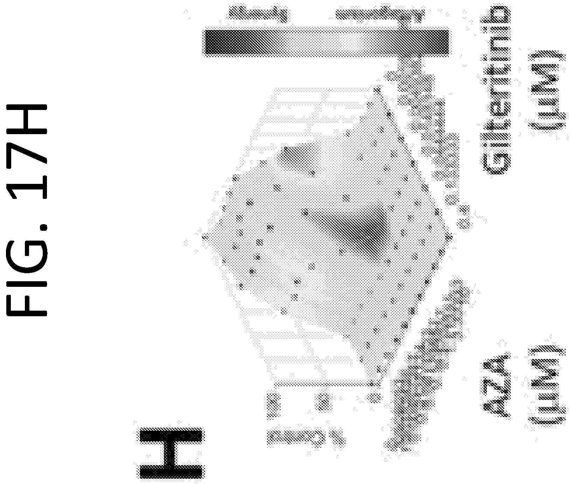

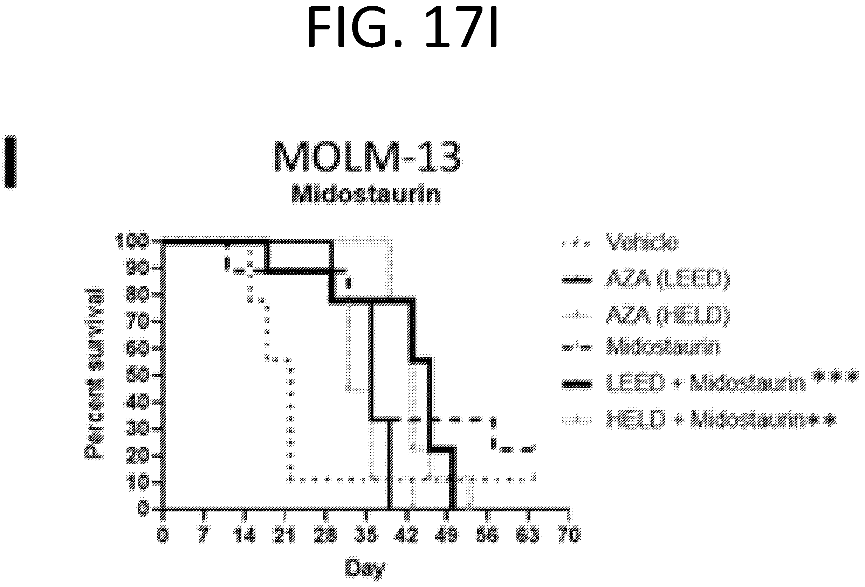

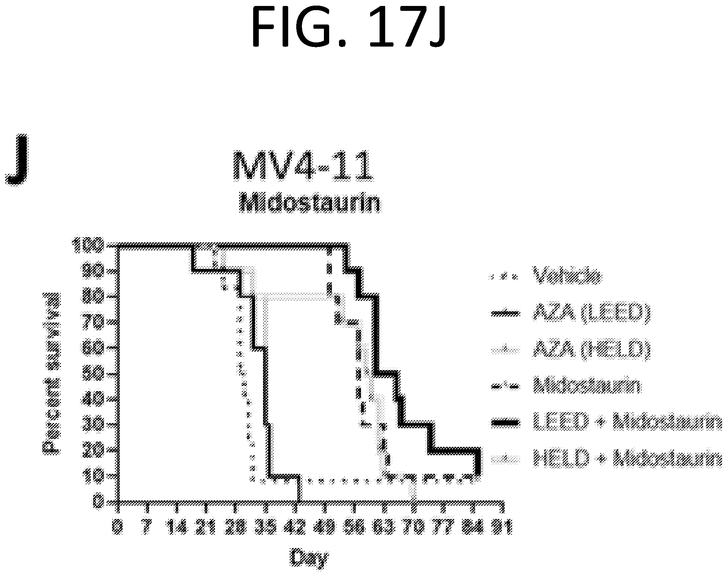

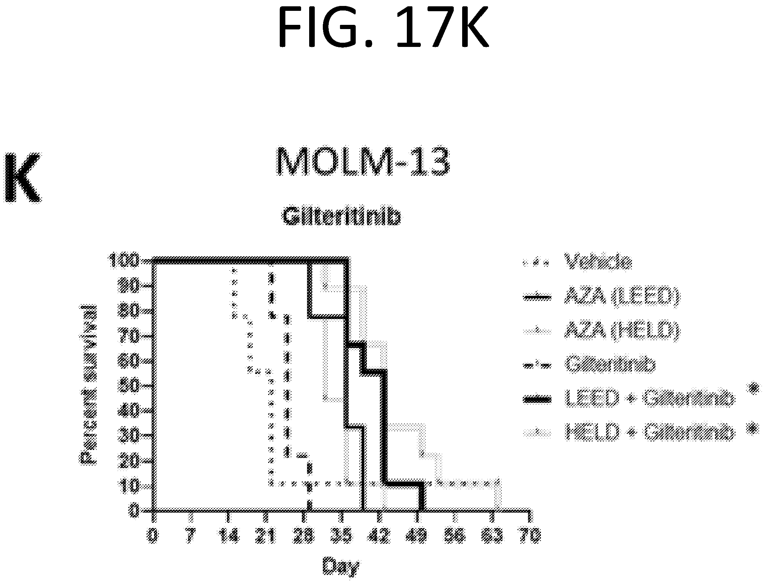

FIGS. 17A, 17B, 17C, 17D, 17E, 17F, 17G, 17H, 17I, 17J, and 17K depict the results of an investigation as to whether co-treatment with 5-azacytidine and FLT3 inhibitors have a synergistic effect in AML cells. FIGS. 17A-D show the results from experiments with MV4-11 cells. FIGS. 17E-H show the results from experiments with MOLM-13 cells. FIGS. 17A, 17B, 17E, and 17F show the results from treatment with 5-azacytidine and midostaurin. FIGS. 17C, 17D, 17G, and 17H show the results from treatment with 5-azacytidine and gilteritinib. Cells were treated with daily doses of 5-azacytidine on Day 1-3, and then treated with a FLT-3 inhibitor (midostaurin or gilteritinib) at Day 4. Cells were collected on Day 7 and cell viability was assessed by CellTiter-Glo^{®} assay. Midostaurin sensitized MV4-11 to 5-azacytidine (FIG. 17A) and showed synergistic activity with 5-azacytidine (FIG. 17B). Similar effects were observed in MV4-11 cells treated with 5-azacytidine and gilteritinib (FIGS. 17C and 17D), as well as in MOLM-13 cells treated with 5-azacytidine and midostaurin (FIGS. 17E and 17F) or gilteritinib (FIGS. 17G and 17H). FIG. 17I shows the results in MOLM-13 cells of percent survival (y-axis) vs day 1-70 for administration of vehicle, 5-azacytidine (LEED), 5-azacytidine (HELD), midostaurin, 5-azacytidine (LEED) and midostaurin, and 5-azacytidine (HELD) and midostaurin. FIG 17J shows the results in MV4-11 cells of percent survival (y-axis) versus day 1-91 for administration of vehicle, 5-azacytidine (LEED), 5-azacytidine (HELD), midostaurin, 5-azacytidine (LEED) and midostaurin, and 5-azacytidine (HELD) and midostaurin. FIG. 17K shows the results in MOLM-13 cells of percent survival (y-axis) versus day 1-70 for administration of vehicle, 5-azacytidine (LEED), 5-azacytidine (HELD), gilteritinib, 5-azacytidine (LEED) and gilteritinib, and 5-azacytidine (HELD) and gilteritinib.

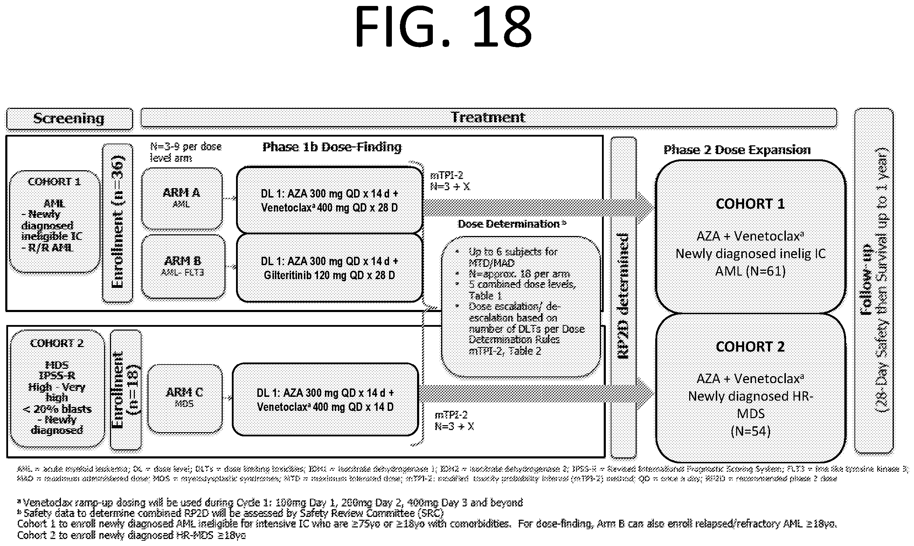

FIG. 18 depict the results the overall study design of Example 3.

### DETAILED DESCRIPTION

### I. Overview

The present disclosure is based in part on the surprising discovery that 5-azacytidine primes cancer cell lines for apoptosis in combination with other agents to treat cancer. In particular, 5-azacytidine was found to reduce levels of myeloid cell leukemia 1 (MCL-1), resulting in priming cancer cells to be responsive to FMS-like tyrosine kinase 3 (FLT3) or venetoclax activity. Low baseline levels of MCL-1 correlate with better synergistic responses to treating cancer, as it may be easier to reduce MCL-1 levels below a certain threshold when baseline levels are already reduced. Thus, it was surprisingly found that 5-azacytidine in combination with other therapeutic agents, such as FLT3 inhibitors or venetoclax, reduce MCL-1 levels more than each agent alone.

Another aspect of the methods disclosed herein is that the 5-azacytidine is administered orally in combination with other agents that are also administered orally. Thus, the 5-azacytidine oral dosing protocol in combination with other oral agents of the present disclosure produces a better patient experience as well as reduced risk of hospital infections, which can be particularly risky for cancer patients with compromised immune systems.

Yet another aspect of the methods disclosed herein is that the 5-azacytidine is given orally at a relatively low dose for a prolonged period of time (*e.g.* 1 mg/kg, once daily for fifteen days (QDx15) and 1 mg/kg, once daily for five days, three times (qdx 5x3)) in combination with one or more agents. Prior to the present disclosure, the rationale for administering 5-azacytidine by injection at relatively high doses (*e.g.,* 75 mg/m² subcutaneously or intravenously, once daily for seven days every four weeks) was because it was thought that high doses of chemotherapy agents, such as 5-azacytidine, would result in greater tumor cell death and therefore longer patient survival. The present disclosure is contrary to this "conventional wisdom." In particular, it was surprisingly found that relatively low oral doses of 5-azacytidine over a prolonged dosing period in combination with one or more agents result in therapeutic efficacy in treating AML and/or MDS. Although lower doses of 5-azacyitidine are administered in combination with other therapeutic agents, the combination therapy surprisingly provides therapeutic efficacy in treating AML and/or MDS.

Regarding cell differentiation, without being bound by theory, it is hypothesized that a decrease in 5-azacytidine dose may result in demethylation of cancer cells. Chemotherapy drugs triggering differentiation are thought to turn blast cells from the bone marrow into circulation and make them into functional cells and reduce their life span. Further, it is thought that exposure to 5-azacytidine would turn blast cells into terminally differentiated immune cells, resulting in killing tumor cells (hematopoietic differentiation pathway); once the blast cells are pushed down the differentiation lineage, they ultimately kill tumor cells. In sum, it is thought that the lower dose of 5-azacytidine given over a longer period of time with one or more other therapeutic agents, such as FLT3 inhibitors or venetoclax, results in greater differentiation and ultimately more significant tumor cell death.

Further, as detailed in the examples below, it was surprising found that in some instances, AML cell lines that were resistant to venetoclax activity were found to be responsive to ventoclax after treatment with the combination of 5-azacytidine and venetoclax, wherein the 5-azacytidine is administered first. Also, the combination of 5-azacytidine and a FLT3 inhibitor or venetoclax provided a synergistic effect and reduced MCL-1 protein levels more than each agent alone. It was also found that levels of MCL-1 expression correlated with the synergy of the 5-azacytidine-venetoclax combination (*e.g*., the lower the levels of MCL-1 expression, the greater the synergistic effect).

In one aspect, the present disclosure is directed to methods of treating a human having acute myeloid leukemia (AML) by administering to the human (i) a pharmaceutical composition comprising 5-azacytidine administered orally; and (ii) at least one additional therapeutic agent, namely venetoclax. Also disclosed herein are (i) pharmaceutical compositions comprising 5-azacytidine administered orally, and (ii) at least one additional therapeutic agent according to the claims for treating AML in a human.

In some embodiments, certain combinations work synergistically in the treatment of particular diseases or disorders, including, *e.g*., types of cancer and certain diseases and conditions associated with, or characterized by, undesired angiogenesis or abnormal cell proliferation.

Acute myeloid leukemia (AML), also known as acute myelogenous leukemia, is an aggressive, heterogeneous, myeloid malignancy. According to the American Cancer Society, AML is the most common type of leukemia diagnosed in adults and makes up 32% of all adult leukemia cases. It is estimated that approximately 19,940 people will be diagnosed with AML in 2020 in the United States (US) with 11,180 patients estimated to die from the disease. The disease is particularly difficult to treat in older adults who account for the majority of patients; thus, the five-year overall survival is only approximately 29%. National Cancer Institute, SEER Cancer Stat Facts: Leukemia - Acute Myeloid Leukemia (AML), https://seer.cancer.gov/statfacts/html/amyl.html (accessed 10 June 2020). Since the 1970s, initial standard therapy, for those fit enough to receive it, consisted of the '7 + 3' regimen, which includes seven days of continuous infusion cytarabine and three days of an anthracycline. Rai et. al. Blood 1981:58: 1203-1212. Over the next 35 years, a number of clinical trials attempting to augment the safety and efficacy of AML treatment have been performed with little change in the standard of care. However, recent data detailing the molecular ontogeny of AML have elucidated causal pathways which have led to efforts to develop targeted drug therapies. E. Winer and R. Stone, Ther. Adv. Hematol., 10:PMC6624910 (July 2019).

There is a long felt need for the combination treatments described herein, as AML has a high rate of relapse. Additionally, relapsed and refractory AML is a disease that is very difficult to treat and is likely driven by multiple abnormal signaling pathways that give the leukemic cell an advantage in overcoming any single pathway that is being inhibited. Thus, successful combination treatments are highly desirable to treat AML patients.

In one aspect of the methods of treatment described herein, the patient to be treated is about age 60 or older. In another aspect of the methods of treatment described herein, the patient to be treated is about age 65 or older, about age 70 or older, about age 75 or older, or about age 80 or older. In yet another aspect, the patient is a relapsed AML patient. In another aspect, the patient is a refractory AML patient. The patient to be treated can also be under about age 60, under about age 55, under about age 50, under about age 45, or under about age 40. In other aspects, the patient to be treated has FLT3 mutations, either FLT3-ITD or FLT3-TKD. In some aspects, the patient to be treated has a recurrent AML mutation. Exemplary AML mutations include, but are not limited to, FMS-related tyrosine kinase 3 (FLT3), Kirsten rat sarcoma viral oncogene homolog (KRAS), neuroblastoma RAS viral (V-Ras) oncogene homolog (NRAS), proto-oncogene c-Kit (KIT), protein tyrosine phosphatase non-receptor type 11 (PTPN11), neurofibromin 1 (NF1), DNA methyltransferase 3A (DNMT3A), isocitrate dehydrogenase 1 (IDH1), isocitrate dehydrogenase 2 (IDH2), ten-eleven translocation-2 (TET2), additional sex comb-like 1 (ASXL1), enhancer of zeste homolog 2 (EZH2), mixed-lineage leukemia 1/histone-lysine N-methyltransferase 2A (MLL/KMT2A), nucleophosmin (NPM1), CCAAT enhancer binding protein alpha (CEBPA), runt-related transcription factor 1 (RUNX1), GATA-binding factor 2 (GATA2), tumor protein p53 (TP53), serine and arginine rich splicing factor 2 (SRSF2), U2 small nuclear RNA auxiliary factor 1 (U2AF 1), splicing factor 3b subunit 1 (SF3B 1), zinc finger (CCCH type), RNA-binding motif and serine/arginine rich 2 (ZRSR2), RAD21 cohesin complex component (RAD21), stromal antigen 1 (STAG1), stromal antigen 2 (STAG2), structural maintenance of chromosomes 1A (SMC1A), and structural maintenance of chromosomes protein 3 (SMC3).

In another aspect, the present disclosure is directed to methods of treating a human having myelodysplastic syndrome (MDS) by administering to the human (i) a pharmaceutical composition comprising 5-azacytidine administered orally; and (ii) at least one additional therapeutic agent, namely venetoclax. Also disclosed herein are (i) pharmaceutical compositions comprising 5-azacytidine administered orally, and (ii) at least one additional therapeutic agent for treating MDS in a human.

In some embodiments, the MDS is a higher risk or high risk MDS. Higher-risk MDS for this disclosure is defined as High or Very High risk according to the Revised International Scoring System (IPSS-R) (Voso M.T., et. al., J Clin Oncol. 2013; 31(21): 2671-2677; and Greenberg P.L. et al., Blood. 2012, 120(12): 2454-2465), where these patients have median survival of 1.6 and 0.8 years respectively.

### A. Overview of Experimental and Clinical Study Protocols

Example 2 details a clinical study analyzing the relationships of drug exposure with efficacy, safety, pharmacodynamics, including establishment of a maximum tolerated dose (MTD) or a maximum administered dose (MAD) in AML patients. The minimal residual disease in blood and/or bone marrow following treatment will also be determined. The study population consists of AML patients who are in first relapse, refractory to 1 or 2 standard induction treatments, or newly diagnosed AML patients who are not candidates to receive intensive IC. The drug combinations to be tested include oral 5-azacytidine when given in combination with ivosidenib, enasidenib, venetoclax, or an FLT3 inhibitor, such as gilteritinib (in AML patients with a FLT3 ITD or TKD mutation). In the present invention the additional therapeutic agent is venetoclax.

Isocitrate dehydrogenase (IDH) is a critical enzyme in the citric acid cycle. Mutated forms of IDH produce high levels of the (R)-enantiomer of 2-hydroxyglutarate (R-2-HG) and can contribute to the growth of tumors. IDH1 catalyzes this reaction in the cytoplasm, while IDH2 catalyzes this reaction in mitochondria. Ivosidenib and enasidenib are IDH inhibitors.

Ivosidenib (Tibsovo^{®}) is a small molecule inhibitor of IDH1. In tumors from patients diagnosed with glioma, Acute Myeloid Leukemia (AML), cholangiocarcinoma, and chondrosarcoma, somatic mutations in the conserved active site of isocitrate dehydrogenase (IDH) 1 and 2 are observed. With these new mutations, these enzymes exhibit new, neomorphic behavior, which results in the reduction of α-ketoglutarate to the oncometabolite R-2-hydroxyglutarate. Ivosidenib competitively inhibits α-ketoglutarate-dependent enzymes, ultimately leading to epigenetic alterations and impaired hematopoietic differentiation.

In *in vitro* studies, ivosidenib showed non-competitive inhibitory behavior towards the alpha-ketoglutarate (α-KG) substrate and to the NADPH cofactor. This is what is believed to lead to ivonsidenib being a rapid equilibrium inhibitor of the mutated isocitrate dehydrogenase 1 (mIDH1)-R132H homodimer.

Enasidenib (Idhifa^{®}) is a small molecule inhibitor of the isocitrate dehydrogenase 2 (IDH2) gene. As noted above, mutated forms of IDH produce high levels of R-2-HG, with IDH1 catalyzing this reaction in the cytoplasm and IDH2 catalyzing this reaction in mitochondria. Enasidenib disrupts this cycle by decreasing total (R)-2-HG levels in the mitochondria

Venetoclax (Venclexta^{®} and Venclyxto^{®}) is a BH3 (Bcl-2 homology domain 3)-mimetic as it blocks the anti-apoptotic B-cell lymphoma-2 (Bcl-2) protein, leading to programmed cell death of chronic lymphocytic leukemia (CLL) cells. Overexpression of Bcl-2 in some lymphoid malignancies has sometimes shown to be linked with increased resistance to chemotherapy.

FLT3 inhibitors are tyrosine kinase inhibitors (TKI). Like other tyrosine kinase inhibitors, FLT3 inhibitors compete for the adenosine triphosphate (ATP) binding site in the active domain of the kinase, which inhibits the ability of the protein to be phosphorylated, and subsequently decreases the activity of that protein. FLT3 mutations are one of the most common findings in patients with acute myeloid leukemia (AML). The FLT3/ITD gene is found in approximately 30% of patients with AML with normal cytogenetics. The FLT3 gene is expressed mainly in human hematopoietic progenitors and dendritic cells and plays key roles in leukemia cell proliferation, differentiation, and survival. Constitutive activation of the FLT3/ITD gene triggers multiple downstream signaling cascades, such as STAT5, RAS, MEK, and PI3K/AKT pathways, and ultimately causes suppression of apoptosis and differentiation of leukemic cells, including dysregulation of leukemic cell proliferation. The FLT3 inhibitors evaluated include midostaurin (Rydapt^{®}) and gilteritinib (Xospata^{®}). Midostaurin is a semi-synthetic derivative of staurosporine, an alkaloid from the bacterium *Streptomyces staurosporeus,* and is active against oncogenic CD135 (FMS-like tyrosine kinase 3 receptor, FLT3). Gilteritinib acts as an inhibitor of AXL receptor tyrosine kinase.

Example 3 details a Phase 1b/2 clinical study evaluating the safety, tolerability, and preliminary efficacy of oral 5-azacytidine in combination with biomarker directed therapies in patients with acute myeloid leukemia (AML) or higher-risk myelodysplastic syndromes (HR-MDS). The study population consists of patients with newly diagnosed AML not eligible for intensive induction chemotherapy and relapsed/refractory (R/R) AM and treatment of subjects with primary or secondary MDS who are Revised International Prognostic Scoring System (IPSS-R) high and very high risk. The drug combinations to be tested include oral 5-azacytidine when given in combination with venetoclax or gilteritinib.

### B. 5-Azacytidine

5-Azacytidine (National Service Center designation NSC-102816; CAS Registry Number 320-67-2) is also known as azacitidine, abbreviated as AZA, or 4-amino-1-B-D-ribofuranosyl-1,3,5-triazin-2(1H)-one. The marketed product VIDAZA^{®} (5-azacytidine for injection) contains 5-azacytidine, and is for subcutaneous or intravenous use. 5-Azacytidine is a pyrimidine nucleoside analog of cytidine. 5-Azacytidine has the following structure:

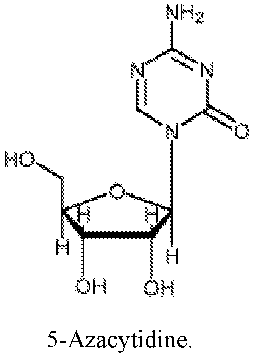

After its incorporation into replicating DNA, 5-azacytidine forms a covalent complex with DNA methyltransferases. DNA methyltransferases are responsible for de novo DNA methylation and for reproducing established methylation patterns in daughter DNA strands of replicating DNA. Inhibition of DNA methyltransferases by 5-azacytidine leads to DNA hypomethylation, thereby restoring normal functions to morphologically dysplastic, immature hematopoietic cells and cancer cells by re-expression of genes involved in normal cell cycle regulation, differentiation and death. The cytotoxic effects of these cytidine analogs cause the death of rapidly dividing cells, including cancer cells, that are no longer responsive to normal cell growth control mechanisms. 5-azacytidine also incorporates into RNA. The cytotoxic effects of 5-azacytidine may result from multiple mechanisms, including inhibition of DNA, RNA and protein synthesis, incorporation into RNA and DNA, and activation of DNA damage pathways.

Injectable 5-azacytidine has been tested in clinical trials and showed significant antitumor activity, such as, for example, in the treatment of myelodysplastic syndromes (MDS), acute myelogenous leukemia (AML), chronic myelogenous leukemia (CML), acute lymphocytic leukemia (ALL), and non Hodgkin's lymphoma (NHL). *See, e.g.,* Aparicio et al., Curr. Opin. Invest. Drugs 3(4): 627-33 (2002).

5-Azacytidine is approved for subcutaneous (SC) or intravenous (IV) administration to treat patients with the following French-American-British (FAB) myelodysplastic syndrome subtypes: refractory anemia (RA) or refractory anemia with ringed sideroblasts (if accompanied by neutropenia or thrombocytopenia or requiring transfusions), refractory anemia with excess blasts (RAEB), refractory anemia with excess blasts in transformation (RAEB-T), and chronic myelomonocytic leukemia (CMMoL). Oral dosing has been studied in clinical trials, such as NCT00761722, NCT01519011, NCT00528982, and NCT01757535. Oral formulations and methods of treatment using 5-azacytidine are disclosed in US Patent No, 8,846,628, which is incorporated by reference in its entirety.

In some embodiments, 5-azacytidine is administered orally. In some embodiments, 5-azacytidine is administered in the form of a capsule or a tablet. In some embodiments, the tablet is a non-enteric-coated tablet. In some embodiments, the 5-azacytidine is administered at a dose of about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg or about 600 mg orally. In some embodiments, the 5-azacytidine is administered at a dose of about 200 to about 300 mg orally. In some embodiments, the 5-azacytidine is administered at a dose of about 200 mg. In some embodiments, the 5-azacytidine is administered at a dose of about 300 mg. In some embodiments, the 5-azacytidine is administered at a dose of 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 310 mg, 320 mg, 330 mg, 340 mg, 350 mg, 360 mg, 370 mg, 380 mg, 390 mg, 400 mg, 410 mg, 420 mg, 430 mg, 440 mg, 450 mg, 460 mg, 470 mg, 480 mg, 490 mg, 500 mg, 510 mg, 520 mg, 530 mg, 540 mg, 550 mg, 560 mg, 570 mg, 580 mg or 600 mg orally. In some embodiments, the 5-azacytidine is administered at a dose of 200 to 300 mg orally. In some embodiments, the 5-azacytidine is administered at a dose of 200 mg. In some embodiments, the 5-azacytidine is administered at a dose of 300 mg. In some embodiments, 5-azacytidine is administered daily orally for the first seven, fourteen, or twenty-one days of a 28-day cycle. In some embodiments, 5-azacytidine is administered daily orally for the first fourteen days of a 28-day cycle. In some embodiments, 5-azacytidine administered to the subject once per day. In some embodiments, 5-azacytidine administered to the subject two times per day.

In some embodiments, the 5-azacytidine is administered orally at a dose of about 200 mg per day for 14 days in a 28-day cycle. In some embodiments, the 5-azacytidine is administered orally at a dose of 200 mg per day for 14 days in a 28-day cycle. In some embodiments, the 5-azacytidine is administered orally at a dose of about 300 mg per day for 14 days in a 28-day cycle. In some embodiments, the 5-azacytidine is administered orally at a dose of 300 mg per day for 14 days in a 28-day cycle. In some embodiments, the 5-azacytidine is administered orally at a dose of about 200 mg per day for 21 days in a 28-day cycle. In some embodiments, the 5-azacytidine is administered orally at a dose of 200 mg per day for 21 days in a 28-day cycle. In some embodiments, the 5-azacytidine is administered orally at a dose of about 300 mg per day for 21 days in a 28-day cycle. In some embodiments, the 5-azacytidine is administered orally at a dose of 300 mg per day for 21 days in a 28-day cycle.

In some embodiments, the 5-azacytidine is administered orally daily for 1, 2, 3, 4, 5, 6, 7, 8, 9, 10, 11, 12, 13, 14, or greater than 14 days, optionally followed by a treatment dosing holiday of 1, 2, 3, 4, 5, 6, 7, 8, 9, 10, 11, 12, 13, 14, or greater than 14 days. In some embodiments, the 5-azacytidine is administered orally daily for 14 or more days, optionally followed by a treatment dosing holiday of 7 or more days. In some embodiments, the 5-azacytidine is administered orally for 21 or more days, optionally followed by a treatment dosing holiday of 7 or more days. In some embodiments, the 5-azacytidine is administered orally for 14 days, optionally followed by a treatment dosing holiday of 14 days. In some embodiments, the 5-azacytidine is administered orally for 21 or more days, followed by a treatment dosing holiday of 7 or more days. In some embodiments, the 5-azacytidine is administered orally for 14 days, followed by a treatment dosing holiday of 14 days.

In some embodiments, the 5-azacytidine is administered orally at a dose of about 300 mg daily for 14 days, followed by a treatment dosing holiday of 14 days. In some embodiments, the 5-azacytidine is administered orally at a dose of 300 mg daily for 14 days, followed by a treatment dosing holiday of 14 days. In some embodiments, the 5-azacytidine is administered orally at a dose of about 200 mg daily for 14 days, followed by a treatment dosing holiday of 14 days. In some embodiments, the 5-azacytidine is administered orally at a dose of 200 mg daily for 14 days, followed by a treatment dosing holiday of 14 days. In some embodiments, the 5-azacytidine is administered orally at a dose of about 300 mg daily for 21 days, followed by a treatment dosing holiday of 7 days. In some embodiments, the 5-azacytidine is administered orally at a dose of 300 mg daily for 21 days, followed by a treatment dosing holiday of 7 days. In some embodiments, the 5-azacytidine is administered orally at a dose of about 200 mg daily, followed by a treatment dosing holiday of 7 days. In some embodiments, the 5-azacytidine is administered orally at a dose of 200 mg daily, followed by a treatment dosing holiday of 7 days.

In some embodiments, the 5-azacytidine is administered orally using a treatment cycle comprising administration of 5-azacytidine per day for 7 days in a 28-day cycle. In some embodiments, the 5-azacytidine is administered orally using a treatment cycle comprising administration of 5-azacytidine per day for 14 days in a 28-day cycle. In some embodiments, the 5-azacytidine is administered orally using a treatment cycle comprising administration of 5-azacytidine per day for 21 days in a 28-day cycle.

5-azacytidine exerts effects on cell viability and epigenetic reprogramming of cells. Taylor and Jones, Cell 20(1):85-93 (1980). At high doses, 5-azacytidine is thought to exercise a predominantly acute cytotoxic effect (Khan et al., Experimental Hematology 36(2): 149-57, 2008), while at low doses it inhibits clonogenicity of tumor cells though differentiation (Tsai et al., Cancer Cell, 21(3): 430-46, 2012).

The marketed product VIDAZA^{®}, the injectable formulation of 5-azacytidine, is administered at relatively higher doses and for shorter duration compared to the oral, non-enteric coated formulation of 5-azacytidine as described in US. Patent No. 8,846,628, including CC-486. Clinical studies revealed that CC-486 induces more sustainable demethylative effects as compared to VIDAZA^{®} (Laille et al., PLOSOne 10(8):e0135520, 2015), although cumulative exposures of 14 or 21 day regimens of CC-486 are lower than VIDAZA^{®}, administered for 5 days (Garcia-Manero et al., Leukemia 30(4):889-96, 2016).

To examine differences in cytotoxic and epigenetic effects as a function of duration of exposure to 5-azacytidine, high exposure, limited duration (HELD) administration of injectable 5-azacytidine was compared with low exposure, extended duration (LEED) administration of 5-azacytidine administered orally. To model injectable and oral azacytidine dosing in non-clinical systems, the total 5-azacytidine exposure was held constant while varying the duration of exposure. In some embodiments, to model the oral administration of 5-azacytidine, the 5-azacytidine was delivered at a low exposure for extended duration (LEED), at a dose of 1 mg/kg, once daily for fifteen days (QDx15). To model the same cumulative dose by intravenous or subcutaneous administration of 5-azacytidine, the 5-azacytidine was administered at a **h**igh **e**xposure for a **l**imited **d**uration (HELD), at a dose of 3 mg/kg, once daily for five days (QDx5).

In some embodiments, LEED administration of 5-azacytidine in combination with other agents provides a sustained pharmacodynamic effect and/or improved patient compliance. A sustained pharmacodynamic effect may include any change elicited by 5-azacytidine, which includes for example MCL-1 degradation, and/or changes in ATF3 or SCD gene expression. In some embodiments, LEED of 5-azacyitidine in combination with other agents provides a reduction in global DNA methylation (e.g., due to increased nucleic acid incorporation) that sustained through the end of the treatment cycle (*i.e.,* a 28-day cycle) compared to HELD of 5-azacyitidine in combination with other agents. In some embodiments, LEED of 5-azacyitidine in combination with other agents provides a differentiation maker upregulation that peaks at Day 21 of a 28-day cycle and has a cell death that is characterized by a gradual loss of viability through Day 28 of a 28-day cycle. In some embodiments, HELD of 5-azacyitidine in combination with other agents provides a differentiation marker upregulation that peaks at Day 7 of a 28-day cycle and has a cell death that is characterized by a peak at Day 14 followed by recovery in a 28-day cycle. In some embodiments, LEED of 5-azacyitidine in combination with other agents provides a higher expression of myeloid differentiation markers, which include but are not limited to CD11b, CD14, CD86, HLA-DR and MERTK, that is sustained through a treatment cycle (*i.e.,* a 28-day cycle) compared to HELD of 5-azacyitidine in combination with other agents. In some embodiments, LEED of 5-azacyitidine in combination with other agents provides more pronounced epigenetic changes and more extensive differentiation compared to HELD of 5-azacyitidine in combination with other agents.

### II. Pharmaceutical Formulations

### A. Overview

Embodiments herein encompass pharmaceutical formulations and compositions comprising 5-azacytidine, and optionally a permeation enhancer, wherein the formulations and compositions are prepared for oral administration. In a particular embodiment, the formulations and compositions are prepared for release of 5-azacytidine substantially in the stomach. In specific embodiments, 5-azacytidine and the pharmaceutical formulations and compositions are used for treating diseases and disorders associated with abnormal cell proliferation, wherein 5-azacytidine, the formulations and compositions are prepared for oral administration, preferably for release of 5-azacytidine substantially in the stomach. Particular embodiments relate to the use 5-azacytidine for the preparation of pharmaceutical formulations and compositions for treating particular medical indications, as provided herein. The pharmaceutical formulations and compositions including 5-azacytidine provided herein are intended for oral delivery of 5-azacytidine to subjects in need thereof. Oral delivery formats include, but are not limited to, tablets, capsules, caplets, solutions, suspensions, and syrups.

Particular embodiments herein provide solid oral dosage forms that are tablets or capsules. In certain embodiments, the formulation is a tablet containing 5-azacytidine. In certain embodiments, the formulation is a capsule containing 5-azacytidine. In certain embodiments, the tablets or capsules provided herein optionally comprise one or more excipients, such as, for example, glidants, diluents, lubricants, colorants, disintegrants, granulating agents, binding agents, polymers, and coating agents. In certain embodiments, embodiments herein encompass the use of 5-azacytidine, for the preparation of a pharmaceutical composition for treating a disease associated with abnormal cell proliferation, wherein the composition is prepared for oral administration.

### Pharmacokinetic Characteristics of Certain Dosage Forms Provided Herein

In certain embodiments, the formulations including 5-azacytidine effect an immediate release of the active pharmaceutical ingredient (API) upon oral administration. In particular embodiments, the formulations including 5-azacytidine comprise a therapeutically effective amount of 5-azacytidine (and, optionally, one or more excipients) and effect an immediate release of the API upon oral administration.

In certain embodiments, the formulations including 5-azacytidine release the API substantially in the stomach upon oral administration. In certain embodiments, the formulations effect an immediate release of 5-azacytidine upon oral administration. In certain embodiments, the formulations further comprise a drug release controlling component which is capable of releasing 5-azacytidine substantially in the stomach. In certain embodiments, the formulations optionally further comprises a drug release controlling component, wherein the drug release controlling component is adjusted such that the release of 5-azacytidine occurs substantially in the stomach. In particular embodiments, the drug release controlling component is adjusted such that the release of 5-azacytidine is immediate and occurs substantially in the stomach. In particular embodiments, the drug release controlling component is adjusted such that the release of 5-azacytidine is sustained and occurs substantially in the stomach. In certain embodiments, the formulation of 5-azacytidine releases the API substantially in the stomach, and, subsequently, releases the remainder of the API in the intestine upon oral administration.

Methods by which skilled practitioners can assess the oral bioavailability of a drug formulation in a subject are known in the art. Such methods, include, for example, comparing various pharmacokinetic parameters, such as, but not limited to, maximum plasma concentration ("Cmax"), time to maximum plasma concentration ("Tmax"), or area-under-the-curve ("AUC") determinations.

Particular embodiments herein provide pharmaceutical formulations (*e.g*., immediate release oral formulations and/or formulations that release the API substantially in the stomach) including 5-azacytidine that achieve a particular AUC value (*e.g.,* AUC(0-t) or AUC(0-∞)) in the subject (*e.g*., human) to which the formulation is orally administered. Particular embodiments provide oral formulations that achieve an AUC value of at least 25 ng-hr/mL, at least 50 ng-hr/mL, at least 75 ng-hr/mL, at least 100 ng-hr/mL, at least 150 ng-hr/mL, at least 200 ng-hr/mL, at least 250 ng-hr/mL, at least 300 ng-hr/mL, at least 350 ng-hr/mL, at least 400 ng-hr/mL, at least 450 ng-hr/mL, at least 500 ng-hr/mL, at least 550 ng-hr/mL, at least 600 ng-hr/mL, at least 650 ng-hr/mL, at least 700 ng-hr/mL, at least 750 ng-hr/mL, at least 800 ng-hr/mL, at least 850 ng-hr/mL, at least 900 ng-hr/mL, at least 950 ng-hr/mL, at least 1000 ng-hr/mL, at least 1100 ng-hr/mL, at least 1200 ng-hr/mL, at least 1300 ng-hr/mL, at least 1400 ng-hr/mL, at least 1500 ng-hr/mL, at least 1600 ng-hr/mL, at least 1700 ng-hr/mL, at least 1800 ng-hr/mL, at least 1900 ng-hr/mL, at least 2000 ng-hr/mL, at least 2250 ng-hr/mL, or at least 2500 ng-hr/mL. In particular embodiments, the AUC determination is obtained from a time-concentration pharmacokinetic profile obtained from the blood samples of human patients following dosing.

Particular embodiments herein provide pharmaceutical formulations (*e.g*., immediate release oral formulations and/or formulations that release the API substantially in the stomach) including 5-azacytidine that achieve a particular maximum plasma concentration ("Cmax") in the subject to which the formulation is orally administered. Particular embodiments provide oral formulations that achieve a Cmax of 5-azacytidine of at least 25 ng/mL, at least 50 ng/mL, at least 75 ng/mL, at least 100 ng/mL, at least 150 ng/mL, at least 200 ng/mL, at least 250 ng/mL, at least 300 ng/mL, at least 350 ng/mL, at least 400 ng/mL, at least 450 ng/mL, at least 500 ng/mL, at least 550 ng/mL, at least 600 ng/mL, at least 650 ng/mL, at least 700 ng/mL, at least 750 ng/mL, at least 800 ng/mL, at least 850 ng/mL, at least 900 ng/mL, at least 950 ng/mL, at least 1000 ng/mL, at least 1100 ng/mL, at least 1200 ng/mL, at least 1300 ng/mL, at least 1400 ng/mL, at least 1500 ng/mL, at least 1600 ng/mL, at least 1700 ng/mL, at least 1800 ng/mL, at least 1900 ng/mL, at least 2000 ng/mL, at least 2250 ng/mL, or at least 2500 ng/mL. Particular embodiments herein provide pharmaceutical formulations (*e.g*., immediate release oral formulations and/or formulations that release the API substantially in the stomach) including 5-azacytidine that achieve a particular time to maximum plasma concentration ("Tmax") in the subject to which the formulation is orally administered. Particular embodiments provide oral formulations that achieve a Tmax of 5-azacytidine of less than 10 minutes, less than 15 minutes, less than 20 minutes, less than 25 minutes, less than 30 minutes, less than 35 minutes, less than 40 minutes, less than 45 minutes, less than 50 minutes, less than 55 minutes, less than 60 minutes, less than 65 minutes, less than 70 minutes, less than 75 minutes, less than 80 minutes, less than 85 minutes, less than 90 minutes, less than 95 minutes, less than 100 minutes, less than 105 minutes, less than 110 minutes, less than 115 minutes, less than 120 minutes, less than 130 minutes, less than 140 minutes, less than 150 minutes, less than 160 minutes, less than 170 minutes, less than 180 minutes, less than 190 minutes, less than 200 minutes, less than 210 minutes, less than 220 minutes, less than 230 minutes, or less than 240 minutes In particular embodiments, the Tmax value is measured from the time at which the formulation is orally administered.

### Design of Certain Dosage Forms Provided Herein

Provided herein are dosage forms designed to maximize the absorption and/or efficacious delivery of 5-azacytidine, upon oral administration, *e.g*., for release substantially in the stomach. Accordingly, certain embodiments herein provide a solid oral dosage form of 5-azacytidine, such as, for example, 5-azacytidine, using pharmaceutical excipients that effect immediate release of the API upon oral administration, *e.g*., substantially in the stomach. Particular immediate release formulations comprise a specific amount of 5-azacytidine and optionally one or more excipients. In certain embodiments, the formulation is an immediate release tablet or an immediate release capsule (such as, *e.g.,* an HPMC capsule).

Provided herein are methods of making the formulations provided herein including 5-azacytidine provided herein (*e.g*., immediate release oral formulations and/or formulations that release the API substantially in the stomach). In particular embodiments, the formulations provided herein are prepared using conventional methods known to those skilled in the field of pharmaceutical formulation, as described, *e.g.,* in pertinent textbooks. *See, e.g.,* REMINGTON, THE SCIENCE AND PRACTICE OF PHARMACY, 20th Edition, Lippincott Williams & Wilkins, (2000); ANSEL et al., PHARMACEUTICAL DOSAGE FORMS AND DRUG DELIVERY SYSTEMS, 7th Edition, Lippincott Williams & Wilkins, (1999); GIBSON, PHARMACEUTICAL PREFORMULATION AND FORMULATION, CRC Press (2001).

In certain embodiments, the formulation is a tablet, wherein the tablet is manufactured using standard, art-recognized tablet processing procedures and equipment. In certain embodiments, the method for forming the tablets is direct compression of a powdered, crystalline and/or granular composition including 5-azacytidine, alone or in combination with one or more excipients, such as, for example, carriers, additives, polymers, or the like. In certain embodiments, as an alternative to direct compression, the tablets are prepared using wet granulation or dry granulation processes. In certain embodiments, the tablets are molded rather than compressed, starting with a moist or otherwise tractable material. In certain embodiments, compression and granulation techniques are used.

In certain embodiments, the compressed tablet of 5-azacytidine is film-coated. In some embodiments, the film-coated tablets are compressed tablets coated with a thin layer of a polymer capable of forming a skin-like film over the tablet. The film is usually colored and has the advantage to be more durable, less bulky, and less time-consuming to apply. By its composition, the coating may be designed to rupture and expose the core tablet at the desired location within the gastrointestinal tract. The film-coating process, which places a thin skin-tight coating of a plastic-like material over the compressed tablet, may produce coated tablets having essentially the same weight, shape, and size as the originally compressed tablet. In some embodiments, the film-coating is colored to make the tablets attractive and distinctive. In some embodiments, the film-coating solutions are non-aqueous or aqueous. In particular embodiments, the non-aqueous solutions are optionally contain one or more of the following types of materials to provide the desired coating to the tablets: (1) a film former capable of producing smooth, thin films reproducible under conventional coating conditions and applicable to a variety of tablet shapes, such as, for example, cellulose acetate phthalate; (2) an alloying substance providing water solubility or permeability to the film to ensure penetration by body fluids and therapeutic availability of the drug, such as, for example, polyethylene glycol; (3) a plasticizer to produce flexibility and elasticity of the coating and thus provide durability, such as, for example, castor oil; (4) a surfactant to enhance spreadability of the film during application, such as, for example, polyoxyethylene sorbitan derivatives; (5) opaquants and colorants to make the appearance of the coated tablets attractive and distinctive, such as, for example, titanium dioxide as an opaquant, and FD&C or D&C dyes as a colorant; (6) sweeteners, flavors, or aromas to enhance the acceptability of the tablet to the subject, such as, for example, saccharin as sweeteners, and vanillin as flavors and aromas; (7) a glossant to provide a luster to the tablets without a separate polishing operation, such as, for example, beeswax; and (8) a volatile solvent to allow the spread of the other components over the tablets while allowing rapid evaporation to permit an effective yet speedy operation, such as, for example, alcohol-acetone mixture. In certain embodiments, an aqueous film-coating formulation contains one or more of the following: (1) film-forming polymer, such as, for example, cellulose ether polymers as hydroxypropyl methyl-cellulose, hydroxypropyl cellulose, and methyl-cellulose; (2) plasticizer, such as, for example, glycerin, propylene glycol, polyethylene glycol, diethyl phthalate, and dibutyl subacetate; (3) colorant and opacifier, such as, for example, FD&C or D&C lakes and iron oxide pigments; or (4) vehicle, such as, for example, water.

In certain embodiments, the pharmaceutical formulation is an immediate release tablet of 5-azacytidine. In certain embodiments, the immediate release tablet is designed, *e.g.,* to disintegrate and release the API absent of any special rate-controlling features, such as special coatings and other techniques.

In certain embodiments, the pharmaceutical formulations provided herein contain 5-azacytidine and, optionally, one or more excipients to form a "drug core." Optional excipients include, *e.g*., diluents (bulking agents), lubricants, disintegrants, fillers, stabilizers, surfactants, preservatives, coloring agents, flavoring agents, binding agents, excipient supports, glidants, permeation enhancement excipients, plasticizers and the like, *e.g.,* as known in the art.

One or more diluents may be used, *e.g.,* to increase bulk so that a practical size tablet is ultimately provided. Diluents also include, *e.g*., ammonium alginate, calcium carbonate, calcium phosphate, calcium sulfate, cellulose acetate, compressible sugar, confectioner's sugar, dextrates, dextrin, dextrose, erythritol, ethylcellulose, fructose, fumaric acid, glyceryl palmitostearate, isomalt, kaolin, lacitol, lactose, mannitol, magnesium carbonate, magnesium oxide, maltodextrin, maltose, medium-chain triglycerides, microcrystalline cellulose, microcrystalline silicified cellulose, powered cellulose, polydextrose, polymethylacrylates, simethicone, sodium alginate, sodium chloride, sorbitol, starch, pregelatinized starch, sucrose, sulfobutylether-β-cyclodextrin, talc, tragacanth, trehalose, and xylitol. In some embodiments, the diluents comprise mannitol and microcrystalline silicified cellulose. Diluents may be used in amounts calculated to obtain a desired volume for a tablet. In some embodiments, a diluent is used in an amount of about 5% or more, about 10% or more, about 15% or more, about 20% or more, about 22% or more, about 24% or more, about 26% or more, about 28% or more, about 30% or more, about 32% or more, about 34% or more, about 36% or more, about 38% or more, about 40% or more, about 42% or more, about 44% or more, about 46% or more, about 48% or more, about 50% or more. In some embodiments, a diluent used in the formulation is between about 20% and about 40% w/w of the drug core.

One or more lubricants may be used, *e.g.,* to facilitate tablet manufacture. Examples of suitable lubricants include, for example, vegetable oils such as peanut oil, cottonseed oil, sesame oil, olive oil, corn oil, and oil of theobroma, glycerin, magnesium stearate, calcium stearate, and stearic acid. In certain embodiments, stearates, if present, represent no more than approximately 2 weight % of the drug-containing core. In particular embodiments, the lubricant is magnesium stearate. In certain embodiments, the lubricant is present, relative to the drug core, in an amount of about 0.2% w/w of the drug core, about 0.4% w/w of the drug core, about 0.6% w/w of the drug core, about 0.8% w/w of the drug core, about 1.0% w/w of the drug core, about 1.2% w/w of the drug core, about 1.4% w/w of the drug core, about 1.6% w/w of the drug core, about 1.8% w/w of the drug core, about 2.0% w/w of the drug core, about 2.2% w/w of the drug core, about 2.4% w/w of the drug core, about 2.6% w/w of the drug core, about 2.8% w/w of the drug core, about 3.0% w/w of the drug core, about 3.5% w/w of the drug core, about 4% w/w of the drug core, about 4.5% w/w of the drug core, or about 5% w/w of the drug core. In some embodiments, the lubricant is present in an amount of between about 0.5% and about 5% w/w of the drug core, or between about 1% and about 3% w/w of the drug core.

One or more disintegrants may be used, *e.g.,* to facilitate disintegration of the tablet, and may be, *e.g*., starches, clays, celluloses, algins, gums or crosslinked polymers. Disintegrants also include, *e.g*., alginic acid, carboxymethylcellulose calcium, carboxymethylcellulose sodium (*e.g.,* AC-DI-SOL, PRIMELLOSE), colloidal silicon dioxide, croscarmellose sodium, crospovidone (e.g., KOLLIDON, POLYPLASDONE), guar gum, magnesium aluminum silicate, methyl cellulose, microcrystalline cellulose, polacrilin potassium, powdered cellulose, pregelatinized starch, sodium alginate, sodium starch glycolate (*e.g*., EXPLOTAB) and starch. In some embodiments, the disintegrant is croscarmellose sodium. In certain embodiments, the disintegrant is, relative to the drug core, present in the amount of about 1% w/w of the drug core, about 2% w/w of the drug core, about 3% w/w of the drug core, about 4% w/w of the drug core, about 5% w/w of the drug core, about 6% w/w of the drug core, about 7% w/w of the drug core, about 8% w/w of the drug core, about 9% w/w of the drug core, or about 10% w/w of the drug core. In some embodiments, the disintegrant is present in the amount of about between about 1% and about 10% w/w of the drug core, between about 2% and about 8% w/w of the drug core.

### B. 5-Azacytidine with At Least One Additional Therapeutic Agent

In particular embodiments, 5-azacytidine compositions provided herein further comprise one, two, three, or more other pharmacologically active substances (also termed herein "additional therapeutic agents," "second active agents," or the like). In some embodiments, the 5-azacytidine compositions are oral formulations. In some embodiments, the 5-azacytidine oral compositions with at least one additional therapeutic agent is used for treating any of the diseases or disorders disclosed herein. In particular embodiments, the oral formulations provided herein comprise the additional therapeutic agent(s) in a therapeutically effective amount. In particular embodiments, 5-azacytidine and the additional therapeutic agent(s) are co-formulated together in the same dosage form using methods of co-formulating active pharmaceutical ingredients, including methods disclosed herein and methods known in the art. In other embodiments, 5-azacytidine and the additional therapeutic agent(s) are co-administered in separate dosage forms. In some embodiments, certain combinations work synergistically in the treatment of particular diseases or disorders, including, *e.g*., types of cancer and certain diseases and conditions associated with, or characterized by, undesired angiogenesis or abnormal cell proliferation.

Examples of additional therapeutic agents include but are not limited to gilteritinib, midostaurin, quizartinib, enasidenib, ivosidenib, and venetoclax. In the present invention the additional therapeutic agent is venetoclax.

### C. Combination of 5-Azacytidine with Venetoclax as the At Least One Additional Therapeutic Agent Surprisingly Safe and Effective for Treating Diseases

In some embodiments, an oral pharmaceutical composition comprising 5-azacytidine is used with venetoclax as the additional therapeutic agent. In some embodiments, the 5-azacytidine oral compositions is used with venetoclax for safely and effectively treating any of the diseases or disorders disclosed herein.

Venetoclax is a selective inhibitor of BCL-2 and is marketed as VENCLEXTA^{™}, which is in the form of a tablet. Venetoclax is indicated in the US: (i) for the treatment of adult patients with chronic lymphocytic leukemia (CLL) or small lymphocytic lymphoma (SLL); (ii) in combination with injectable 5-azacytidine or decitabine or low-dose cytarabine for the treatment of newly-diagnosed acute myeloid leukemia (AML) in adults who are age 75 years or older, or who have comorbidities that preclude use of intensive induction chemotherapy. In some embodiments, patients receive 20 mg/m² of cytarabine once a day subcutaneously for 10 consecutive days every 4 weeks as the low-dose cytarabine.

Therapy with venetoclax is initiated according to a weekly ramp-up schedule over a specific period of several days or weeks to the recommended daily dose. For treating CLL and SLL, venetoclax is at administered at a daily dose of 20 mg for Week 1, a daily dose of 50 mg for Week 2, a daily dose of 100 mg for Week 3, a daily dose of 200 mg for Week 4, and a daily dose of 400 mg for Week 5 and beyond. For treating AML in combination therapy with another agent, such as injectable 5-azacytidine, venetoclax is at administered at a daily dose of 100 mg for Day 1, a daily dose of 200 mg for Day 2, and a daily dose of 400 mg for Days 3 and beyond. VIDAZA^{®} (5-azacytidine for injection) is administered in 28-day cycles, beginning on Day 1 of venetoclax treatment, at a dosage of 75 mg/m², IV or subcutaneously, on Days 1-7 of each cycle.

In some embodiments, venetoclax is administered orally. In some embodiments, the venetoclax is administered in a form of a tablet. In some embodiments, venetoclax is administered daily. In some embodiments, venetoclax is administered at a dose of from about 20 mg to about 400 mg, such as about 20 mg, about 50 mg, about 100 mg, about 200 mg, or about 400 mg. In some embodiments, venetoclax is administered at a dose of about 400 mg.

In some embodiments, 5-azacytidine and venetoclax are administered concomitantly. In some embodiments, 5-azacytidine and venetoclax are administered sequentially. In some embodiments, where the 5-azacytidine and venetoclax are administered sequentially, the 5-azacytidine is administered first. In some embodiments, 5-azacytidine and venetoclax are administered as separate dosage forms, such as injections suitable for intravenous or subcutaneous use and/or tablets or capsules for oral use. In some embodiments, 5-azacytidine and venetoclax are co-formulated as a single unit dosage form, such as an injection suitable for intravenous or subcutaneous use or a tablet or capsule for oral use.

### D. Combination of 5-Azacytidine with Gilteritinib as the At Least One Additional Therapeutic Agent Surprisingly Safe and Effective for Treating Diseases (not part of the claimed invention).

In some embodiments, an oral pharmaceutical composition comprising 5-azacytidine is used with gilteritinib as the additional therapeutic agent. In some embodiments, the 5-azacytidine oral compositions is used with gilteritinib for safely and effectively treating any of the diseases or disorders disclosed herein.

Gilteritinib is a tyrosine kinase inhibitor and is marketed as XOSPATA^{®}, which is in the form of a tablet. Gilteritinib is indicated in the US for the treatment of adult patients who have relapsed or refractory acute myeloid leukemia (AML) with a FLT3 mutation as detected by an FDA-approved test. The recommended starting dose for gilteritinib is 120 mg orally once daily with or without food.

In some embodiments, the gilteritinib is administered orally. In some embodiments, the gilteritinib is administered in a form of a tablet. In some embodiments, the gilteritinib is administered daily. In some embodiments, the gilteritinib is administered at a dose of from about 20 mg to about 400 mg, from about 40 mg to about 400 mg, from about 40 mg to about 200 mg, such as about 20 mg, about 40 mg, about 50 mg, about 80 mg, about 100 mg, about 120 mg, about 160 mg, about 200 mg, or about 400 mg. In some embodiments, the gilteritinib is administered at a dose of about 120 mg.

In some embodiments, 5-azacytidine and gilteritinib are administered concomitantly. In some embodiments, 5-azacytidine and gilteritinib are administered sequentially. In some embodiments, where the 5-azacytidine and gilteritinib are administered sequentially, the 5-azacytidine is administered first. In some embodiments, 5-azacytidine and gilteritinib are administered as separate dosage forms, such as injections suitable for intravenous or subcutaneous use and/or tablets or capsules for oral use. In some embodiments, 5-azacytidine and gilteritinib are co-formulated as a single unit dosage form, such as an injection suitable for intravenous or subcutaneous use or a tablet or capsule for oral use.

### E. Methods of Use for 5-Azacytidine and At Least One Additional Therapeutic Agent

As described herein, certain embodiments herein provide methods of treating a subject having acute myeloid leukemia (AML), wherein the method includes administering to the subject (i) a pharmaceutical composition comprising 5-azacytidine; and (ii) at least one additional therapeutic agent. In some embodiments, the pharmaceutical composition comprising 5-azacytidine is administered orally. Also in some embodiments, the pharmaceutical composition comprising 5-azacytidine as described herein is used with at least one additional therapeutic agent are used for treating AML in a subject, including a human patient.

In some embodiments, 5-azacytidine and one or more therapeutic agents are co-administered to subjects to yield a synergistic therapeutic effect. The co-administered agent may be a cancer therapeutic agent dosed orally or by injection.

In certain embodiments, methods provided herein for treating disorders related to abnormal cell proliferation comprise orally administering a formulation comprising a therapeutically effective amount of 5-azacytidine. Particular therapeutic indications relating to the methods provided herein are disclosed herein. In certain embodiments, the therapeutically effective amount of 5-azacytidine in the pharmaceutical formulation is an amount as disclosed herein. In certain embodiments, the precise therapeutically effective amount of 5-azacytidine in the pharmaceutical formulation will vary depending on, *e.g.,* the age, weight, disease and/or condition of the subject.

Disorders related to abnormal cell proliferation include, but are not limited to, myelodysplastic syndrome (MDS), acute myeloid leukemia (AML), acute lymphoblastic leukemia (ALL), chronic myeloid leukemia (CML), leukemia, chronic lymphocytic leukemia (CLL), lymphoma (including non-Hodgkin's lymphoma (NHL) and Hodgkin's lymphoma), multiple myeloma (MM), sarcoma, melanoma, carcinoma, adenocarcinoma, chordoma, breast cancer, colorectal cancer, ovarian cancer, lung cancer (*e.g*., non-small-cell lung cancer and small-cell lung cancer), testicular cancer, renal cancer, pancreatic cancer, bone cancer, gastric cancer, head and neck cancer, and prostate cancer In particular embodiments, the disorder related to abnormal cell proliferation is MDS. In particular embodimentss, the disorder related to abnormal cell proliferation is AML.

Particular embodiments herein provide methods for treating a subject having a disease or disorder provided herein by orally administering a pharmaceutical composition provided herein, wherein the treatment results in improved survival of the patient. In certain embodiments, the improved survival is measured as compared to one or more standard care regimens. Particular embodiments herein provide methods for treating a subject having a disease or disorder provided herein by orally administering a pharmaceutical composition provided herein, wherein the treatment provides improved effectiveness for treating the disease or disorder. In particular embodiments, the improved effectiveness is measured using one or more endpoints for cancer clinical trials, as recommended by the U.S. Food and Drug Administration (FDA). For example, FDA provides Guidance for Industry on Clinical Trial Endpoints for the Approval of Cancer Drugs and Biologics (http://www.fda.gov/CbER/gdlns/clintrialend.htm). The FDA endpoints include, but are not limited to, Overall Survival, Endpoints Based on Tumor Assessments such as (i) Disease-Free Survival (ii) Objective Response Rate, (iii) Time to Progression and Progression-Free Survival and (iv) Time-to-Treatment Failure. Endpoints Involving Symptom Endpoints may include Specific Symptom Endpoints such as (i) Time to progression of cancer symptoms and (ii) A composite symptom endpoint. Biomarkers assayed from blood or body fluids may also be useful to determine the management of the disease. In some embodiments, the improvement can be about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%.

Subjects in need of treatment can be members of a patient population with an increased risk of AML. For example, several inherited genetic disorders and immunodeficiency states are associated with an increased risk of AML. These include disorders with defects in DNA stability, leading to random chromosomal breakage, such as Bloom's syndrome, Fanconi's anemia, Li-Fraumeni kindreds, ataxia-telangiectasia, and X-linked agammaglobulinemia.

In certain embodiments, methods provided herein comprise treating acute promyelocytic leukaemia (APML) by administering a pharmaceutical composition comprising 5-azacytidine in combination with one or more additional agents to a subject in need thereof. APML is a rare sub-type of AML and is sometimes referred to as AML M31. This subtype is characterized by promyelocytic blasts containing the 15;17 chromosomal translocation. This translocation leads to the generation of the fusion transcript comprised of the retinoic acid receptor and a sequence PML.

In some embodiments, methods described herein are used to treat specific types of acute myeloid leukemia. Illustrative types of acute myeloid leukemia include but are not limited to, acute myeloid leukemia with recurrent genetic abnormalities, acute myeloid leukemia with myelodysplasia-related changes, therapy-related myeloid neoplasms, myeloid sarcoma, myeloid proliferations related to Down syndrome, blastic plasmacytoid dendritic cell neoplasm, and/or acute promyelocytic leukaemia.

In some embodiments, the AML is characterized as caused by any one of the following mutations: Fms-related tyrosine kinase 3 (FLT3), Kirsten rat sarcoma viral oncogene homolog (KRAS), neuroblastoma RAS viral (V-Ras) oncogene homolog (NRAS), proto-oncogene c-Kit (KIT), protein tyrosine phosphatase non-receptor type 11 (PTPN11), neurofibromin 1 (NF1), DNA methyltransferase 3A (DNMT3A), isocitrate dehydrogenase 1 (IDH1), isocitrate dehydrogenase 2 (IDH2), ten-eleven translocation-2 (TET2), additional sex comb-like 1 (ASXL1), enhancer of zeste homolog 2 (EZH2), mixed-lineage leukemia 1/histone-lysine N-methyltransferase 2A (MLL/KMT2A), nucleophosmin (NPM1), CCAAT enhancer binding protein alpha (CEBPA), runt-related transcription factor 1 (RUNX1), GATA-binding factor 2 (GATA2), tumor protein p53 (TP53), serine and arginine rich splicing factor 2 (SRSF2), U2 small nuclear RNA auxiliary factor 1 (U2AF 1), splicing factor 3b subunit 1 (SF3B1), zinc finger (CCCH type), RNA-binding motif and serine/arginine rich 2 (ZRSR2), RAD21 cohesin complex component (RAD21), stromal antigen 1 (STAG1), stromal antigen 2 (STAG2), structural maintenance of chromosomes 1A (SMC1A), and structural maintenance of chromosomes protein 3 (SMC3).

In some embodiments, the AML is characterized as caused by a FLT3-ITD mutation. In some embodiments, the AML is resistant to treatment with the at least one additional therapeutic agent alone. In some embodiments, the 5-azacytidine is administered before the at least one additional therapeutic agent. In some embodiments, the AML is responsive to treatment with a FLT3 inhibitor. In some embodiments, the AML is characterized as having an overexpression of MCL-1.

In some embodiments, the 5-azacytidine primes the cancer cells for apoptosis mediated by the at least one additional therapeutic agent by downregulating the expression of MCL-1. In some embodiments, downregulating the expression of MCL-1 is mediated by caspase-dependent and independent mechanisms. In some embodiments, the 5-azacytidine and at least one additional therapeutic agent augments MCL-1 degradation.

In certain embodiments, not part of the claimed invention, methods provided herein comprise treating lymphoma by administering a pharmaceutical composition comprising 5-azacytidine in combination with one or more additional agents to a subject in need thereof. Types of lymphomas include non-Hodgkin lymphoma and Hodgkin's disease. Examples of lymphoma include, but are not limited to, diffuse large B-cell lymphoma, anaplastic large-cell lymphoma, Burkitt lymphoma, lymphoblastic lymphoma, mantle cell lymphoma, peripheral T-cell lymphoma, follicular lymphoma, cutaneous T-cell lymphoma, lymphoplasmacytic lymphoma, marginal zone B-cell lymphoma, MALT lymphoma, small-cell lymphocytic lymphoma, and angioimmunoblastic T-cell lymphoma. In some embodiments, the lymphoma is angioimmunoblastic T-cell lymphoma.

In certain embodiments, methods provided herein comprise treating myelodysplastic syndromes, by administering a pharmaceutical composition comprising 5-azacytidine in combination with one or more additional agents to a subject in need thereof. MDS may also be classified by using the Revised International Prognostic Scoring System (IPSS-R), which classifies patients into 1 of 5 groups, from very low risk to very high risk, based on risk of mortality and transformation to acute myeloid leukemia (AML). Higher-risk MDS as used in the disclosure is defined as High or Very High risk according to the Revised International Scoring System (IPSS-R). Greenberg, P. L. et al., Blood 2012 Sep 20; 120 (12): 2454-2465. The scoring system for the IPSS-R is based on the following factors: the percentage of blasts (very early forms of blood cells) in the bone marrow, the type and number of chromosome abnormalities in the cells, the level of red blood cells (measured as hemoglobin) in the patient's blood, the level of platelets in the patient's blood, and the level of neutrophils (a type of white blood cell) in the patient's blood. Each factor is assigned a score and the total sum of the score is used to assign the MDS patient into one of the following five risk groups: Very low (Risk score of ≤ 1.5); Low risk (Risk score of > 1.5-3); Intermediate risk (Risk score of > 3-4.5); High risk (Risk Score of > 4.5-6); and Very high risk (Risk Score of > 6). About 13% of MDS patients are classified as High Risk, which has a mean overall survival of 1.6 years while about 10% of MDS patients are classified as Very High Risk, which has a mean overall survival of 0.8 years. In some embodiments, the MDS is MDS that is classified as High Risk or Very High Risk as defined by the IPSS-R.

### F. Dosing Regimens for 5-Azacytidine and an Additional Therapeutic Agent

Certain embodiments herein provide methods of treating diseases or disorders disclosed herein (*e.g*., diseases or disorders involving abnormal cell proliferation), wherein the methods comprise co-administering an oral formulation disclosed herein (such as, for example, an oral formulation comprising 5-azacytidine) with one or more additional therapeutic agents (such as, for example, a cancer therapeutic agent) to yield a synergistic therapeutic effect. Particular co-administered therapeutic agents useful in the methods disclosed herein are disclosed throughout the specification. In particular embodiments, the additional therapeutic agent is co-administered in an amount that is a therapeutically effective amount. In particular embodiments, the additional therapeutic agent is co-administered in a separate dosage form from 5-azacytidine dosage form with which it is co-administered. In particular embodiments, the additional therapeutic agent is co-administered in a dosage form (*e.g.,* a single unit dosage form) together with 5-azacytidine with which it is co-administered. In such cases, 5-azacytidine (*e.g*., azacitidine) and the additional therapeutic agent may be co-formulated together in the same dosage form using methods of co-formulating active pharmaceutical ingredients, including methods disclosed herein and methods known in the art.

In some embodiments provided herein is a method of treating a human having acute myeloid leukemia, wherein the method includes administering to the human a pharmaceutical composition including 5-azacytidine; and wherein the method further includes administering at least one additional therapeutic agent.

In some embodiments provided herein is a method of treating a human having myelodysplastic syndrome, wherein the method includes administering to the human a pharmaceutical composition including 5-azacytidine; and wherein the method further includes administering at least one additional therapeutic agent.

The additional therapeutic agent is venetoclax.

In some embodiments provided herein, the additional therapeutic agent is a Bcl2 inhibitor, namely venetoclax.

In one embodiment provided herein, the pharmaceutical composition that includes 5-azacytidine is administered orally.

In one embodiment provided herein, the pharmaceutical composition including 5-azacytidine is a capsule.

In one embodiment provided herein, the pharmaceutical composition including 5-azacytidine is a tablet.

In some embodiments, the 5-azacytidine and the at least one additional therapeutic agent are administered concomitantly. In some embodiments, the 5-azacytidine and the at least one additional therapeutic agent are administered sequentially wherein the 5-azacytidine is administered first. In some embodiments, 5-azacytidine and the at least one additional therapeutic agent are co-formulated as a single unit dosage form. In some embodiments, the additional therapeutic agent is administered parenterally. In some embodiments, the additional therapeutic agent is administered orally.

In some embodiments, the 5-azacytidine is administered orally. In some embodiments, the 5-azacytidine is administered at a dose of about 50 mg, about 60 mg, about 70 mg, about 80 mg, about 90 mg, about 100 mg, about 150 mg, about 200 mg, about 250 mg, about 300 mg, about 350 mg, about 400 mg, about 450 mg, about 500 mg, about 550 mg, or 600 mg orally. In some embodiments, the 5-azacytidine is administered at a dose of about 200 mg. In some embodiments, the 5-azacytidine is administered at a dose of about 300 mg. In some embodiments, 5-azacytidine is administered orally for the first seven, fourteen, or twenty-one days of a cycle. In some embodiments, the 5-azacytidine administered to the subject once or two times per day. In some embodiments, the 5-azacytidine is administered in the form of a capsule or a tablet. In some embodiments, the tablet is a non-enteric coated tablet.

In some embodiments, the at least one additional therapeutic agent is gilteritinib. In some embodiments, the at least one additional therapeutic agent is midostaurin. In some embodiments, the at least one additional therapeutic agent is venetoclax.

In some embodiments, the 5-azacytidine and the at least one additional therapeutic agent provides a synergistic effect to treat the diseases disclosed herein. Synergy may be measured by using the highest single agent (HSA) model and Combenefit package (Di Veroli et al., Bioinformatics. 2016 Sep 15;32(18):2866-8.) A negative cell line is used as a control to determine whether there was a shift in EC₅₀ and/or an augmentation of the maximal inhibitory effect. In other words, the EC₅₀ and maximal inhibitory effect from the negative control cell line provide baseline potency results, and the shift in EC₅₀ and maximal inhibitory effect of the drug combination is compared to the results from the negative control cell line to determine whether the drug combination provided a synergistic effect. Specifically, the following steps are used to determine the synergistic interactions between two drugs: (a) a demonstration of shift in dose response curves determined from their EC₅₀ (*i.e.,* a potency shift) and/or an augmentation of the maximal inhibitory effect compared to the results from the negative control cell line; (b) response surface analyses to visualize synergy, additivity or antagonism over a matrix of concentration between the two drugs; and (c) analyzing the combination index score (derived using a software application Combenefit). The limit of where the synergy index becomes significant (such that the drug combination exhibits synergistic effects) is determined empirically and is based on the variance in the data and a confirmation in a potency shift in EC₅₀. In other words, a combination index, without the clear shift in dose response curves would not constitute a synergistic interaction. As used herein, in some embodiments, the synergistic effect is defined as having an EC₅₀ shift at about greater than about 4 and/or a synergy index of greater than about 20 as measured by the HSA model and Combenefit package. In some embodiments, the synergistic effect is defined as having an EC₅₀ shift at about greater than 4 and/or a synergy index of greater than 20 as measured by the HSA model and Combenefit package.

In some embodiments, the 5-azacytidine primes the cancer cells for apoptosis mediated by the at least one additional therapeutic agent by downregulating the expression of MCL-1. In some embodiments, downregulating the expression of MCL-1 is mediated by caspase-dependent and independent mechanisms. In some embodiments, the 5-azacytidine and at least one additional therapeutic agent augments MCL-1 degradation.

In some embodiments, the 5-azacytidine alters cell metabolism. In some embodiments, the 5-azacytidine causes cell cycle arrest. In some embodiments, the 5-azacytidine suppresses oxidative phosphorylation. In some embodiments, the 5-azacytidine increases expression of ATF3 (activating transcription factor 3). In some embodiments, the 5-azacytidine decreases expression of SCD (stearoyl-CoA desaturase).

In some embodiments, the therapeutic effect of (1) 5-azacytidine administered orally and at least one additional therapeutic agent is better than the therapeutic effect of (2) the 5-azacytidine alone, (3) the at least one additional therapeutic alone, and/or (4) the combination of the 5-azacytidine administered intravenously or subcutaneously and at the at least one additional therapeutic agent.

In some embodiments, the 5-azacytidine and at least one additional therapeutic agent increases median survival as compared to the 5-azacytidine alone. In some embodiments, the 5-azacytidine and at least one additional therapeutic agent increases median survival as compared to the 5-azacytidine alone by about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%, as measured by any clinically recognized technique.

In some embodiments, the 5-azacytidine and venetoclax increases median survival as compared to the 5-azacytidine alone. In some embodiments, the 5-azacytidine and venetoclax increases median survival as compared to the 5-azacytidine alone by about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%, as measured by any clinically recognized technique.

In some embodiments, the 5-azacytidine and at least one additional therapeutic agent increases median survival as compared to at least one additional therapeutic agent alone. In some embodiments, the 5-azacytidine and at least one additional therapeutic agent increases median survival as compared to at least one additional therapeutic agent alone by about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%, as measured by any clinically recognized technique.

In some embodiments, the 5-azacytidine and venetoclax increases median survival as compared to venetoclax alone. In some embodiments, the 5-azacytidine and venetoclax increases median survival as compared to venetoclax alone by about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%, as measured by any clinically recognized technique.

In some embodiments, the 5-azacytidine and at least one additional therapeutic agent increases median survival as compared to 5-azacytidine administered intravenously or subcutaneously and at least one additional therapeutic agent. In some embodiments, the 5-azacytidine and at least one additional therapeutic agent increases median survival as compared to 5-azacytidine administered intravenously or subcutaneously and at least one additional therapeutic agent by about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%, as measured by any clinically recognized technique.

In some embodiments, the 5-azacytidine and venetoclax increases median survival as compared to 5-azacytidine administered intravenously or subcutaneously and venetoclax. In some embodiments, the 5-azacytidine venetoclax increases median survival as compared to 5-azacytidine administered intravenously or subcutaneously and venetoclax by about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%, as measured by any clinically recognized technique.

In one embodiment provided herein, the method includes: (i) administering 5-azacytidine to the subject for 1, 2, or 3 days; and (ii) administering the at least one additional therapeutic agent to the subject for one or more days. In one embodiment provided herein, the method further includes repeating steps (i) and (ii).

In one embodiment provided herein, the method includes: (i) administering 5-azacytidine daily to the subject for 1, 2, 3, 4, 5, 6, 7, 8, 9, 10, 11, 12, 13, or 14 days; (ii) administering the at least one additional therapeutic agent to the subject for one or more days; and (iii) optionally repeating steps (i) and (ii).

In one embodiment provided herein, the method includes: (i) administering 5-azacytidine daily to the subject for 1, 2, 3, 4, 5, 6, 7, 8, 9, 10, 11, 12, 13, 14, 15, 16, 17, 18, 19, 20, or 21 days; and (ii) administering the at least one additional therapeutic agent to the subject for one or more days. In one embodiment provided herein, the method further includes repeating steps (i) and (ii).

In one embodiment provided herein, the method includes: (i) administering 5-azacytidine daily to the subject for 1, 2, 3, 4, 5, 6, 7, 8, 9, 10, 11, 12, 13, or 14 days of a 28-day cycle; (ii) concurrently administering the at least one additional therapeutic agent daily to the subject for 1, 2, 3, 4, 5, 6, 7, 8, 9, 10, 11, 12, 13, 14, 15, 16, 17, 18, 19, 20, 21, 22, 23, 24, 25, 26, 27, or 28 days of a 28-day cycle; and (iii) optionally repeating steps (i) and (ii).

In one embodiment provided herein, the method includes: (i) administering 5-azacytidine daily to the subject for 1, 2, 3, 4, 5, 6, 7, 8, 9, 10, 11, 12, 13, 14, 15, 16, 17, 18, 19, 20, or 21 days of a 28-day cycle; (ii) concurrently administering the at least one additional therapeutic agent daily to the subject for 1, 2, 3, 4, 5, 6, 7, 8, 9, 10, 11, 12, 13, 14, 15, 16, 17, 18, 19, 20, 21, 22, 23, 24, 25, 26, 27, or 28 days of a 28-day cycle; and (iii) optionally repeating steps (i) and (ii).

In one embodiment provided herein, the method includes the sequential steps of: (i) administering 5-azacytidine to the subject for 7 days of a 28-day cycle; (ii) administering the at least one additional therapeutic agent to the subject for 1 day of a 28-day cycle; (iii) administering 5-azacytidine to the subject for 6 days of a 28-day cycle; and (iv) repeating steps (i) to (iii) after 7 days of a resting period.

In one embodiment provided herein, the method includes the sequential steps of: (i) administering 5-azacytidine daily to the subject for 1, 2, 3, 4, 5, 6, 7, 8, 9, 10, 11, 12, 13, 14, 15, 16, 17, 18, 19, 20, or 21 days of a 28-day cycle; (ii) administering the at least one additional therapeutic agent daily to the subject for 1, 2, 3, 4, 5, 6, 7, 8, 9, 10, 11, 12, 13, 14, 15, 16, 17, 18, 19, 20, or 21 days of a 28-day cycle; (iii) administering 5-azacytidine daily to the subject for 1, 2, 3, 4, 5, 6, 7, 8, 9, 10, 11, 12, 13, 14, 15, 16, 17, 18, 19, 20, or 21 days of a 28-day cycle; and (iv) repeating steps (i) to (iii) after 1, 2, 3, 4, 5, 6, 7, 8, 9, 10, 11, 12, 13, 14, 15, 16, 17, 18, 19, 20, or 21 days of a resting period.

In one embodiment provided herein, the pharmaceutical composition of 5-azacytidine includes about 50 mg, about 75 mg, about 100 mg, about 100 mg, about 200 mg, about 250 mg, about 300 mg, about 350 mg, about 360 mg, about 370 mg, about 400 mg, about 470 mg, about 480 mg, about 490 mg, about 500 mg, about 550 mg, or about 600 mg of 5-azacytidine.

In one embodiment provided herein, the at least one additional therapeutic agent is administered parenterally.

In one embodiment provided herein, the at least one additional therapeutic agent is administered orally.

In one embodiment provided herein, the subject is a human.

### III. Definitions

Unless defined otherwise, all technical and scientific terms used herein have the same meaning as commonly understood by one of ordinary skill in the art. All publications and patents referred to herein are incorporated by reference herein in their entireties.

As used in the specification and the accompanying claims, the indefinite articles "a" and "an" and the definite article "the" include plural as well as singular referents, unless the context clearly dictates otherwise.

The term "about" or "approximately" means an acceptable error for a particular value as determined by one of ordinary skill in the art, which depends in part on how the value is measured or determined. In certain embodiments, the term "about" or "approximately" means within 1, 2, 3, or 4 standard deviations. In certain embodiments, the term "about" or "approximately" means within 30%, 25%, 20%, 15%, 10%, 9%, 8%, 7%, 6%, 5%, 4%, 3%, 2%, 1%, 0.5%, 0.1%, or 0.05% of a given value or range.

As used herein, and unless otherwise specified, the terms "treat," "treating" and "treatment" refer to the eradication or amelioration of a disease or disorder, or of one or more symptoms associated with the disease or disorder. In certain embodiments, the terms refer to minimizing the spread or worsening of the disease or disorder resulting from the administration of one or more prophylactic or therapeutic agents to a subject with such a disease or disorder. In some embodiments, the terms refer to the administration of a compound or dosage form provided herein, with or without one or more additional active agent(s), after the onset of symptoms of the particular disease.

As used herein, amelioration of the symptoms of a particular disorder by administration of a particular pharmaceutical composition refers to any lessening, whether permanent or temporary, lasting or transient, that can be attributed to or associated with administration of the composition.

As used herein, and unless otherwise specified, the terms "therapeutically effective amount" and "effective amount" of a compound mean an amount sufficient to provide a therapeutic benefit in the treatment or management of a disease or disorder, or to delay or minimize one or more symptoms associated with the disease or disorder. A "therapeutically effective amount" and "effective amount" of a compound mean an amount of therapeutic agent, alone or in combination with one or more other agent(s), which provides a therapeutic benefit in the treatment or management of the disease or disorder. The terms "therapeutically effective amount" and "effective amount" can encompass an amount that improves overall therapy, reduces or avoids symptoms or causes of disease or disorder, or enhances the therapeutic efficacy of another therapeutic agent.

"Tumor," as used herein, refers to all neoplastic cell growth and proliferation, whether malignant or benign, and all pre-cancerous and cancerous cells and tissues. "Neoplastic," as used herein, refers to any form of dysregulated or unregulated cell growth, whether malignant or benign, resulting in abnormal tissue growth. Thus, "neoplastic cells" include malignant and benign cells having dysregulated or unregulated cell growth.

The terms "cancer" and "cancerous" refer to or describe the physiological condition in mammals that is typically characterized by unregulated cell growth. Examples of cancer include, but are not limited to blood borne (*e.g*., lymphoma, leukemia) and solid tumors.

The terms "composition," "formulation," and "dosage form," as used herein are intended to encompass compositions comprising the specified ingredient(s) (in the specified amounts, if indicated), as well as any product(s) which result, directly or indirectly, from combination of the specified ingredient(s) in the specified amount(s). By "pharmaceutical" or "pharmaceutically acceptable" it is meant that any diluent(s), excipient(s) or carrier(s) in the composition, formulation, or dosage form are compatible with the other ingredient(s) and not deleterious to the recipient thereof. Unless indicated otherwise, the terms "composition," "formulation," and "dosage form" are used herein interchangeably.

The term "immediate release," when used herein in reference to a composition, formulation, or dosage form provided herein, means that the composition, formulation, or dosage form does not comprise a component (*e.g.,* a coating) that serves to delay the spatial and/or temporal release of some or all of the API from the composition, formulation, or dosage form beyond the stomach following oral administration. In certain embodiments, an immediate release composition, formulation, or dosage form is one that releases the API substantially in the stomach following oral administration. In specific embodiments, an immediate release composition, formulation, or dosage form is one that is not delayed-release. In specific embodiments, an immediate release composition, formulation, or dosage form is one that does not comprise an enteric coating.

The term "non-enteric-coated," when used herein, refers to a pharmaceutical composition, formulation, or dosage form that does not comprise a coating intended to release the active ingredient(s) beyond the stomach (*e.g.,* in the intestine). In certain embodiments, a non-enteric-coated composition, formulation, or dosage form is designed to release the active ingredient(s) substantially in the stomach.

The term "substantially in the stomach," when used herein in reference to a composition, formulation, or dosage form provided herein, means that at least about 99%, at least about 95%, at least about 90%, at least about 85%, at least about 80%, at least about 75%, at least about 70%, at least about 65%, at least about 60%, at least about 55%, at least about 50%, at least about 45%, at least about 40%, at least about 35%, at least about 30%, at least about 25%, at least about 20%, at least about 15%, or at least about 10% of 5-azacytidine is released in the stomach. The term "released in the stomach" and related terms as used herein refer to the process whereby 5-azacytidine is made available for uptake by or transport across cells lining the stomach and then made available to the body.

The term "subject" is defined herein to include animals such as mammals, including, but not limited to, primates (e.g., humans), cows, sheep, goats, horses, dogs, cats, rabbits, rats, mice and the like. In specific embodiments, the subject is human.

### EXAMPLES

### Example 1

### Materials and Methods:

### Cells, culture conditions and reagents

AML cell lines were purchased from the American Tissue Culture Collection (ATCC) or were obtained from the Celgene master cell line bank. Cells were cultured in RPMI 1640 medium supplemented with 10% or 20% fetal bovine serum and 10 mM L-glutamine at 37 °C in a humidified atmosphere with 5% CO₂. BaF3-FLT3wt, BaF3-FLT3ITD, BaF3-FLT3D538Y were generated by Kyinno (Beijing, China). Cells were grown in RPMI with 10% FBS with 0.5 ug/ml puromycin. Exponentially growing cells were used for all *in vitro* studies. 5-azacytidine (l0 mM in DMSO) was obtained from the Celgene compound collection bank was obtained from the Celgene compound collection bank. Gilteritinib (ASP2215), Midostaurin (PKC412), venetoclax (ABT-199), quizartinib (AC220), the pan caspase inhibitor Z-VAD-FMK were purchased from Selleckchem (Houston, TX) and reconstituted as a l0 mM stock in DMSO.

### Cell Viability Assay

Cells were plated in 384-well plates (Coming Cat#3764) at 2000 cells/well in 50 µl medium. Relative cell numbers, calculated as % DMSO control well, were measured using Cell Titer-Glow (Promega, Madison, WI)) according to the manufacturer's instructions. Luminescence values were quantified at the time indicated using an EnvVsion plate reader (PerkinElmer). Cells were treated daily with 5-azacytidine for three days and/or once with midostaurin, gilteritinib or venetoclax. Nine doses of 5-azacytidine titrated depending on sensitivity to 5-azacytidine were combined with six doses of the second drug evaluated, yielding 54 possible combinations, each evaluated in duplicate for every experiment. Prism version 7.03 (Prism Software Corporation) was used to calculate EC₅₀ values.

### Data Analysis of Combination Effects

Cell survival was plotted as a function of drug concentration and used to calculate EC₅₀ values using GraphPad Prism software (San Diego, CA). Synergy indices were calculated by the highest single agent model and Combenefit software) Combenefit: an interactive platform for the analysis and visualization of drug combinations (Di Veroli et al., Bioinformatics. 2016 Sep 15;32(18):2866-8).

### Western blots

After treatment with 5-azacytidine and/or inhibitors at the indicated doses/times, protein was harvested using lysis buffer (Cell Signaling, #9803, Cell Signaling Technologies, Danvers, MA) containing 1mM PMSF. Lysates were quantified using a bicinchoninic acid (BCA) kit (Piece/Thermo Fisher, Waltham, MA). 20 to 30 µg protein was resolved on a 4-12% SDS-PAGE gel, transferred to PVDF membranes (80V/90 minute by wet-transfer), and blocked with Oddysey TBS blocking buffer for 1 hour and then probed with appropriate primary antibodies overnight at 4 °C using dilution as recommended by manufacturer. Membranes were washed three times for a total of 30 minutes and then incubated with secondary antibodies at room temperature in the dark for 1 hour. After another three washes, Odyssey infrared imaging system and companion software (LI-COR biosciences, Lincoln, NE, USA) were used to scan immunoblot membranes and to quantify band intensity according to the manufacturer's instructions. The ratio of proteins of interest to loading control in treated samples was normalized to the corresponding ratio in untreated cells. Antibodies used for immunoblotting were purchased from the following sources: BCL-2 (sc-7382), MCL1- (sc-819) from Santa Cruz Biotechnology (Dallas, TX, USA, Bim (2819), caspase-3 (9664) from Cell Signaling Technology; beta-Actin (A2228) from Sigma-Aldrich; DNMTI (abl88453) from AbCam; IRDye 680 goat anti-rabbit and IRDye 800 goat anti-mouse secondary antibodies (#925-68073 and #925-32212) were purchased from Li-COR Biosciences (Lincoln, NE).

### RNASeq Analysis

MV4-11 cells were treated with PBS or 1 µM of 5-azacytidine for 24 hours or daily with 1µM of 5-azacytidine for 48 hours in triplicate. After treatment, cells were recovered, washed once in PBS, and flash frozen as cell pellets. Cell pellets were sent to Canopy Biosciences for RNA extraction and library preparation and sequencing. RNA was extracted using the Qiagen RNeasy Mini Kit according to manufacturer's instructions. A modified protocol was used to preserve miRNA species. Total RNA Seq libraries were prepared using 200 ng of total RNA and the NEBNext Ultra II Directional Library prep kit. rRNA depletion was performed using an RNase-H based method (New England Biolabs, Ipswich, MA). *MCL1* RNA levels in other cell lines were quantified by RNASeq using standard methods.

Libraries were multiplexed and sequenced using Illumina HiSeq. All gene counts were then imported into the R/Bioconductor package EdgeR and TMM normalization size factors were calculated to adjust for samples for differences in library size. Ribosomal genes and genes not expressed in the smallest group size minus one samples greater than one count-per-million were excluded from further analysis. Differential expression analysis was then performed to analyze for differences between conditions and the results were filtered for only those genes with Benjamini-Hochberg false-discovery rate adjusted p-values less than or equal to 0.05. Global perturbations in known Gene Ontology (GO) terms and KEGG pathways were detected using the R/Bioconductor package GAGE to test for changes in expression of the reported log 2 fold-changes reported by Limma in each term versus the background log 2 fold-changes of all genes found outside the respective term. The R/Bioconductor package heatmap and Pathview was used to display heatmaps or annotated KEGG graphs across groups of samples for each GO term or KEGG pathway (respectively) with a Benjamini-Hochberg false-discovery rate adjusted p-value less than or equal to 0.05. To find differentially expressed genes, the raw counts were variance stabilized with the R/Bioconductor package DESeq2.

To validate ATF3 and SCD expression, MV4-11 cells were treated with PBS or 0.3 µM 5-azacytidine, 1 µM 5-azacytidine, or 3 µM 5-azacytidine for 24 hours and 48 hours. At this time, cells were recovered and RNA was extracted using Qiagen RNeasy kit according to manufacturer's instructions. Reverse transcription was performed using SuperScript VILO cDNA synthesis kit. Validated Taqman probes and Taqman Fast Advanced Master Mix was used with Viia 7 Real-Time PCR System (Invitrogen/ThermoFisher Scientific, Waltham, MA) to quantify transcripts of ATF3, SCD, and 18S mRNA.

### Interfering RNA gene silencing

ATF3, SCD, or control Silencer Select siRNAs (16nM siRNA, Invitrogen) were transfected into MV4-11 cells using Lipofectamine 2000 according to the manufacturer's suggested protocol. Untreated cells were mock transfected without siRNA. Cells were then treated with varying concentrations of 5-azacytidine daily for 3 days. At day 4, cells were treated with venetoclax, followed by examination of cell viability at day 7 using Cell Titer Glo according to manufacturer's protocol. Synergy was calculated using Combenefit and compared using Highest Single Agent analysis.

Confirmation of gene knockdown was performed on siRNA transfected cells at 72 hours after transfection (without 5-azacytidine or venetoclax treatment). RNA was extracted using Qiagen RNeasy kit, and reverse transcription was performed using SuperScript VILO cDNA synthesis kit. Validated Taqman probes and Taqman Fast Advanced Master Mix was used with Viia 7 Real-Time PCR System (Invitrogen/ThermoFisher Scientific, Waltham, MA) to quantify transcripts of ATF3, SCD, and 18S mRNA.

### In vivo analysis of 5-azacytidine combinations with LEED 5-azacytidine - and HELD 5-azacytidine dosing

As used throughout the Examples, LEED refers to the delivery of 5-azacytidine at a low exposure for an extended duration (LEED) at 1 mg/kg, once daily for fifteen days (QDx15). To deliver the same cumulative dose of 5-azacytidine, the 5-azacytidine is administered at a high exposure for a limited duration (HELD), at 3 mg/kg, once daily for five days (QDx5). LEED models oral administration of AZA, while HELD models intravenous or subcutaneous administration of AZA.

Experiments were carried out at Charles River Laboratories (Morissville, NC) with female NOD/SCID mice (NOD.CB17-Prkdcscid/NcrCrl, Charles River) that were eight weeks old with a body weight (BW) range of 17.6 to 28.4 grams on Day 1 of the study. The animals were fed *ad libitum* water (reverse osmosis, 1 ppm Cl), and NIH 31 Modified and Irradiated Lab Diet^{®} consisting of 18.0% crude protein, 5.0% crude fat, and 5.0% crude fiber. The mice were housed on irradiated Enrich-o'cobs^{™} Laboratory Animal Bedding in static microisolators on a 12-hour light cycle at 20-22°C (68-72°F) and 40-60% humidity.

Celgene provided LEED 5-azacytidine (Lot No. SOOL10), HELD 5-azacytidine (Lot Nos. SOOL10 and SOOL13), midostaurin (MedChemExpress, Monmouth Junction, NJ), gilteritinib (Sigma Aldrich, St. Louis, MO), and venetoclax (ABT-199, Sigma Aldrich, St. Louis, MO). The vehicle used in this study was 6% Gelucire^{®} 44/14 (Gattefossé, Paramus, NJ) in deionized (DI) water, which was a waxy solid that required a water bath heat to 44°C for melting, dosed PO (per oral), and phosphate buffered saline (PBS), dosed IP (intraperitoneal). On each day of dosing, an appropriate amount of LEED or HELD 5-azacytidine was resuspended in PBS to yield a dosing suspension at 0.1 mg/mL or 0.3 mg/mL, respectively. On each day of dosing, an appropriate amount of midostaurin was dissolved in 6% Gelucire 44/14 to yield a dosing solution at 10 mg/mL. On each day of dosing, an appropriate amount of gilteritinib was dissolved in 0.5% methylcellulose in DI water to yield a dosing solution at 0.4 mg/mL. Each week, an appropriate amount of venetoclax was dissolved in 10% ethanol:30% PEG400:60% phosal 50 propylene glycol to yield a dosing solution at 10 mg/mL. Cells used for inoculation were harvested during log phase growth and resuspended at a concentration of 5 x 107 cells/mL in PBS. Each test mouse received 5 x 10⁶ MOLM-13 cells or 10⁷ MV4-11 cells (0.2 mL cell suspension) by tail vein injection. Dosing was initiated three days after tumor cell inoculation, which was designated as Day 1 of the study. NOD/SCID mice (n = 9-12/group) were randomized according to body weight and dosed. Phosphate buffered saline, LEED 5-azacytidine, and HELD 5-azacytidine were administered intraperitoneally (IP), while midostaurin, gilteritinib, and venetoclax were administered PO. Vehicle was administered both IP and PO. The dosing volume for all treatments was 10 mL/kg, scaled to the weight of each individual animal.

Animals were monitored individually for an endpoint of moribundity due to progression of the leukemia. Full hind limb paralysis, severe ocular proptosis, or moribundity was considered sufficient for euthanasia due to tumor progression. Moribund animals were defined as sick animals unable to reach food and water. These deaths were classified as death on survival study. The time to endpoint (TTE), in days, was recorded for each mouse that died of its disease or was euthanized due to extensive tumor progression. Animals that did not reach the endpoint were euthanized at the end of the study and were assigned a TTE value equal to the last day. An animal classified as having died from treatment-related (TR) causes was assigned a TTE value equal to the day of death. An animal classified as having died from non-treatment-related (NTR) causes, or used for sampling before endpoint, was excluded from TTE calculations and all further analyses. The median TTE value was calculated for each group. The median TTE of treated mice was expressed as a percentage of the median TTE of the control mice (%T/C), and the increase in life span (ILS) was calculated as: ILS = %T/C - 100%, where T = median TTE treated, and C = median TTE control. Thus, if T = C, ILS = 0%.

Animals were weighed daily on Days 1-5, then twice per week until the completion of the study. The mice were observed frequently for overt signs of any adverse, treatment-related (TR) side effects, and clinical signs were recorded when observed. Individual body weight loss was monitored as per protocol and any animal that exceeded the limits for acceptable body weight loss was euthanized. Group mean body weight loss also was monitored as per protocol. Dosing was suspended in any group that exceeded the limits for acceptable mean body weight loss. If mean body weight recovered, then dosing may be resumed in that group, but at a lower dosage or less frequent dosing schedule. Acceptable toxicity for the maximum tolerated dose was defined as a group mean body-weight loss of less than 20% during the study and not more than one TR death among ten treated animals. A death was classified as TR if attributable to treatment side effects as evidenced by clinical signs and/or necropsy or may also be classified as TR if due to unknown causes during the dosing period or within 14 days of the last dose. A death was classified as NTR if there was no evidence that death was related to treatment side effects or tumor progression. Non-treatment-related deaths may be further characterized based on cause of death. A death may be classified as NTRa if it resulted from an accident or human error. A death may be classified as NTRu if the cause of death is unknown and there is no available evidence of death related to treatment side effects, metastasis, accident or human error, although death due to these etiologies cannot be excluded. Survival was analyzed by the Kaplan-Meier method, based on TTE values. The logrank (Mantel-Cox) and Gehan-Breslow-Wilcoxon tests determined the significance of the difference between the overall survival experiences (survival curves) of two groups, based on TTE values.

### Results

### Combinations of 5-Azacytidine with Midostaurin, Gilteritinib or Venetoclax

FIGS. 1-7 provide various information and data regarding the experiment. For example, FIG. 1 is a bar graph representing the maximum EC₅₀ fold shift of 5-azacytidine in combination with gilteritinib, and 5-azacytidine in combination with midostaurin, both with cell lines MV4-11 and MOLM-13. The results from three different dosing schedules are shown: (i) 5-azacytidine administered first (black bar); (ii) the two agents administered concurrently (light gray bar); and (iii) 5-azacytidine administered second (medium gray bar). FIG. 2 represents the three different dosing schedules of (i) 5-azacytidine (AZA) administered first at intervals before the FLT3 inhibitor (FLT3i); (ii) the two agents (5-azacytidine and FLT3i) administered concurrently; and (iii) 5-azacytidine administered second at intervals after the FLT3i is administered; where the FLT3i may be any suitable FLT3 inhibitor, such as midostaurin or gilteritinib. FIGS. 3A-D represent the maximum EC₅₀ fold shift of 5-azacytidine in combination with venetoclax with cell lines MV4-11 (FIG. 3A) and MOLM-13 (FIG. 3C). Three different dosing schedules are shown, (i) 5-azacytidine administered first (black bar); (ii) the two agents administered concurrently (light gray bar); and (iii) 5-azacytidine administered second (medium gray bar). A synergy index is also shown for 5-azacytidine administered in combination with venetoclax with cell lines MV4-11 (FIG. 3B) and MOLM-13 (FIG. 3D) for the three different dosing schedules.

FIGS. 4A-C represent Response Surface Analyses showing synergy of 5-azacytidine with venetoclax in MV4-11 cells when 5-azacytidine is administered first (FIG. 4A), the relatively lower synergy with simultaneous administration (FIG. 4B), and synergy with venetoclax administered first (FIG. 4C). Response surface methodology (RSM) explores the statistical relationships between several explanatory variables and one or more response variables. RSM uses a sequence of designed experiments to obtain an optimal response, which in the present case is the synergistic effects of 5-azacytidine with venetoclax.

FIG. 5 depicts a western blot showing that (a) 5-azacytidine and midostaurin ("aza+0.3 µM Mido") and (b) 5-azacytidine and gilteritinib ("aza + 0.3 µM Gilt") augment MCL-1 degradation in MV4-11 cell lines. In addition, FIG. 6 depicts a western blot showing that 5-azacytidine and venetoclax treatment decreases MCL-1 Levels in FLT3ITD MV4-11 cells.

Finally, FIGS. 7A-C depict *in vivo* assessments of 5-azacytidine combinations in a MOLM-13 xenograft model, with a graph of percent survival (y-axis) vs day 0 to 70 (x-axis). Dosing for the experiments shown in FIGS. 7A-Cwas as follows: (i) 5-azacytidine (low exposure, extended duration, LEED): 1 mg/kg interperitoneally (IP), once daily for five days, three times (qdx 5x3); (ii) 5-azacytidine (high exposure, limited duration, HELD): 3 mg/kg interperitoneally (IP), once daily for five days (qdx5); (iii) Midaustaurin (100 mg/kg orally (PO), once daily for twenty-one days (qdx21)); (iv) Gilteritinib (4 mg/kg orally (PO), once daily for twenty-one days (qdx21)); and (v) Venetoclax (100 mg/kg orally (PO), once daily for twenty-one days (qdx21)). P-value (relative to best single agent) *P < 0.05; **P<0.001; ***P<0.0001. FIG. 7A shows the results of the combination of 5-azacytidine and midostaurin, FIG. 7B shows the results of 5-azacytidine combined with venetoclax, and FIG. 7C shows the results of the combination of 5-azacytidine and gilteritinib. For FIG. 7A, the compositions tested were vehicle, 5-azacytidine (low exposure, extended duration, LEED, schedule of 1 mg/kg 5-azacytidine, once daily for fifteen days (qdx15)), 5-azacytidine (high exposure, limited duration, HELD, schedule of 3 mg/kg 5-azacytidine, once daily for five days (qdx5)), midostaurin (100 /kg, once daily for twenty eight days (qdx28)), LEED + midostaurin, and HELD + midostaurin. For FIG. 7B, the compositions tested were vehicle, 5-azacytidine (LEED), 5-azacytidine (HELD), venetoclax, LEED + venetoclax, and HELD + venetoclax. For FIG. 7C, the compositions tested were vehicle, 5-azacytidine (LEED), 5-azacytidine (HELD), gilteritinib, LEED + gilteritinib, and HELD + gilteritinib. Both LEED and HELD 5-azacytidine dosing caused statistically significantly increases in survival compared to vehicle alone (LEED vs vehicle, p = 0.003 by Gehan-Breslow-Wilcoxon test; HELD vs vehicle, p = 0.003 by Gehan-Breslow-Wilcoxon test). Midostaurin alone and in combination with LEED or HELD 5-azacytidine significantly increased survival compared to vehicle alone (Midostaurin vs vehicle, p = 0.027; LEED + midostaurin vs vehicle, p = 0.012; HELD + midostaurin vs vehicle, p = 0.003). HELD 5-azacytidine dosing in combination with midostaurin significantly increased survival compared to LEED or HELD 5-azacytidine, respectively (LEED + midostaurin vs LEED, p = 0.028; HELD + midostaurin vs HELD, p = 0.039). No significant changes in survival were observed between LEED or HELD in combination with midostaurin compared to midostaurin treatment alone. Median survival was increased with LEED or HELD 5-azacytidine in combination with midostaurin compared to vehicle or single agents (LEED + midostaurin = 45 days, HELD + midostaurin = 43 days, vehicle = 19 days, midostaurin = 34 days, LEED = 36 days, HELD = 32 days, (FIG. 7A). Gilteritinib alone and in combination with LEED or HELD 5-azacytidine significantly increased survival compared to vehicle alone (gilteritinib vs vehicle, p = 0.003; LEED + gilteritinib vs vehicle, p = 0.003; HELD + gilteritinib vs vehicle, p = 0.003). Low exposure, extended duration or HELD 5-azacytidine dosing in combination with gilteritinib significantly increased survival compared to either LEED or HELD 5-azacytidine alone (LEED + gilteritinib vs LEED, p = 0.019; LEED + gilteritinib vs HELD, p = 0.004; HELD + gilteritinib vs LEED, p = 0.008; HELD + gilteritinib vs HELD, p = 0.003. Furthermore, LEED or HELD 5-azacytidine dosing in combination with gilteritinib significantly increased survival compared to gilteritinib alone (LEED + gilteritinib vs gilteritinib, p < 0.001; HELD + gilteritinib vs gilteritinib, p < 0.001). Venetoclax alone and in combination with LEED or HELD 5-azacytidine significantly increased survival compared to vehicle alone (venetoclax vs vehicle, p = 0.003; LEED + venetoclax vs vehicle, p = 0.002; HELD + venetoclax vs vehicle, p = 0.004) (FIG. 7B). Low exposure, extended duration or HELD 5-azacytidine dosing in combination with venetoclax significantly increased survival compared to either LEED or HELD 5-azacytidine alone (LEED + venetoclax vs LEED, p = 0.001; LEED + venetoclax vs HELD, p < 0.001; HELD + venetoclax vs LEED, p = < 0.001; HELD + venetoclax vs HELD, p = < 0.001. Furthermore, LEED or HELD 5-azacytidine dosing in combination with venetoclax significantly increased survival compared to venetoclax alone (LEED + venetoclax vs venetoclax, p < 0.001; HELD + venetoclax vs venetoclax, p < 0.001). Low exposure, extended duration in combination with venetoclax was not significantly different than HELD in combination with venetoclax. Median survival was increased with LEED or HELD 5-azacytidine in combination with venetoclax compared to vehicle or single agents (LEED + venetoclax = 46 days, HELD + venetoclax = 45 days, vehicle = 19 days, venetoclax = 29 days, LEED = 36 days, HELD = 32 days). Median survival was increased with LEED or HELD 5-azacytidine in combination with gilteritinib compared to vehicle or single agents (LEED + gilteritinib = 45 days, HELD + gilteritinib = 43 days, vehicle = 19 days, gilteritinib = 34 days, LEED = 36 days, HELD = 32 days, (FIG. 7C).

FIGS. 8A, 8B, and 8C show the sensitivity of 22 AML cell lines to 5-azacytidine (AZA) and venetoclax as single agents (FIG. 8A and 8B, respectively) and the combination of 5-azacytidine and venetoclax (FIG. 8C). FIG. 8A shows that 5-azacytidine showed cytotoxic effects in most cell lines, with EC₅₀ values ranging from 0.15 µM to 2.5 µM. In contrast, FIG. 8B shows that 11/22 of the AML cell lines examined were sensitive to venetoclax (EC₅₀ < 10 µM). FIG. 8C shows the combinatorial activity of 5-azacytidine with venetoclax using surface response analysis and highest single agent model, where 10/22 cell lines showed synergistic activity above the arbitrary threshold of 20. Notably, three cell lines that were resistant to venetoclax (Kasumi-1, Kasumi-2 and NOMO-1) showed reversal of venetoclax resistance with co-treatment with 5-azacytidine. Cell lines that carried FLT3-ITD, a recurrent mutation in AML, also showed synergistic activity with 5-azacytidine and venetoclax.

These results surprisingly demonstrate that the combination of 5-azacytidine with venetoclax provides a synergistic effect in AML cell lines, and in particular AML cell lines that are resistant to venetoclax. The results support that the combination of 5-azacytidine with venetoclax is safe and effective for treating AML patients.

Whether the specific schedule of 5-azacytidine and venetoclax administration has an influence on the synergistic effect provided by 5-azacytidine and venetoclax was also investigated. FIGS. 9A-F show the cell survival of MV4-11 cells (FIGS. 9A-C) and MOLM-13 cells (Figs. 9D-F) seven days after the start of treatment with 5-azacytidine and venetoclax. The following schedules were tested: 5-azacytidine administration on Days 1, 2 and 3, followed by venetoclax administration on Day 4 (5-azacytidine (AZA) First) (FIGS. 9A and 9D); 5-azacytidine and venetoclax co-administration on Day 1, followed by 5-azacytidine administration on Days 2 and 3 (Simultaneous) (Figs. 9B and 9E); and venetoclax administration on Day 1, followed by 5-azacytidine on Days 2, 3 and 4 (venetoclax first) (FIGS. 9C and 9F). The results show that for both cell lines, the regimen where 5-azacytidine was administered first provided the maximal synergistic effects. These results suggest that 5-azacytidine that may prime AML cells for venetoclax activity.

One of the factors for venetoclax resistance is the expression of the apoptotic regulator MCL-1, which is upregulated in FLT3 mutated AML and is downregulated after 5-azacytidine treatment. To examine whether MCL-1 levels correlate with the degree of the synergistic effect of the combination of 5-azacytidine and venetoclax, a panel of engineered BaF3 cell lines expressing either wild-type FLT3, FLT3-ITD or FLT3 (D835Y) mutations was examined. Engineered BaF3 cell lines also proliferated independently of IL-3. FIG. 10A shows that these engineered BaF3 cell lines were resistant to venetoclax (EC₅₀ >1 µM), but sensitive to FLT3 inhibitors, such as gilteritinib, midostaurin and quizartinib. The data shown in FIG. 10A is also shown in Table 1, below.

**Table 1**

| **BaF3 cells expressing various FLT3 (EC50, nM)** | | | |
|---|---|---|---|
| | **BaF-FLT3(WT)** | **BaF-FLT3-ITD** | **BaF-FLT3(D835Y)** |
| **AZA** | 189 | 598 | 456 |
| **Gilteritinib** | 4 | 1.8 | 1.8 |
| **Midostaurin** | 23 | 5.5 | 5.4 |
| **Quizartinib** | 3 | 0.008 | 9.5 |
| **Venetoclax** | >10000 | >10000 | 4332 |

FIG. 10B shows that MCL-1 was detected in all lines, with the highest expression levels observed in the FLT-ITD mutant line, followed by FLT3 (D835Y). The combination of 5-azacytidine with venetoclax showed a synergistic effect, with the highest synergy index observed in FLT3 (wildtype), expressing the lowest levels of MCL-1, followed by FLT3 (D835Y) (intermediate MCL-1 levels) and FLT3-ITD (highest MCL-1) (FIG. 10C). These results suggests that MCL-1 expression may be a determinant factor for the 5-azacytidine-venetoclax synergy.

To further explore the relationship between MCL-1 and 5-azacytidine-venetoclax synergy further, the relationship between *MCL1* RNA levels and 5-azacytidine-venetoclax synergy indices was examined explored in a panel of 20 AML cell lines. FIG. 11 shows that *MCL1* RNA levels correlated directly with the synergy index (r² = -0.5607, p = 0.0101) in a panel of 20 AML cell lines. These results show that MCL-1 may be a key regulator for AZA priming for venetoclax-induced apoptosis, specifically 5-azacytidine may lower MCL-1 below a certain threshold to allow venetoclax-mediated apoptosis.

Next, the extent of 5-azacytidine-mediated MCL-1 degradation in four different AML cell lines was explored KG1α (FIG. 12A), MV4-11 (FIG. 12B), THP-1 (FIG. 12C) and OCI-AML-2 (FIG. 12D). The results showed 5-azacytidine-venetoclax synergistic activity with KG1α (FIG. 12E) and MV4-11 (FIG. 12F) cell lines (synergy index (SI) of 70 and 35.5, respectively) and very little or no synergistic activity with THP-1 (FIG. 12G) and OCI-AML-2 (FIG. 12H) cell lines (SI of 20.2 and 10.8, respectively). For the KG1α (FIGS. 12A) and MV4-11 (FIG. 12B) cell lines, where 5-azacytidine- venetoclax had the greatest synergistic effect (FIGS. 12E and 12F), 5-azacytidine led to MCL-1 degradation the fastest, starting 6 hours after treatment. In contrast, for THP-1 (FIG. 12C), where 5-azacytidine-venetoclax only provided minor synergistic activity showed 5-azacytidine-mediated MCL-1 degradation later, starting at 16 hours, with incomplete degradation by 24 hours (FIG. 12G). For OCI-AML2 (FIG. 12D), where 5-azacytidine-venetoclax had the lowest synergistic effect (FIG. 12H), 5-azacytidine treatment did not lead to any degradation of MCL-1. These results support the hypothesis that 5-azacytidine primes cells for venetoclax-mediated apoptosis by lowering MCL-1 levels.

One possible mechanism by which 5-azacytidine downregulates MCL-1 is by inducing caspase activation. Caspase activation can be assayed by evaluating the degradation of caspase 3 in a western blot (FIG. 13A). To find out whether this effect is caspase-dependent, the cells were treated with Z-VAD-FMK, a pan-caspase inhibitor, and the extent of MCL-1 degradation by 5-azacytidine was measured (FIG. 13B). In particular, FIG. 13B shows a bar graph of MCL-1 degradation by 5-azacytidine, where cells were treated with 20 µM Z-VAD-FMK for 1 hours before 5-azacytidine treatment for another 16 hours. Caspase inhibition partially ablated MCL-1 degradation by 5-azacytidine in MV4-11 cells, suggesting additional, caspase-independent mechanisms of MCL-1 degradation. It was found that Z-VAD-FMK partially ablated the ability of 5-azacytidine to degrade MCL-1, suggesting this process is mediated by caspase-dependent and independent mechanisms.

To further understand how 5-azacytidine primes venetoclax for acute apoptosis, RNAseq was performed on MV4-11 cells treated with PBS (vehicle), 1 µM AZA for 24 hours (FIG. 14A), or with 1µM AZA for 48 hours (FIG. 14B). Table 2 is the pathway analysis for RNASeq data in FIG. 14A and shows the analysis after 5-azacytidine treatment, which was the categorization of genes that were significantly induced or repressed by 5-azacytidine based on KEGG pathways.

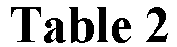

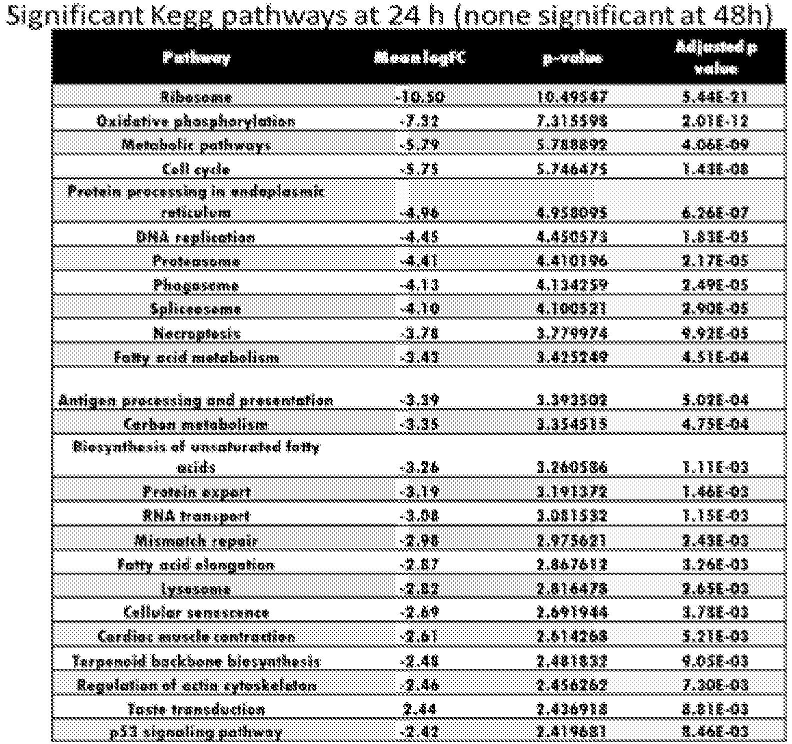

No significant differences were observed in KEGG pathways regulated by 5-azacytidine after 48 hours of treatment. However, the top KEGG pathways differentially regulated after 24 hours of 5-azacytidine treatment were "Ribosome", "Oxidative Phosphorylation", "Metabolic Pathways", and "Cell Cycle". These results support the hypothesis that 5-azacytidine has a role in altering cell metabolism, causing cell cycle arrest, suppressing oxidative phosphorylation, which was previously observed in patients treated with 5-azacytidine+venetoclax combination.

Volcano plots of significantly modified genes at 24 hours (FIG. 14A) and 48 hours (FIG. 14B) show 5-azacytidine induced 133 differentially expressed genes at 24 hours and 226 differentially expressed genes at 48 hours. Upon further analysis of the 5-azacytidine-induced differentially expressed genes, two genes were identified that have previously been shown to regulate MCL1 expression: activating transcription factor 3 (ATF3) and stearoyl-CoA desaturase (SCD). ATF3 is a stress responsive transcription factor that was shown to regulate MCL-1, as well as immune and metabolic genes. ATF3 expression was increased two-fold 48 hours after 5-azacytidine treatment. On the other hand, the expression of SCD, a regulator of lipid metabolism and MCL1, was decreased 2.5-fold by 5-azacytidine treatment at 48 hours. Alterations in ATF3 (FIG. 14C) and SCD (FIG. 14D) expression were validated in a separate experiment using real-time PCR. ATF3 expression was increased in a time- and concentration- dependent manner, as 0.3 µM 5-azacytidine treatment was not sufficient to induce ATF3 expression at either 24 or 48 hours (Fig. 14C). Similarly, SCD expression was decreased rapidly within 24 hours when treated with 3 µM 5-azacytidine, although it was not affected by low concentrations of 5-azacytidine at this timepoint (FIG. 14D).

Given their connection with regulating *MCL1* expression, it was hypothesized that ATF3 and/or SCD may contribute to 5-azacytidine-venetoclax synergy. To explore this further, siRNA knockdown of these genes was utilized in MV4-11 cells to assess their function in synergy. MV4-11 cells were left untransfected or transfected with ATF3, SCD, or control (scrambled) siRNA. As a control, cells were transfected with siRNA and collected for RNA and qPCR 72 hours after transfection. (FIG. 15A) This confirmed that siRNA knockdown decreased, but did not completely ablate, mRNA expression of ATF3 or SCD when cells were transfected with ATF3 or SCD siRNA, respectively. Furthermore, in cells treated with scramble siRNA, no changes in ATF3 (FIG. 15B) or SCD (FIG. 15C) expression were seen. Following transfection, cells were treated with various concentrations of 5-azacytidine daily for Days 1-3. At Day 4, cells were dosed with venetoclax, followed by cell viability test using CellTiter-Glo^{®} 7 after treatment initiation. 5-Azacytidine-venetoclax synergy was calculated using Combenefit and Highest Single Agent analysis (FIGS. 15D-G). 5-Azacytidine-venetoclax synergy was confirmed in cells that were not transfected (Synergy Index = 43) (FIG. 15D), and the synergy was not affected by transfection itself, as cells transfected with scramble siRNA (FIG. 15E) had a synergy index of 46. When ATF3 was knocked down (FIG. 15F), 5-azacytidine-venetoclax had decreased synergy (Synergy Index = 19). On the other hand, when SCD (FIG. 15G) was knocked down, 5-azacytidine-venetoclax had increased synergy (Synergy Index = 60). This data suggests that 5-azacytidine-induced increases in ATF3 and decreases in SCD play at least a partial role in 5-azacytidine-venetoclax synergy.

Whether 5-azacytidine and venetoclax have synergistic activity *in vivo* at doses and schedules corresponding to injectable 5-azacytidine (HELD) or oral 5-azacytidine (LEED) was next evaluated. MV4-11 (FIG. 16A-C) and MOLM-13 (FIGS. D-F), two cell lines that showed 5-azacytidine-venetoclax synergy (FIG. 8C), were used to generate disseminated AML xenograft mice in immunodeficient animals. *In vitro,* venetoclax sensitized both cell lines to venetoclax (FIGS. 16A and 16D) and synergized with 5-azacytidine (FIGS. 16B and 16E). To model oral 5-azacytidine (LEED) regimes, mice were treated with 1 mg/kg 5-azacytidine for 15 days (low exposure, extended duration). Alternatively, to use the same cumulative dose but with an injectable 5-azacytidine (HELD) regime, mice were treated with 3 mg/ml 5-azacytidine for 5 days (high exposure, limited duration).

For MV4-11 implantation, female NCG mice were injected via tail vein with 1x10⁷ cells in 0.2mL cell suspension. Day 1 was designated as fourteen days after implantation. On Day 1, mice were sorted into treatment groups based on body weight and dosing was initiated as follows: mice treated with vehicle, high dose 5-azacytidine (HELD, 3mg/kg once daily for five days (qdx5)), low dose 5-azacytidine (LEED, 1mg/kg once daily for five days, three times (qdx5x3)), venetoclax (100mg/kg, once daily for twenty one days (qdx21)), HELD+venetoclax, or LEED+venetoclax. Mice were monitored for body weight loss and moribundity for up to 56 days after initial treatment to determine when mice succumbed to tumor burden. Venetoclax alone or in combination with LEED or HELD 5-azacytidine significantly increased survival compared to vehicle alone (venetoclax vs vehicle, p = 0.0493; LEED + venetoclax vs vehicle, p = 0.0123; HELD + venetoclax vs vehicle, p = 0.04). LEED or HELD 5-azacytidine in combination with venetoclax significantly increased survival compared to 5-azacytidine alone (LEED + venetoclax vs LEED, p = 0.001; HELD + venetoclax vs HELD, p = 0.0004). However, only LEED + 5-azacytidine was significantly better than venetoclax alone (LEED + venetoclax vs venetoclax, p = 0.0378). Furthermore, LEED or HELD 5-azacytidine combination with venetoclax did increase median survival compared to single agents (LEED + venetoclax = 38, HELD + venetoclax = 37, vehicle = 29.5, HELD = 35, LEED = 35, venetoclax = 35.5). (FIG. 16C)

These experiments were repeated with a second FLT3-ITD cell line, MOLM-13. Briefly, 5x10⁶ MOLM-13 cells were injected into 12 NOD/SCI mice per group. Three days after tumor cell inoculation, mice were treated with the same dosing regimen as MV4-11 cells. Mice were monitored for body weight loss and moribundity for up to 70 days after initial treatment to determine when mice succumbed to disease burden. Venetoclax alone and in combination with LEED or HELD 5-azacytidine significantly increased survival compared to vehicle alone (venetoclax vs vehicle, p = 0.003; LEED + venetoclax vs vehicle, p = 0.002; HELD + venetoclax vs vehicle, p = 0.004). Low exposure, extended duration or HELD 5-azacytidine dosing in combination with venetoclax significantly increased survival compared to either LEED or HELD 5-azacytidine alone (LEED + venetoclax vs LEED, p = 0.001; LEED + venetoclax vs HELD, p < 0.001; HELD + venetoclax vs LEED, p = < 0.001; HELD + venetoclax vs HELD, p = < 0.001. Furthermore, LEED or HELD 5-azacytidine dosing in combination with venetoclax significantly increased survival compared to venetoclax alone (LEED + venetoclax vs venetoclax, p < 0.001; HELD + venetoclax vs venetoclax, p < 0.001). Median survival was increased with LEED or HELD 5-azacytidine in combination with venetoclax compared to vehicle or single agents (LEED + venetoclax = 46 days, HELD + venetoclax = 45 days, vehicle = 19 days, venetoclax = 29 days, LEED = 36 days, HELD = 32 days). (FIG. 16F) Altogether, these results show that patients with Flt3-ITD mutations may benefit from 5-azacytidine+venetoclax combination therapy.

### Combinations of 5-Azacytidine with FLT-3 Inhibitors

FLT3 mutations occur in ~30% of AML patients and have been associated with poor prognosis. The broad-acting FLT-3 inhibitor midostaurin and the selective FLT3 inhibitor, gilteritinib, have been approved for the treatment of AML. To investigate whether co-treatment with 5-azacytidine and FLT3 inhibitors have a synergistic effect in AML cells, two FLT3-ITD cell lines, MV4-11 and MOLM-13 cells were treated with 5-azacytidine+midostaurin or 5-azacytidine+gilteritinib. Cells were treated with daily doses of 5-azacytidine on Day 1-3, and then treated with a FLT-3 inhibitor (midostaurin or gilteritinib) at Day 4. Cells were collected on Day 7 and cell viability was assessed by CellTiter-Glo^{®} assay. Midostaurin sensitized MV4-11 to 5-azacytidine (FIG. 17A) and showed synergistic activity with 5-azacytidine (FIG. 17B). Similar effects were observed in MV4-11 cells treated with 5-azacytidine and gilteritinib (FIGS. 17C and 17D), as well as in MOLM-13 cells treated with 5-azacytidine and midostaurin (FIGS. 17E and 17F) or gilteritinib (FIGS. 17G and 17 H).

Next synergistic activity between FLT3 inhibitors and 5-azacytidine administered using a dose and schedule similar to injectable (high exposure, limited duration, or HELD regimen) or oral (low exposure, extended duration, LEED) was examined. Two disseminated xenograft models of AML based on MOLM-13 and MV4-11 cell lines were used. Mice were treated with 5-azacytidine using a HELD regimen (3 mg/kg, daily for 5 days) or LEED (1 mg/kg, once daily for fifteen days (qdx15)). FLT3 inhibitors midostaurin at 100mg/kg daily for 21 days and gilteritinib at 4mg/kg, qdx21 were administered as single agents or with HELD or LEED 5-azacytidine regimens. In MOLM-13 xenograft models, midostaurin alone and in combination with LEED or HELD 5-azacytidine significantly increased survival compared to vehicle alone (midostaurin vs vehicle, p = 0.027; LEED + midostaurin vs vehicle, p = 0.012; HELD + midostaurin vs vehicle, p = 0.003) (FIG. 17I). Low exposure, extended duration or HELD 5-azacytidine dosing in combination with midostaurin significantly increased survival compared to LEED or HELD 5-azacytidine, respectively (LEED + midostaurin vs LEED, p = 0.028; HELD + midostaurin vs HELD, p = 0.039). No significant changes in survival were observed between LEED or HELD in combination with midostaurin compared to midostaurin treatment alone. Median survival was increased with LEED or HELD 5-azacytidine in combination with midostaurin compared to vehicle or single agents (LEED + midostaurin = 45 days, HELD + midostaurin = 43 days, vehicle = 19 days, midostaurin = 34 days, LEED = 36 days, HELD = 32 days) (FIG. 17I).

In MV4-11 xenograft models, midostaurin alone and in combination with LEED or HELD 5-azacytidine increased survival compared to vehicle alone (Midostaurin vs vehicle, p = 0.0067; LEED + midostaurin vs vehicle, p = 0.0084; HELD + midostaurin vs vehicle, p = 0.0625). LEED or HELD 5-azacytidine dosing in combination with midostaurin significantly increased survival compared to either LEED or HELD 5-azacytidine alone (LEED + midostaurin vs LEED, p = <0.0001; HELD + midostaurin vs HELD, p = 0.0015). Furthermore, LEED or HELD 5-azacytidine in combination with midostaurin did not significantly increase survival compared to midostaurin alone (LEED + midostaurin vs midostaurin, p = 0.1704; HELD + midostaurin vs midostaurin, p = 0.8308). Median survival was increased with LEED or HELD 5-azacytidine in combination with midostaurin compared to vehicle or single agents HELD or LEED (LEED + midostaurin = 64.5, HELD + midostaurin = 59.5, vehicle = 29.5, LEED = 35, HELD = 35, midostaurin = 57) (FIG. 17J).

In MOLM-13 xenograft models, gilteritinib alone and in combination with LEED or HELD 5-azacytidine significantly increased survival compared to vehicle alone (gilteritinib vs vehicle, p = 0.003; LEED + gilteritinib vs vehicle, p = 0.003; HELD + gilteritinib vs vehicle, p = 0.003). Low exposure, extended duration or HELD 5-azacytidine dosing in combination with gilteritinib significantly increased survival compared to either LEED or HELD 5-azacytidine alone (LEED + gilteritinib vs LEED, p = 0.019; LEED + gilteritinib vs HELD, p = 0.004; HELD + gilteritinib vs LEED, p = 0.008; HELD + gilteritinib vs HELD, p = 0.003. Furthermore, LEED or HELD 5-azacytidine dosing in combination with gilteritinib significantly increased survival compared to gilteritinib alone (LEED + gilteritinib vs gilteritinib, p < 0.001; HELD + gilteritinib vs gilteritinib, p < 0.001). Median survival was increased with LEED or HELD 5-azacytidine in combination with gilteritinib compared to vehicle or single agents (LEED + gilteritinib = 45 days, HELD + gilteritinib = 43 days, vehicle = 19 days, gilteritinib = 34 days, LEED = 36 days, HELD = 32 days) (FIG. 17K).

Altogether, these results suggest that LEED or HELD 5-azacytidine in combination with a FLT3 inhibitor is significantly more effective at killing AML cells as compared to single agent 5-azacytidine or FLT3 inhibitor alone.

### Example 2

**Study Objectives:** The primary objective of the study is as follows.

Combination Arm A (oral 5-azacytidine + Ivosidenib arm): Objectives are: (1) To establish a maximum tolerated dose (MTD) or a maximum administered dose (MAD) of oral 5-azacytidine when given in combination with Ivosidenib in AML patients with an IDH1 mutation. (2) To establish the safety and tolerability of oral 5-azacytidine when given in combination with Ivosidenib in AML patients with an IDH1 mutation.

Combination Arm B (oral 5-azacytidine + Enasidenib arm): Objectives are: (1) To establish a maximum tolerated dose (MTD) or a maximum administered dose (MAD) of oral 5-azacytidine when given in combination with Enasidenib in AML patients with an IDH2 mutation. (2) To establish the safety and tolerability of oral 5-azacytidine when given in combination with Enasidenib in AML patients with an IDH2 mutation.

Combination Arm C (oral 5-azacytidine + FLT3 arm): Objectives are: (1) To establish a maximum tolerated dose (MTD) or a maximum administered dose (MAD) of oral 5-azacytidine when given in combination with a FLT3 inhibitor in AML patients with a FLT3 ITD or TKD mutation. (2) To establish the safety and tolerability of oral 5-azacytidine when given in combination with a FLT3 inhibitor in AML patients with a FLT3 ITD or TKD mutation.

Combination Arm D (oral 5-azacytidine + venetoclax arm): Objectives are: (1) To establish a maximum tolerated dose (MTD) or a maximum administered dose (MAD) of oral 5-azacytidine when given in combination with venetoclax in AML patients. (2) To establish the safety and tolerability of oral 5-azacytidine when given in combination with venetoclax in AML patients.

The secondary objectives are as follows: (1) To characterize the pharmacokinetics (PK) of oral 5-azacytidine in combination with Ivosidenib, Enasidenib, venetoclax, and a FLT3 inhibitor in AML patients; and (2) To assess the preliminary efficacy of oral 5-azacytidine in combination with Ivosidenib, Enasidenib, venetoclax, and a FLT3 inhibitor in AML patients.

The exploratory objectives are as follows: (1) To explore the relationships of drug exposure with efficacy, safety, pharmacodynamics and/or other exploratory endpoints; of oral 5-azacytidine when administered with Ivosidenib, Enasidenib, venetoclax, or a FLT3 inhibitor. (2) To evaluate the minimal residual disease (MRD), by flow cytometry (or relevant gene sequencing methods (for example, FLT3, IDH1/2 variant allele frequency (VAF), in blood and/or bone marrow. (3) To investigate the pertinent pharmacodynamics markers of oral 5-azacytidine and each of the combination partner agents Ivosidenib, Enasidenib, venetoclax, or a FLT3 inhibitor to fully understand the pharmacological response at each dose/schedule evaluated. (4) To evaluate additional exploratory molecular, cellular and/or metabolic markers to enable prognostic and/or predictive associations with efficacy and/or resistance in each of the treatment arms

Study endpoints are displayed below in Table 3.

| **Table 3: Study endpoints for the study.** | | | |
|---|---|---|---|
| **Endpoint** | **Name** | **Description** | **Timeframe** |
| Primary | MTD/MAD | Review of dose-limiting toxicities (DLTs) and safety by DRT. | Approximately 8 months |
| | Safety / tolerability | Type, frequency, seriousness and severity of AEs, and relationship of AEs to study treatment. | Approximately 13 months |
| Secondary | Pharmacokinetics | To evaluate the pharmacokinetics (PK) of oral 5-azacytidine and each combination drug | Approximately 13 months |
| | Overall Response Rate (as assessed by the investigator) | Rate of CR + CRi + MLFS + PR according to ELN 2017 AML response criteria. | Approximately 13 months |
| | CR+CRi, CRh | CR+CRi rate. | Approximately 13 months |
| | | CRi defined as response of bone marrow blast <5% but with absolute neutrophil count (ANC) < 1 × 10⁹/L or platelet < 100 × 10⁹/L | |
| | | CRh partial hematologic recovery: defined as less than 5% blasts in a BM aspirate sample with marrow spicules plus ANC > 0.5 × 10⁹/L and platelet count > 0.50 × 10⁹/L. | |
| | Time to response | Time from first dose of study drug to first documented CR/CRi/MLFS/PR according to criteria | Approximately 13 months |
| | Duration of Response | Time from the first documented MLFS/CR/CRi/PR to documented morphologic relapse, progression according to 2017 ELN response criteria, or death due to any cause, whichever occurs first | Approximately 13 months |
| | Event-Free Survival | Time from randomization to documented morphologic relapse, progression according to 2017 ELN response criteria, or death from any cause, whichever occurs first. | Approximately 13 months |
| | Overall Survival | Time from randomization to death due to any cause. | Approximately 30 months |
| | One-year survival rate | The probability of survival at 1 year from randomization | Approximately 30 months |
| | CR without minimal residual disease (CRMRD-) | CR with negativity for a genetic marker by RT-qPCR, or CR with negativity by multi-color flow cytometry | Approximately 8 months |
| | Hematologic improvement rate | Rate of HI-N + HI-P + HI-E according to IWG MDS HI criteria. | Approximately 8 months |
| Exploratory | Pharmacodynamics | To evaluate the pharmacodynamics (PD) of each combination agent to associate drug exposure with extent of target modulation and/or efficacy/tolerability. (in each treatment cohort during dose/schedule escalation to RP2D) | Approximately 8 months |
| | Correlative studies | Evaluation of molecular, cellular and metabolic markers in peripheral blood (PB) and/or bone marrow (BM) which may be predictive of antitumor activity and/or resistance. | Approximately 8 months |
| | | Utilizing both pre- and post-treatment bone marrow biopsy samples acquired for purposes of disease assessment, change in baseline of candidate biomarkers will also be assessed using molecular/cell based assays ie, multiparametric flow cytometry, DNA (gene sequencing), RNA expression (Nanostring or RNAseq), and/or in vitro assays. | |
| | | For example (but not limited to), MRD: flow cytometry or gene/PCR based assays (VAF); | |
| | | Gene mutation characterization: using next generation sequencing at baseline, during treatment, to relapse. | |
| | | Oral 5-azacytidine: DNA methylation changes in whole blood/PBMC/PBLs or BM | |
| | | Ivosidenib and Enasidenib (IDH1/2): αKG and 2-HG levels | |
| | | Venetoclax: BCL-2 expression /amplification levels and characterization of other Bcl-2 family members, or Bcl-2 mimetic assays | |
| | | Midostaurin and Gilteritinib: pFLT3/FLT3, pSTAT5/STAT5 | |

**Study Design** This is an open-label, Phase I, multicenter umbrella trial to evaluate the safety and tolerability of oral 5-azacytidine as a backbone in combination with biomarker directed and novel therapies as listed below:
Combination Arm A: oral 5-azacytidine + Ivosidenib in AML patients with IDH1 mutation,
Combination Arm B: oral 5-azacytidine + Enasidenib in AML patients with IDH2 mutation,
Combination Arm C: oral 5-azacytidine + a FLT3 inhibitor in AML patients with a FLT3 ITD or TKD mutation, and
Combination Arm D: oral 5-azacytidine + venetoclax in AML patients.

The study population consists of AML patients who are in first relapse, refractory to 1 or 2 standard induction treatments, or newly diagnosed AML patients who are not candidates to receive intensive IC. The study comprises of a Pre-screening Phase, Screening Phase, Treatment Phase and a Follow-up Phase.

**Pre-Screening Phase** All patients will sign a pre-screening consent for the collection and determination of specific AML mutation results. For patients who have recent local hematopathology and gene mutation testing available from bone marrow aspirate and/or peripheral blood samples since the time of relapse (or for newly diagnosed patients), those results will be used for screening. For patients without local hematopathology and gene mutation testing, a referral lab testing for specific AML mutations will be made available.

Patients will be assigned to the appropriate Combination Arm based on mutation testing results. All other AML patients without a specific AML mutation (IDH1/2 or FLT3 wild-type) or if the specific mutation Arm is filled/closed, and; who meet all eligibility criteria may enroll in oral 5-azacytidine + venetoclax Combination Arm (Arm D) until this arm is completed/closed.

Note: In the rare case in which an eligible patient has multiple mutations, assignment to the potential treatment combination will be based on joint investigator and medical monitor decision and documented in the source document. For example: A patient with both IDH1 and FLT3 mutations may be assigned to either the oral Ivosidenib or FLT3 treatment arms.

**Screening Phase** Following pre-screening, and confirmation of specific AML mutation eligible for study entry, all enrolled patients will undergo screening procedures during the screening period i.e., within 28 days prior to the start of study treatment. This will help determine study eligibility based on all eligibility criteria defined in the protocol.

**Treatment Phase** Upon confirmation of eligibility, patients will be enrolled and begin treatment in the appropriate Combination Arm. Combination Arms and specific dosing levels will be made available for enrollment as per the sponsor.

Study treatment should be initiated on Day 1 of each treatment cycle. Treatment cycles are 28 days in duration. Visit windows are ±1 day in Cycles 1 and 2; and starting in Cycle 3, windows are ±3 days.

During the treatment phase, the Dose Determination for each Combination Arm and dose level will be examined.

**Dose Determination** For the primary objective of determining the MTD/MAD for each combination arm during "dose determination", a modified toxicity probability interval (mTPI-2) method will be used. The mTPI-2 method relies upon a statistical probability algorithm, calculated using all patients treated in prior and current cohorts at the same dose level to determine where future cohorts should involve dose re-escalation, stay in current dose, or dose de-escalation. Planned doses for each Combination being tested are listed in Table 4. The dose determination rules based on the mTPI-2 method are illustrated in Table 5.

A Dose Review Team (DRT) will review all adverse events (AEs) experienced by patients during Cycle 1 of each dose level to determine the safety and tolerability of oral 5-azacytidine when administered in combination with Ivosidenib, Enasidenib, a FLT3 inhibitor, and venetoclax.

Starting dose levels are based on currently used doses in larger trials for oral 5-azacytidine, Ivosidenib, and a FLT3 inhibitor; and approved doses for combination agents of Enasidenib, a FLT3 inhibitor, and venetoclax. Intermediate dose levels and adjustments to dosing may be discussed and modified by the DRT. De-escalation to Dose Level -1A or -1B, will be based on the available safety information and attribution of the toxicity to either oral 5-azacytidine (to Level -1A) or the combination agent (Level -1B). If toxicity is attributed to both drugs or unable to be assessed, both dose levels may be opened upon discussion with the DRT. Intrapatient dose adjustments for oral 5-azacytidine may occur after discussion with the Celgene medical monitor up to 300mg QD for 21 days.

The first group of patients enrolled at a new dose level will begin treatment in a staggered manner as determined by the sponsor.

| **Table 4: Dose Determination Starting Dose Levels for Combination Arms** | | | | |
|---|---|---|---|---|
| | **Combination A: oral 5-azacytidine + Ivosidenib** | **Combination B: oral 5-azacytidine + Enasidenib** | **Combination C: oral 5-azacytidine + Midostaurin** | **Combination D: oral 5-azacytidine + venetoclax** |
| Dose Level +1 | oral 5-azacytidine 300mg QD x 21 d + | oral 5-azacytidine 300mg QD x 21 d + | oral 5-azacytidine 300mg QD x 21 d + | oral 5-azacytidine 300mg QD x 21 d + |
| | Ivosidenib 500mg QD | Enasidenib 100mg QD | Midostaurin 50mg BID | Venetoclax 400mg QD |
| **Starting Dose (Dose Level 0)** | oral 5-azacytidine 300mg QD x 14 d + | oral 5-azacytidine 300mg QD x 14 d + | oral 5-azacytidine 300mg QD x 14 d + | oral 5-azacytidine 300mg QD x 14 d + |
| | Ivosidenib 500mg QD | Enasidenib 100mg QD | Midostaurin 50mg BID | Venetoclax 400mg QD |
| Dose Level -1A | oral 5-azacytidine 200mg QD x 14 d + | oral 5-azacytidine 200mg QD x 14 d + | oral 5-azacytidine 200mg QD x 14 d + | oral 5-azacytidine 200mg QD x 14 d + |
| | Ivosidenib 500mg QD | Enasidenib 100mg QD | Midostaurin 50mg BID | Venetoclax 400mg QD |
| Dose Level -1B | oral 5-azacytidine 300mg QD x 14 d + | oral 5-azacytidine 300mg QD x 14 d + | oral 5-azacytidine 300mg QD x 14 d + | oral 5-azacytidine 300mg QD x 14 d + |
| | Ivosidenib 250mg QD | Enasidenib 50mg QD | Midostaurin 25mg BID | Venetoclax 300mg QD |
| Dose Level -2 | oral 5-azacytidine 200mg QD x 14 d + | oral 5-azacytidine 200mg QD x 14 d + | oral 5-azacytidine 200mg QD x 14 d + | oral 5-azacytidine 200mg QD x 14 d + |
| | Ivosidenib 250mg QD | Enasidenib 50mg QD | Midostaurin 25mg BID | Venetoclax 300mg QD |

A cycle is defined as 28 days. Study visits will occur weekly for the first two cycles, then every two weeks (Day 1 and Day 15) starting from Cycle 3. After dose determination is complete for each combination arm, all ongoing patients will continue to receive the same dose and treatment until discontinuation.

Modified Toxicity Probability Interval (mTPI-2). During dose determination, a modified toxicity probability interval (mTPI-2) method design will be used to determine the Maximum Tolerated Dose (MTD) or Maximum Administered Dose (MAD) for the combination. The MTD is defined as the highest dose level evaluated that does not exceed the target DLT probability of 0.30 in the first cycle of the treatment. The mTPI-2 determination is independent for every dose level in each Combination Arm. The dose determination rules based on the mTPI-2 method are illustrated in Table 5.

| **Table 5: mTPI-2 Dose Determination Rules** | | | | | | | | | | |
|---|---|---|---|---|---|---|---|---|---|---|
| | 3 | 4 | 5 | 6 | 7 | 8 | 9 | 10 | 11 | 12 |
| 0 | **E** | **E** | **E** | **E** | **E** | **E** | **E** | **E** | **E** | **E** |
| 1 | **S** | **S** | **E** | **E** | **E** | **E** | **E** | **E** | **E** | **E** |
| 2 | **D** | **D** | **D** | **S** | **S** | **S** | **E** | **E** | **E** | **E** |
| 3 | **DU** | **DU** | **D** | **D** | **D** | **D** | **S** | **S** | **S** | **S** |
| 4 | | **DU** | **DU** | **DU** | **D** | **D** | **D** | **D** | **D** | **S** |
| 5 | | | **DU** | **DU** | **DU** | **DU** | **DU** | **D** | **D** | **D** |
| 6 | | | | **DU** | **DU** | **DU** | **DU** | **DU** | **DU** | **D** |
| 7 | | | | | **DU** | **DU** | **DU** | **DU** | **DU** | **DU** |
| 8 | | | | | | **DU** | **DU** | **DU** | **DU** | **DU** |
| 9 | | | | | | | **DU** | **DU** | **DU** | **DU** |
| 10 | | | | | | | | **DU** | **DU** | **DU** |
| 11 | | | | | | | | | **DU** | **DU** |
| 12 | | | | | | | | | | **DU** |

| | | | | | | | | | | |
|---|---|---|---|---|---|---|---|---|---|---|
| Target toxicity probability = **0.3,** ε₁ = **0.05,** ε₂= **0.05** * **Column** indicates the number of patients treated. **Row** indicates the number of patients with DLTs * **E:** Escalate to the next higher dose; S: Stay at the same dose; **D:** De-escalate to the previous lower dose; **DU:** De-escalate to the previous lower dose and the current dose will never be used again in the trial. | | | | | | | | | | |

In general, the rules for dose-finding using the mTPI-2 method include the following:
- Each Cohort within a Dose Level Arm will enroll 2-6 patients; (Note: at least 3 patients should be enrolled and treated in the first cohort at a new dose level)
- When at least 3 patients are evaluated for DLT at the current dose level or experience a DLT, the next cohort will be enrolled and assigned to the dose level per Table 4. All patients evaluated at the same dose level, including prior cohort(s) and current cohort, will be used to determine the dose level of the next cohort.
- If a patient does not receive the protocol-specified administrations of the study medication within the DLT observation period for reasons other than study drug-related toxicity, another patient will be enrolled as a replacement at the current dose level.
- The determination rules are independent for each Dose Level in every Combination Arm.
- The dose-finding phase for each combination will conclude when at least 10 patients have been treated at a dose level that is predicted to be the MTD or doses appear to be overly toxic and the MTD cannot be determined in the current trial.
- Patients must complete at least 80% of the planned total dose of oral 5-azacytidine and the combination agent, or experience a DLT during DLT observation period, in order to be considered evaluable for DLT. The dose escalation decision rules are defined in the Statistical Methods Section.

The DLT observation period for evaluation for each dose level is the first cycle (28 days) of treatment of the current dose level, or up to the DLT if it occurs earlier.

The following is an example of dose finding procedure for Combination Arm B (oral 5-azacytidine + Enasidenib). The four predetermined dose levels are:
- **Dose Level +1:** oral 5-azacytidine 300 mg QD Days 1-21 + Enasidenib 100 mg QD Days 1-28;
- **Starting Dose Level 0:** oral 5-azacytidine 300 mg QD Days 1-14 + Enasidenib 100 mg QD Days 1-28;
- **Dose Level -1A:** oral 5-azacytidine 200 mg QD Days 1-14 + Enasidenib 100 mg QD Days 1-28;
- **Dose Level -1B:** oral 5-azacytidine 300 mg QD Days 1-14 + Enasidenib 50 mg QD Days 1-28;
- **Dose Level -2:** oral 5-azacytidine 200 mg QD Days 1-14 + Enasidenib 50 mg QD Days 1-28.

The dose-finding will start with enrollment of 3 patients and treatment at the Starting Dose Level (Dose Level 0): oral 5-azacytidine 300 mg QD Days 1-14 + Enasidenib 100 mg QD Days 1-28. By the end of the DLT observation period of the 3 patients, the next cohort will be enrolled and assigned to the dose level which is determined based on the number of DLTs and the mTPI-2 rule table (Table 5):
- If 3/3 DLTs - De-escalate to Dose Level -1A or -1B (depending on the attributed agent), enroll and assign this new dose level cohort with 3 patients. The current or higher dose is too toxic and cannot be explored again.
- If 2/3 DLTs - De-escalate to Dose Level -1A or -1B, enroll and assign this new dose level cohort with 3 patients.
- If 1/3 DLT - Stay at the same dose level (Dose Level 0), enroll additional 2 patients (2^{nd} cohort). Note the total number of patients at Dose Level 0 becomes 5.
- If 0/3 DLT - Stay at the same dose level (Dose Level 0) with an option to escalate (if applicable), enroll an additional 2 patients (2^{nd} cohort). If the dose is escalated, assign a new dose level cohort with 3 patients.

If a new Dose Level is started, the mTPI-2 rule table resets and the new dose cohort level is evaluated separately from the prior Dose Level.

At the end of the DLT observation period of the 2^{nd} cohort for a dose level, the next cohort (3^{rd} cohort) will be enrolled and assigned to the dose level which is determined based on the number of DLTs and the mTPI-2 rule table (Table 4). All patients and their DLTs at the same dose level will be used, including the current cohort and any prior cohort(s).

Except for requiring at least 3 patients in the first cohort at each dose level, the size of the additional cohorts in a dose level can be flexible. For example, if the dose level continues at the same level per the mTPI-2 rules, the sequence of the cohort sizes could be 3, 2, 2, 3, etc.

The dose-finding will conclude when at least 10 patients have been treated at a dose that is predicted to be the MTD or if doses appear to be overly toxic and the MTD cannot be determined in the current combination arm for the study.

After determination of the MTD/MAD, the DRT will review all safety data for patients who were treated in the combination arm (Arms A through E) to determine the RP2D for the Combination Arm which may be used in an expansion cohort.

End of Treatment (EOT): Treatment will continue until documented disease progression, unacceptable adverse event(s), intercurrent illness that prevents further administration of treatment, investigator's decision to withdraw the patient, patient withdraws consent, pregnancy of the patient, noncompliance with trial treatment or procedure requirements, or administrative reasons.

At the EOT visit, an evaluation will be performed with a visit window of ±7 days. If the EOT occurs during a scheduled visit, all EOT assessments must also be completed.

**Follow-up Phase** After the end of treatment, each patient will be followed for 28 days for AE monitoring. Patients who discontinue treatment for reasons other than disease progression will have post-treatment follow-up for disease status until disease progression, initiating a non-study cancer treatment, withdrawing consent, or becoming lost to follow-up.

The Follow-up Phase will have assessments every 28 days for the first year then every 3 months thereafter.

In addition, all patients will be followed by telephone contact for survival until death, withdrawal of consent or the end of the trial, whichever comes first.

**Study Population / Estimated No. Patients** Up to approximately 72 patients will be enrolled in this study (4 Combination Arms x 18 patients each).

For each Combination Arm dose level, approximately 12 patients may be enrolled to evaluate safety, tolerability and preliminary efficacy. Depending on the dose level used for dose determination, up to 18 patients may be enrolled for each Combination Arm.

Additional patients for dose expansion may also be enrolled after completion of the primary objective for each Combination Arm.

**Key Inclusion Criteria** Patients must satisfy the following criteria to be enrolled in the study:
1. Patient is ≥ 18 years of age at the time of signing the informed consent form (ICF).
2. Patients with AML as defined by the WHO Classification:
   a. First relapse (i.e. recurring after having achieved an initial response [CR/CRi] to chemotherapy except promyelocytic leukemia [APML]); or
   b. Persisting/refractory after 1 to 2 primary induction courses (ie, no response after 1 to 2 prior chemotherapy regimens); or
   c. Newly diagnosed AML who are not candidates for intensive chemotherapy
3. Patients must have the following AML specific mutation in bone marrow aspirate and/or peripheral blood sample documented since last progression/relapse (or recent testing for newly diagnosed):
   a. For Combination Arm -A (IDH1): Have confirmation of an IDH1 mutation
   b. For Combination Arm -B (IDH2): Have confirmation of an IDH2 mutation
   c. For Combination Arm -C (FLT3): Have confirmation of a FLT3 mutation (ITD or TKD)
   d. For Combination Arm D (BCL-2): Patients without any of the specific mutations listed or have a mutation in a closed combination arm above may be enrolled into this oral 5-azacytidine+ venetoclax arm.
4. Patient has Eastern Cooperative Oncology Group (ECOG) performance status of 0, 1 or 2
5. Patient has adequate organ function defined as:
   - Aspartate aminotransferase (AST)/serum glutamic oxaloacetic transaminase (SGOT) and alanine aminotransferase (ALT)/serum glutamic pyruvic transaminase (SGPT) ≤ 2.5 x upper limit of normal (ULN), unless considered due to leukemic organ involvement
   - Serum total bilirubin ≤ 1.5 x ULN, unless considered due to Gilbert's syndrome (eg, a gene mutation in UGT1A1) or leukemic organ involvement
   - Creatinine clearance > 30 mL/min based on the Modification of Diet in Renal Disease (MDRD) glomerular filtration rate (GFR): $GFR \left(mL/min/1.73 m2\right) = 175 \times \left(serum creatinine\right) - 1.154 \times \left(Age\right) - 0.203 \times \left(\begin{matrix}0.742 if \\ female\end{matrix}\right) \times \left(1.212 if African American\right)$
6. Agree to serial bone marrow aspirate/biopsies.
7. Females of childbearing potential (FCBP)* must:
   Have two negative pregnancy tests as verified by the Investigator prior to starting study therapy. She must agree to ongoing pregnancy testing during the course of the study, and after end of study therapy. This applies even if the patient practices true abstinence* from heterosexual contact.
   Either commit to true abstinence* from heterosexual contact (which must be reviewed on a monthly basis and source documented) or agree to use, and be able to comply with highly effective contraception without interruption, -3 days prior to starting investigational product, during the study therapy (including dose interruptions), and for 90 days after discontinuation of study therapy, or longer if required for each compound and/or by local regulations.
      For Combination Arm A: the timeframe is extended to 4 (four) months following the last study treatment dose
      For Combination Arm B: the timeframe is extended to 4 (four) months following the last study treatment dose
      For Combination Arm D: the timeframe is extended to 6 (six) months following the last study treatment dose.
8. Male patients must practice true abstinence* (which must be reviewed on a monthly basis) or agree to use a condom during sexual contact with a pregnant female or a female of childbearing potential while participating in the study, during dose interruptions and for at least 90 days following investigational product discontinuation, or longer if required for each compound and/or by local regulations, even if he has undergone a successful vasectomy.
   For Combination Arm A: the timeframe is extended to 4 (four) months following the last study treatment dose
   For Combination Arm B: the timeframe is extended to 4 (four) months following the last study treatment dose
   For Combination Arm D: the timeframe is extended to 6 (six) months following the last study treatment dose.
9. Patient must understand and voluntarily sign an ICF prior to any study-related assessments/procedures being conducted.
10. Patient is willing and able to adhere to the study visit schedule and other protocol requirements.

**Exclusion Criteria** The presence of any of the following will exclude a patient from enrollment:
1. Patient is suspected or proven to have acute promyelocytic leukemia based on morphology, immunophenotype, molecular assay, or karyotype
2. Patient has AML secondary to chronic myelogenous leukemia or patient is BCR-ABL1 t(9;22)(q34;q11) positive.
3. Patient has a white blood cell count of >15,000 cells/mcl (15x 10⁹/L). Note: Hydroxyurea is allowed up to 72 hours prior to the screening blood draw and limited use only during Cycle 1 for cytoreduction (to control high white blood cell count).
4. Patient has received systemic anticancer therapy or radiotherapy < 28 days prior to the start of study treatment. Note that hydroxyurea is allowed prior to the start of study treatment for the control of leukocytosis.
5. Patient has received therapy with hypomethylating agents for AML and did not achieve CR/CRi/PR/MLFS/SD during first line therapy
6. Patient has received investigational agents < 30 days or 5 half-lives, whichever is longer, prior to the start of study treatment
7. Patient has undergone HSCT within 90 days prior to the start of study treatment, or on immunosuppressive therapy post HSCT at the time of screening, or with graft-versus-host disease (GVHD). The use of a stable dose of oral steroid post-HSCT, or topical steroids for ongoing skin GVHD is permitted.
8. Patient has persistent, clinically significant non-hematologic toxicities from prior therapies which have not recovered to < Grade 2
9. Patient has or is suspected of having central nervous system (CNS) leukemia. Evaluation of cerebrospinal fluid is only required if CNS involvement by leukemia is suspected during screening.
10. Patient has active uncontrolled systemic fungal, bacterial, or viral infection (defined as ongoing signs/symptoms related to the infection without improvement despite appropriate antibiotics, antiviral therapy, and/or other treatment). The patient should be afebrile for at least 72 hours.
11. Patient has immediate life-threatening, severe complications of leukemia such as uncontrolled bleeding, pneumonia with hypoxia or shock, and/or disseminated intravascular coagulation
12. Patient has prior history of malignancy, other than MDS, MPN or AML, unless the patient has been free of the disease for ≥ 1 year prior to the start of study treatment. However, patients with the following history/concurrent conditions are allowed:
   - Basal or squamous cell carcinoma of the skin
   - Carcinoma in situ of the cervix
   - Carcinoma in situ of the breast
   - Incidental histologic finding of prostate cancer (T1a or T1b using the tumor, nodes, metastasis clinical staging system)
13. Patient is known seropositive or active infection with human immunodeficiency virus (HIV), or active infection with hepatitis B virus (HBV) or hepatitis C virus (HCV)
14. Patient is known to have dysphagia, short-gut syndrome, gastroparesis, or other conditions that limit the ingestion or gastrointestinal absorption of drugs administered orally
15. Patients has uncontrolled hypertension (systolic blood pressure [BP] > 180 mmHg or diastolic BP > 100 mmHg) or has not been stable for at least 1 month prior to treatment
16. Significant active cardiac disease within the previous 6 months prior to signing the ICF, including:
   New York Heart Association (NYHA) Class III or IV congestive heart failure;
   Unstable angina or angina requiring surgical or medical intervention;
   Significant cardiac arrhythmia; and/or
   Myocardial infarction
17. Patient is a pregnant or lactating female
18. Patient has known or suspected to have hypersensitivity to any of the components of the assigned study treatments
19. Patient has any significant medical condition, laboratory abnormality, or psychiatric illness that would prevent the patient from participating in the study
20. Patient has any condition including the presence of laboratory abnormalities, which places the patient at unacceptable risk if he/she were to participate in the study
21. Patient has any condition that confounds the ability to interpret data from the study
22. Additional exclusion criteria for patients based on the planned Combination Arm are:
   a. For Combination Arm -A (IDH1):
      - Patient has QTc interval (ie, Fridericia's correction [QTcF]) ≥ 450 ms or other factors that increase the risk of QT prolongation or arrhythmic events (eg, heart failure, hypokalemia, family history of long QT interval syndrome) at screening
      - Patient is taking those medications that are known to prolong QT interval unless the patient can be transferred to other medications at least 5 half-lives prior to the start of study treatment
      - Patient has significant active cardiac disease within 6 months prior to the start of study treatment; acute coronary syndrome (ACS); and/or stroke; or left ventricular ejection fraction (LVEF) < 40% by echocardiogram (ECHO) or multi-gated acquisition (MUGA) scan obtained within 28 days prior to the start of study treatment
   b. For Combination Arm -B (IDH2):
      - Patient is taking the breast cancer resistance protein (BCRP) transporter-sensitive substrate rosuvastatin should be excluded from the study unless the patient can be transferred to other medications at least 5 half-lives prior to the start of study treatment
      - Patient is taking the following sensitive CYP substrate medications that have a narrow therapeutic range are excluded from the study unless the patient can be transferred to other medications at least 5 half-lives prior to the start of study treatment: paclitaxel and docetaxel (CYP2C8), phenytoin (CYP2C9), S-mephenytoin (CYP2C19), thioridazine (CYP2D6), theophylline, and tizanidine (CYP1A2)
   c. For Combination Arm -C (non-specific FLT3):
      Patients with hypokalemia and hypomagnesemia at Screening (values below lower limit of normal [LLN]).
      Patient has a history of congestive heart failure NYHA class 3 or 4, unless a screening echocardiogram performed within 3 months prior to study entry results in a left ventricular ejection fraction that is ≥ 45%.
      Patients with mean of triplicate Fridericia-corrected QT interval (QTcF) > 450 ms at Screening.
      Patients with Long QT Syndrome at Screening.
      Patient requires treatment with concomitant drugs that are strong inducers of cytochrome P450 (CYP)3A.
      Patient requires treatment with concomitant drugs that are strong inhibitors or inducers of P glycoprotein (P-gp) with the exception of drugs that are considered absolutely essential for the care of the patient.
      Patient requires treatment with concomitant drugs that target serotonin 5-hydroxytryptamine receptor 1 (5HT1R) or 5-hydroxytryptamine receptor 2B (5HT2BR) or sigma nonspecific receptor with the exception of drugs that are considered absolutely essential for the care of the patient.
   d. For Combination Arm -D (BCL-2):
      - Received strong CYP3A inhibitors, moderate CYP3A inhibitors, strong CYP3A inducers, moderate CYP3A inducers, strong CYP2C8 inhibitors, CYP2C8 substrates, or OATP1B1/3 substrates within 7 days prior to initiation of study treatment
      - Received strong CYP2C8 inducers within 14 days prior to initiation of study treatment

**Length of Study** The full length of the study is expected to be approximately 30 months including recruitment, screening, treatment, and follow up. For a single patient, the expected duration of the study is approximately 8 months, including a screening period phase of up to 28 days.

The End of Trial is defined as either the date of the last visit of the last patient to complete the post-treatment follow-up, or the date of receipt of the last data point from the last patient that is required for primary, secondary, and/or exploratory analysis, as pre-specified in the protocol, whichever is the later date.

**Overview of Key Efficacy Assessments** Response to treatment will be assessed by the investigators according to the European Leukemia Net (ELN) AML Response Criteria (Döhner, H et al., Blood, 2017 Jan 26, 129(4): 424-47). Hematologic improvement (HI) in patients with newly diagnosed AML will also be assessed according to the IWG myelodysplastic syndromes HI criteria (Cheson, 2006). Patients are to undergo end-of-treatment evaluations when study treatment is discontinued. The reason for treatment discontinuation will be recorded in the electronic case report form (eCRF) pages and in the source document.

At baseline, a bone marrow aspirate and biopsy sample are required. Serial blood and bone marrow aspirate sampling (with bone marrow biopsy if needed) will be used to determine response to therapy starting at Cycle 2 Day 1 and will be assessed every other cycle (Cycle 4, Cycle 6, etc) until remission (or EOT if no remission). At remission, an additional bone marrow aspirate and biopsy are required. During remission, an aspirate should be collected every 4 months or when clinically indicated. At EOT, an additional bone marrow aspirate will be collected. Response will be assessed locally according to 2017 ELN criteria based on reported hematology laboratory parameters, peripheral blood smear, bone marrow aspirates and/or biopsies. The site needs to ensure peripheral blood and BMA/BMB samples are collected and stored for exploratory testing at the time of every bone marrow collection.

Patients who discontinue study treatment prior to relapse or progression will complete monthly site visits until confirmation of relapse or progression. For patients who have discontinued study treatment due to relapse or progression, monthly follow up can be performed by site visits or phone calls. Patients will be followed until they have died, are lost to follow up, withdraw consent for further data collection, or until study closure.

**Overview of Key Safety Assessments** All patients will be monitored for AEs during the study. Adverse events will be graded in severity according to the guidelines outlined in the NCI Common Terminology Criteria for Adverse Events (CTCAE) version 5.

**Dose Limiting Toxicities (DLTs)** Toxicity severity will be graded according to the Common Terminology Criteria for Adverse Events (CTCAE) version 4.03. DLTs during dose determination will be based on adverse events that occur during the DLT observation period. For this study, the DLT observation period is defined as the first treatment cycle (28 days) and patients will be evaluated at the completion of each cohort at a dose level. If DLT events occurs during the 2nd treatment cycle, no new patients may begin treatment until review and discussion by the DRT. Events that are considered DLTs based on their duration will be considered a DLT if they start during the DLT assessment period.

Any of the following events will be considered a DLT unless the event can be attributed by the investigator to a clearly identifiable cause such as underlying illness or disease progression, other concurrent illness, or concomitant medication:
- A hematological DLT is defined as non-recovery from a Grade 4 hematological toxicity within 42 days from Day 1 of the previous cycle.
   For the purpose of DLT determination, the Grade 4 *hematological toxicity* is defined as: (i) ANC <0.5×10⁹/L (Grade ≥4) and/or (ii) PLT count <25.0×10⁹/L (Grade ≥4)
   A hematological DLT can only be assigned if the delay in recovery per investigator's judgement is due to treatment-induced toxicity rather than residual effect of AML (a bone marrow sample may be indicated to document that this is not due to AML with bone marrow blasts <5%).
      - For the purpose of DLT determination, *recovery* from hematological toxicity is defined as an increase in cell counts from nadir by ≥50% of the reduction from baseline observed at the time of nadir
      - The use of G-CSF or platelet transfusions are prohibited during the DLT evaluation period. Patients receiving these treatments will be determined as not evaluable and will be replaced.
      - Patients who enter study with low hematology counts (Grade 3 and Grade 4), will not be considered evaluable for a hematologic DLT unless a clinically meaningful change has occurred (non-recovery of counts within 42 days of Day 1 of the previous cycle and a mandatory bone marrow assessment indicates a treatment induced bone marrow toxicity).
   A non-hematological DLT is defined as the occurrence of a Grade ≥3 clinically significant non-hematologic adverse event or abnormal laboratory value occurring during the first cycle on study that cannot be attributed by the investigator to a clearly identifiable cause such as disease progression, underlying illness, concurrent illness, or concomitant medication, with the following EXCEPTIONS:
      Grade 3 fatigue
      Grade 3 nausea, vomiting, or diarrhea that can be managed to ≤ Grade 2 (or baseline) with standard antiemetic or antidiarrheal medications within 72 hours
      For IDH1 or IDH2 combination: Grade 3 or 4 leukocytosis, or Grade 3 differentiation syndrome (DS), that can be managed to < Grade 3 with hydroxyurea and/or corticosteroids
      Grade 3 Tumor Lysis Syndrome unless controlled by medical management

For any DLT occurring during the DLT observation period which may require interruption of both oral 5-azacytidine and/or the combination agent, reintroduction at a reduced dose after the toxicity grade has returned to Grade ≤1 or to baseline may occur based on discussion with the Celgene Medical Monitor. The start of the subsequent cycle may need to be delayed to allow for recovery from a DLT or any other adverse event. A dose modification schedule will be available for each combination arm as guidance. Patients with neutropenia or thrombocytopenia as a consequence of the disease prior to the start of therapy do not require treatment interruptions for myelosuppression. However, the investigator may use clinical judgement based on patient's disease status, clinical condition, and comorbidities to make the decision regarding treatment modifications. Dose reductions of the study treatment in these patients should be considered on an individual case basis and discussed with the Medical Monitor.

Any reduced Dose Level of oral 5-azacytidine and/or the combination agent will be jointly defined by the investigator and the Celgene Medical Monitor (MM). The dose may be increased thereafter upon joint determination of the investigator and the Celgene MM. All decisions regarding continued dosing for individual patients with DLT will be medically managed by the investigator, and per discussion with the Celgene MM, as appropriate.

All patients are allowed to receive antibiotic prophylaxis (prohibited medication excluded) per standard institution guidelines during the DLT observation period.

**Criteria for Evaluation** Safety evaluations include, but are not limited to capturing AEs, clinical laboratory testing (hematology and chemistry), physical examinations, vital sign measurements, electrocardiogram (ECG) testing, as a measure of safety and tolerability for the entire study duration.

For each Combination Arm, dose escalation/de-escalation decisions during dose determination will be guided by assessment of DLTs and based on the cumulative number of patients who experience a DLT at a given dose level.

**Study Treatments** Oral 5-azacytidine will be administered orally once daily on Days 1-14 (or 21) of each 28-day treatment cycle. Patients should be instructed to take their daily dose at approximately the same time each day. Each dose should be taken with approximately 240 mL (8 ounces) of room temperature water. Oral 5-azacytidine may be taken on an empty stomach or with food.

Ivosidenib is administered orally once a day (QD) on Days 1-28 of each 28-day cycle. Patients should be instructed to take their daily dose at approximately the same time each day ± 6 hours. Each dose should be taken with a glass of water and consumed over as short a time as possible. Patients should be instructed to swallow tablets whole and to not chew the tablets.

Enasidenib is administered orally once a day (QD) on Days 1-28 of each 28-day cycle. Patients should be instructed to take their daily dose at approximately the same time each day ± 6 hours. Each dose should be taken with a glass of water and consumed over as short a time as possible. Patients should be instructed to swallow tablets whole and to not chew the tablets. Fasting is required for 2 hours prior to and 1 hour following Enasidenib administration. Water is allowed during fasting.

Midostaurin is administered orally twice daily (BID) on Days 1-28 of each 28-day cycle. Patients should be instructed to take their doses at approximately the same time each day ± Y hours. Each dose should be taken with a meal and water, and consumed over as short a time as possible. Patients should be instructed to swallow tablets whole and to not chew the tablets

Gilteritinib is administered orally once a day (QD) on Days 1-28 of each 28-day cycle. Patients should be instructed to take their daily dose at approximately the same time each day ± Y hours. Each dose should be taken with a meal and water, and consumed over as short a time as possible. Patients should be instructed to swallow tablets whole and to not chew the tablets.

Venetoclax is administered orally once a day (QD) on Days 1-28 of each 28-day cycle. A brief dose ramp-up occurs for Cycle 1 with the dosing of 100mg on Day 1, 200mg on Day 2, and 400mg on Day 3. Patients should be instructed to take their daily dose at approximately the same time each day ± 6 hours. Each dose should be taken with a meal and water, and consumed over as short a time as possible. Patients should be instructed to swallow tablets whole and to not chew the tablets.

In cases of dose interruption/modification, cycle visits will continue on the same schedule and timing (e.g. Cycle 3 is 28 days after Cycle 2, etc). If a drug interruption occurs, the other study drug may continue the treatment schedule.

**Statistical Methods** At the end of the dose determination, the isotopically transformed DLT rate which is closest to the target DLT rate will be selected as the estimated MTD/MAD. If two or more doses are equally close to the target DLT rate, the highest dose among the tied doses will be chosen if they are less than the target DLT rate, or the lowest dose among the tied doses will be chosen if they are higher than the target DLT rate.

Statistical analyses will be primarily descriptive in nature. Tabulations will be produced for disposition, demographic and baseline disease characteristics, safety, PK, PD, and clinical activity parameters. Categorical data will be summarized by frequency distributions (numbers and percentages of patients) and continuous data will be summarized by descriptive statistics (mean, standard deviation, median, minimum, and maximum). Data will be summarized by dose level and overall where appropriate.

The results of the clinicaly study will show that: (1) oral 5-azacytidine in combination with ivosidenib at the maximum tolerated dose (MTD) and/or maximum administered dose (MAD) is safe and tolerated in AML patients with an IDH1 mutation; (2) oral 5-azacytidine in combination with enasidenib at the maximum tolerated dose (MTD) and/or maximum administered dose (MAD) is safe and tolerated in AML patients with an IDH2 mutation; (3) oral 5-azacytidine in combination with an FLT3 inhibitor, such as midostaurin and gilteritinib, at the maximum tolerated dose (MTD) and/or maximum administered dose (MAD) is safe and tolerated in AML patients with a FLT3 ITD or TKD mutation; and/or (4) oral 5-azacytidine in combination with venetoclax at the maximum tolerated dose (MTD) and/or maximum administered dose (MAD) is safe and tolerated in AML patients.

### Example 3

**Study Objectives:** The primary objective of the study is as follows.

Dose-finding (Phase 1b): To establish maximum tolerated dose (MTD) or maximum administered dose (MAD) and/or the recommended Phase 2 dose (RP2D) by evaluating safety and tolerability of oral 5-azacytidine in combination with venetoclax or gilteritinib in patients with AML or higher-risk MDS.

Dose expansion (Phase 2): To assess efficacy of oral 5-azacytidine when given in combination with venetoclax at the RP2D in newly diagnosed patients with AML or higher-risk MDS.

The secondary objectives are as follows:
Dose-finding (Phase 1b): To assess the preliminary efficacy of oral 5-azacytidine in combination with venetoclax or gilteritinib in patients with AML or higher-risk MDS.
Dose expansion (Phase 2): To further evaluate safety and tolerability of oral 5-azacytidine when given in combination with venetoclax at the RP2D in newly diagnosed patients with AML or higher-risk MDS.
All parts (Phase 1b/2): To characterize the pharmacokinetics (PK) of oral 5-azacytidine combination with venetoclax or gilteritinib.

The exploratory objectives are as follows: All parts (Phase 1b/2): (1) To characterize the pharmacodynamics (Pd) to understand the mechanistic effects of oral 5-azacytidine in combination with venetoclax or gilteritinib. (2) To evaluate the MRD response rate and MRD conversion rate by multicolor flow cytometry (MFC) and/or Next Generation Sequencing (NGS). (3) To explore the duration of MRD response by assessments of bone marrow aspirate (BMA) and examination of peripheral blood smears (PBS). (4) To investigate the relationship between PK, PD biomarkers, and/or clinical outcomes of oral 5-azacytidine in combination with venetoclax or gilteritinib. (5) To evaluate molecular and/or cellular markers in the BM and blood that may be predictive of efficacy of oral 5-azacytidine in each combination

Study endpoints are displayed below in Table 6.

| **Table 6: Study endpoints for the study.** | | | |
|---|---|---|---|
| **Endpoint** | **Name** | **Description** | **Timeframe** |
| AML Cohort - Phase 1b | | | |
| Primary | MTD/MAD, and/or RP2D | Dose-limiting toxicities (DLTs) evaluated using NCI CTCAE Version 5.0 | From first dose (C1D1) to the end of Cycle 1 |
| | Safety / tolerability | Type, frequency, seriousness and severity of AEs (using NCI CTCAE Version 5.0), and relationship of AEs to study treatment, clinical laboratory evaluations | From informed consent form (ICF) signature to 28 days after last dose of IP |
| Secondary | Preliminary efficacy | CR/CRh rate: defined as the rate of achieving CR or CRh | Preliminary efficacy |

| AML Cohort - Phase 2 | | | |
|---|---|---|---|
| Primary | Efficacy at RP2D | CR/CRh rate: defined as the rate of achieving CR or CRh | From first dose to 28 days after last dose |
| Secondary | Safety / Tolerability | Type, frequency, seriousness and severity of AEs (using NCI CTCAE Version 5.0), and relationship of AEs to study treatment, clinical laboratory evaluations | From ICF signature to 28 days after last dose of IP |

| AML Cohort - Phase 1b and 2 | | | |
|---|---|---|---|
| Secondary | Efficacy | ORR defined as the rate of achieving CR, CRi, CRp, PR, or MLFS | From first dose to 28 days after last dose |
| | | Response assessed by Investigator according to the European Leukemia Net (ELN) AML Response Criteria (Döhner, H et al., Blood, 2017 Jan 26, 129(4): 424-47) and IWG for AML Response Criteria (Cheson B.D. et al., J Clin Oncol. 2003, 21(24): 4642-92003). | |
| | | Time to response (CR, CRi, CRp, PR, MLFS) | From first dose to first response CR, CRi, CRp, PR, MLFS |
| | | DOR (CR/CRh) | From first response CR or CRh to first relapse or death, whichever occurs first |
| | | DOR (CR/ CRi/ CRp/ PR/ MLFS) | From first response (CR, CRi, CRp, PR, or MLFS) to first relapse, disease progression, or death, whichever occurs first |
| | | Event-Free Survival (EFS) Relapse-Free Survival (RFS) | From first dose to first relapse, disease progression, or death from any cause, whichever occurs first From first response to relapse, death, or last follow-up |
| Exploratory | MRD Response Rate | Rate of having at least a one log reduction in disease burden or an MRD negative (10⁻³) test result | MRD assessments will be performed at Screening, Day 1 of Cycles 2, 3, 4, then every 2 cycles thereafter (ie, Cycles 6, 8, 10, etc) until remission or EOT |
| | MRD Conversion Rate | Rate of achieving MRD negativity (10⁻³) at any time on therapy | MRD assessments will be performed at Screening, Day 1 of Cycles 2, 3, 4, then every 2 cycles thereafter (ie, Cycles 6, 8, 10, etc) until remission or EOT |
| | Duration of MRD Response | Evaluate the durability of MRD response by serial bone marrow aspirate assessment for MRD | Time from achieving first MRD response to last MRD negative assessment or having a positive MRD result or increase in disease burden by at least one log. Death will be censored. |

| MDS Cohort - Phase 1b | | | |
|---|---|---|---|
| Primary | MTD/MAD, and/or RP2D | Dose-limiting toxicities (DLTs) evaluated using NCI CTCAE Version 5.0 | From first dose to the end of Cycle 1 |
| | Safety / tolerability | Type, frequency, seriousness and severity of AEs (using NCI CTCAE Version 5.0), and relationship of AEs to study treatment, clinical laboratory evaluations | From ICF signature to 28 days after last dose of IP |
| Secondary | Preliminary efficacy | CR, PR, marrow (mCR): defined as the rate of achieving CR, PR, mCR | From first dose to 28 days after last dose |
| | | Response assessed by Investigator according to IWG for MDS Response Criteria (Cheson B.D. et al., Blood 2006; 108(2): 419-425) | |

| MDS Cohort - Phase 2 | | | |
|---|---|---|---|
| Primary | Efficacy at RP2D | CR, PR, mCR: defined as the rate of achieving CR, PR, mCR | From first dose to 28 days after last dose |
| Secondary | Safety / Tolerability | Type, frequency, seriousness and severity of AEs (using NCI CTCAE Version 5.0), and relationship of AEs to study treatment, clinical laboratory evaluations | From ICF signature to 28 days after last dose of IP |

| MDS Cohort - Phase 1b and 2 | | | |
|---|---|---|---|
| Secondary | Efficacy | Time to response (CR, mCR, PR) according to IWG Response Criteria for MDS (Cheson B.D. et al., Blood 2006; 108(2): 419-425) | From first dose to first response CR, mCR, PR |
| | | DOR (CR, mCR, PR) | From first response (CR, mCR, PR) to first relapse, disease progression, or death, whichever occurs first |
| | | Event-Free Survival (EFS) | From first dose to first relapse, disease progression, or death from any cause, whichever occurs first |
| | | Relapse-Free Survival (RFS) | From first response to relapse, death, or last follow-up |

| AML and MDS Cohorts - Phase 1b and 2 | | | |
|---|---|---|---|
| Secondary | Efficacy Hematologic improvement rate (HI) | Overall Survival (OS) Rate of HI-N + HI-P + HI-E according to IWG MDS HI criteria (Cheson B.D. et al., Blood 2006; 108(2): 419-425) | From first dose to death |
| | One-year survival rate | The probability of survival at 1 year from first dose | From first dose to approximately 12 months |
| | PK | PK parameters of oral 5-azacytidine and each combination agent, including observed maximum concentration (Cmax), time at which maximum concentration is observed (tmax), half-life (t1/2), area under the curve (AUC), and other parameters as appropriate | All planned timepoints in Cycles 1, 2, and 3 |
| Exploratory | Pharmacody namics (Pd) | To evaluate the Pd of each combination agent to associate drug exposure with extent of target modulation and/or efficacy/tolerability (in each treatment cohort during dose/schedule escalation to RP2D) | Time from achieving first MRD response to last MRD negative assessment, having a positive MRD result or increase in disease burden by at least one log. Death will be censored. |
| | PK/Pd | Assess the relationship between PK/Pd biomarkers and clinical outcomes of oral 5-azacytidine in combination with venetoclax | All planned timepoints in Cycles 1, 2, and 3 |
| | Correlative Sciences | Evaluation of molecular, cellular and metabolic markers in peripheral blood (PB) and/or bone marrow (BM) which may be predictive of antitumor activity and/or resistance. | Approximately 8 months |
| | | Utilizing both pre- and post-treatment bone marrow biopsy samples acquired for purposes of disease assessment, change in baseline of candidate biomarkers will also be assessed using molecular/cell based assays ie, multi-parametric flow cytometry, DNA (gene sequencing), RNA expression (RNAseq), and/or in ex vivo assays. | |
| | | For example (but not limited to), MRD: MFC or NGS/PCR based assays; Gene mutation characterization: using next generation sequencing at baseline, during treatment, to relapse. | |
| | | Oral 5-azacytidine: DNA methylation changes in whole blood/PBMC/PBLs or BM | |
| | | Venetoclax: BCL-2 expression /amplification levels and characterization of other Bcl-2 family members, and/or Bcl-2 mimetic assays | |
| | | Gilteritinib: pFLT3/FLT3, allelic ratio for FLT3, pSTAT5/ STAT5 | |
| Abbreviations: AE = adverse event; AUC = area under the blood concentration time-curve; BM = bone marrow; Cmax = maximum blood concentration; CR = complete response; CRi = complete response with incomplete recovery of PB counts; CRR = complete response rate; CRp = complete response with incomplete platelet recovery; DLT = dose-limiting toxicity; DOR = duration of response; EFS = event free survival; IP = investigational product; MAD = maximum accepted dose; MLFS = morphologic leukemia-free state; MTD = maximum-tolerated dose; ORR = overall response rate; OS = overall survival; PB = peripheral blood; PCR = polymerase chain reaction; Pd = pharmacodynamics; PD = progressive disease; PK = pharmacokinetics; PR = partial response; t½ = elimination half-life; tmax = time to peak (maximum) blood concentration | | | |

**Study Design** This is an open-label, Phase 1b/2, multi-center, dose-determination, dose expansion, umbrella study that will evaluate the safety, tolerability, and preliminary efficacy of oral 5-azacytidine as a backbone in combination with biomarker directed and novel therapies in 2 separate disease cohorts: AML and HR-MDS.

Newly diagnosed patients with AML who are ineligible to receive intensive induction chemotherapy due to age ≥ 75 years old, or ≥ 18 years old with comorbidities; and AML patients ≥ 18 years old, who relapsed or refractory to 1 or 2 standard induction treatments will be enrolled in this study. Newly diagnosed patients with HR-MDS who are ≥ 18 years old will be enrolled in this study.

The study will include 2 parts: Phase 1b dose-finding and Phase 2 dose expansion.

Phase 1b, dose-finding part will evaluate safety, tolerability, preliminary efficacy, establish the MTD/MAD and determine the RP2D of oral 5-azacytidine in combination with venetoclax or gilteritinib. Dose-finding part will use dose determination rules based on an mTPI-2 method. Patients will enroll to one of the following combination arms:
Cohort 1 AML
   - Arm A: oral 5-azacytidine + Venetoclax for newly diagnosed AML with all mutations
   - Arm B: oral 5-azacytidine + Gilteritinib for newly diagnosed or R/R AML with a FLT3 ITD or TKD
Cohort 2 HR-MDS
   - Arm C: oral 5-azacytidine + Venetoclax for newly diagnosed, high and very high-risk MDS patients per IPSS-R

Phase 2, dose expansion part will further evaluate the safety, PK/Pd and efficacy of oral 5-azacytidine in combination with venetoclax at the RP2D. Patients will enroll to one of the following:
Cohort 1 AML: oral 5-azacytidine + Venetoclax for newly diagnosed AML with all mutations
Cohort 2 HR-MDS: oral 5-azacytidine + Venetoclax for newly diagnosed HR-MDS per Revised International Prognostic Scoring System (IPSS-R)

The study consists of a Screening Period, a Treatment Period (dose-finding and dose expansion), and a Follow-up Period. The study design is illustrated in FIG 18.

In one embodiment of this study, additional treatment arms are included, such those described in Example 2. For instance, an additional treatment arm may include oral 5-azacytidine + Ivosidenib in AML patients with IDH1 mutation (Combination Arm A) and/or oral 5-azacytidine + Enasidenib in AML patients with IDH2 mutation (Combination Arm B).

Dose-Finding Phase The objective of the dose-finding part of the study is to identify the MTD/MAD (if reached) and/or RP2D of oral 5-azacytidine in combination with venetoclax or gilteritinib in 28-day cycles. The dose-finding part is planned to evaluate 5 dose levels of oral 5-azacytidine in combination with venetoclax or gilteritinib (Table 7) and will enroll 3 to 9 patients in each dose level. Approximately 9 to 18 patients will enroll into each combination arm.

**Table 7: Dose Levels by Combination Arms and Disease Cohort**

| | **AML Cohort** | | **HR-MDS Cohort** |
|---|---|---|---|
| | **Arm A: Oral 5-Azacytidine + Venetoclax ^{a}** | **Arm B: Oral 5-Azacytidine + Gilteritinib** | **Arm C: Oral 5-Azacytidine + Venetoclax ^{a, c}** |
| Dose Level 2 | Oral 5-Azacytidine 300mg QD x 21 d + | Oral 5-Azacytidine 300mg QD x 21 d + | Oral 5-Azacytidine 300mg QD x 21 d + |
| | Venetoclax 400mg QD x 28 d | Gilteritinib 120mg QD x 28 d | Venetoclax 400mg QD x 14 d |
| Starting Dose (Dose Level 1) | Oral 5-Azacytidine 300mg QD x 14 d + | Oral 5-Azacytidine 300mg QD x 14 d + | Oral 5-Azacytidine 300mg QD x 14 d + |
| | Venetoclax 400mg QD x 28 d | Gilteritinib 120mg QD x 28 d | Venetoclax 400mg QD x 14 d |
| Dose Level -1A | Oral 5-Azacytidine 200mg QD x 14 d + | Oral 5-Azacytidine 200mg QD x 14 d + | Oral 5-Azacytidine 200mg QD x 14 d + |
| | Venetoclax 400mg QD x 28 d | Gilteritinib 120mg QD x 28 d | Venetoclax 400mg QD x 14 d |
| Dose Level -1B | Oral 5-Azacytidine 300mg QD x 14 d + | Oral 5-Azacytidine 300mg QD x 14 d + | Oral 5-Azacytidine 300mg QD x 14 d + |
| | Venetoclax 400mg QD x 21 d ^{b} | Gilteritinib 80mg QD x 28 d | Venetoclax 200mg QD x 14 d |
| Dose Level -2 | Oral 5-Azacytidine 200mg QD x 14 d + | Oral 5-Azacytidine 200mg QD x 14 d + | Oral 5-Azacytidine 200mg QD x 14 d + |
| | Venetoclax 400mg QD x 21 d ^{b} | Gilteritinib 80mg QD x 28 d | Venetoclax 200mg QD x 14 d |
| a For Cycle 1 only, venetoclax will be administered using a ramp-up dosing schedule: 100mg on Day 1, 200mg on Day 2, 400mg on Day 3 and beyond | | | |
| b For Arm A dose levels -1B and -2, venetoclax will decrease by treatment duration in days to 400mg x 21 days instead of 28 days. | | | |
| c For Arm E, venetoclax dosing will be 400mg QD x 14 days for dose levels 1, 2, and -1A. The venetoclax de-escalated dose will be 200mg QD x 14 days for dose levels -1B and -2. | | | |

In each dose level, dose limiting toxicities (DLTs) will be assessed for the respective DLT period, defined as the first 28 days following initiation of treatment with oral 5-azacytidine (Cycle 1). A pre-calculated decision table of all the optimal decisions will be used to guide the selection of doses and/or schedules. For a dose level to be for dose expansion, at least 6 patients should be evaluated for DLT during the dose escalation to declare a tolerable dose level.

For a patient to be considered DLT evaluable, the patient must have either a Cycle 1 DLT after receiving at least one dose of oral 5-azacytidine or completed Cycle 1 without a DLT. If a patient is taken off study for reasons other than toxicities (ie, personal reason or disease progression) prior to completing 80% of the planned doses of oral 5-azacytidine (11/14 or 16/21 days of 28-day cycle) and the combination agent (11/14, 16/21, or 22/28 days for venetoclax; or 22/28 days for gilteritinib) of the first cycle of therapy, this patient will not be considered as having completed the treatment cycle and will be replaced.

After the evaluation of the 5 planned dose levels of oral 5-azacytidine with venetoclax or gilteritinib is complete, the MTD and/or RP2D will be determined, and the dose expansion part will open.

During the dose-finding part, the Investigators will recommend dose escalation/de-escalation decisions and the combination RP2D of oral 5-azacytidine for use in the dose expansion based on an integrated assessment of the safety, PK and PD data, and preliminary efficacy information. Dose determination decision rules will be guided by an mTPI-2 design (Guo et al., Contemp Clin Trials, 2017 Jul: 58:23-33), and a target DLT rate of 30% will be used to make recommendations to the SRC for the dose level of the next enrolled patient.

**Dose Expansion Phase** Once the MTD and/or RP2D of oral 5-azacytidine and venetoclax combination therapy has been determined, approximately 61 newly diagnosed AML and 54 newly diagnosed MDS patients will be enrolled in two separate disease cohorts. The dose expansion part will further evaluate safety and efficacy of the administered combination RP2D of oral 5-azacytidine with venetoclax.

After dose determination is complete for each combination arm, all ongoing patients will continue to receive the same dose and treatment until discontinuation.

Dose reductions may occur based on the observed safety per the dose modification guidelines
**Study Duration** The expected duration of the entire study is approximately 4 years, which includes an overall enrollment period of approximately 24 months (12 months in Phase 1b, 12 months in Phase 2), and approximately 2 years duration per patient: a 28-day Screening Period, a Treatment Period of approximately 12 months, and a Follow-up Period of 1 year post last dose. The actual duration of the study will be dependent upon the median treatment duration and follow-up for patients.

**End of Trial** The End of Trial is defined as either the date of the last visit of the last patient to complete the post-treatment follow-up, or the date of receipt of the last data point from the last patient that is required for primary, secondary and/or exploratory analysis, as prespecified in the protocol, whichever is the later date.

**Study Population / Estimated No. Patients** Approximately 169 patients are planned for enrollment. The Phase 1b dose-finding part will enroll up to approximately 54 patients (36 AML, 18 MDS). Each combination arm to enroll approximately 18 patients to evaluate safety, tolerability and preliminary efficacy. The Phase 2 dose expansion part will enroll approximately 115 patients (61 AML, 54 MDS).

**Key Inclusion Criteria** Patients must satisfy the following criteria to be enrolled in the study:
1. Patient is ≥ 18 years of age at the time of signing the informed consent form (ICF).
2. AML Cohort: confirmation of the following for AML as defined by the updated 2016 World Health Organization (WHO) Classification.
   a. First relapse (ie, recurring after having achieved an initial response [CR/CRi/CRp] to intensive induction chemotherapy except acute promyelocytic leukemia [APML]); or
   b. Persisting/refractory after 1 to 2 intensive induction courses (ie, no response after 1 to 2 prior chemotherapy regimens); or
   c. Newly diagnosed AML who are not candidates for intensive induction chemotherapy due to age ≥ 75 years old, or ≥ 18 years old with any of the following comorbidities:
      - Eastern Cooperative Oncology Group (ECOG) performance status of 2
      - Cardiac history of Congestive Heart Failure (CHF) requiring treatment or Ejection Fraction ≤ 50% or chronic stable angina determined by multi-gated acquisition (MUGA) or echocardiogram (ECHO)
      - Creatinine clearance ≥ 30 mL/min to < 45 mL/min calculated by Cockcroft-Gault formula
      - Moderate hepatic impairment with total bilirubin > 1.5 to ≤ 3.0 x upper limit of normal (ULN)
      - Any other comorbidity that the investigator judges to be incompatible with intensive chemotherapy must be reviewed by the Sponsor during screening and before study enrollment.
   d. Intermediate or poor risk status cytogenetics for newly diagnosed AML (Appendix E)
   e. Confirmed AML specific mutation in bone marrow aspirate and/or peripheral blood sample documented since last progression/relapse (or recent testing for newly diagnosed) as detected by an approved Institutional lab test:
      - Arm A: all mutations
      - Arm B: FLT3 (ITD or TKD)
3. MDS Cohort:
   f. Confirmation of diagnosis of previously untreated primary or secondary MDS as defined by the WHO Classification. Results of pathology review are required prior to receiving the first dose of IP.
   g. Confirmation of the MDS risk classification per IPSS-R High or Very High risk. Results of pathology review are required prior to receiving the first dose of IP.
4. Eastern Cooperative Oncology Group (ECOG) performance status of 0, 1 or 2
5. Patients must have the following baseline laboratory values:
   h. White blood cell (WBC) count of ≤ 25 x 10⁹/L. Hydroxyurea or leukapheresis are permitted to meet this criterion. Note: hydroxyurea is allowed up to 24 hours prior to the starting treatment and limited use only during Cycle 1 for cytoreduction (to control high white blood cell count).
   i. Potassium and magnesium within normal limits or correctable with supplements.
   j. Uric acid ≤ 7.5 mg/dL (446 µmol/L). Prior and/or concurrent treatment with hypouricemic agents (eg, allopurinol, rasburicase) are allowed. Rasburicase is contraindicated in patients with baseline glucose-6-phosphate dehydrogenase (G6PD) deficiency.
   k. International normalized ratio (INR) < 1.5 x ULN and activated partial thromboplastin time (aPTT) < 1.5 x ULN.
6. Adequate organ function defined as:
   l. Renal function: Creatinine clearance ≥ 30 mL/minute, calculated by the Cockcroft-Gault formula or measured by 24 hours urine collection
   m. Hepatic function: aspartate aminotransferase (AST), ALT ≤ 3.0 x ULN, bilirubin ≤ 1.5 x ULN, unless due to Gilbert's syndrome or leukemic organ involvement. Patients who are < 75 years of age may have a bilirubin of ≤ 3.0 x ULN.
   n. Left ventricular ejection fraction (LVEF) > 40% by multigated acquisition (MUGA) or (echocardiogram) ECHO (see exclusion criteria #22 for any additional LVEF requirement in each combination arm)
7. Agree to serial bone marrow aspirate/biopsies
8. Females of childbearing potential (FCBP)* must:
   o. Have two negative pregnancy tests as verified by the Investigator prior to starting study therapy. She must agree to ongoing pregnancy testing during the course of the study, and after end of study therapy. This applies even if the patient practices true abstinence from heterosexual contact.
   p. Either commit to true abstinence from heterosexual contact (which must be reviewed on a monthly basis and source documented) or agree to use, and be able to comply with effective contraception without interruption during the study therapy (including dose interruptions), and for 90 days after discontinuation of study therapy, or longer if required for each compound and/or by local regulations.
   q. For Combinations Arm A and Arm C: the timeframe is extended to 6 months following the last study treatment dose.
9. Male patients must practice true abstinence* (which must be reviewed on a monthly basis) or agree to use a condom during sexual contact with a pregnant female or a female of childbearing potential while participating in the study, during dose interruptions and for at least 90 days following investigational product discontinuation, or longer if required for each compound and/or by local regulations, even if he has undergone a successful vasectomy.
   r. For Combinations Arm A and Arm C: the timeframe is extended to 6 months following the last study treatment dose.
10. Patients must understand and voluntarily sign an ICF prior to any study-related assessments/procedures being conducted.
11. Patients is willing and able to adhere to the study visit schedule and other protocol requirements.

**Exclusion Criteria** The presence of any of the following will exclude a patient from enrollment:
1. Patient is suspected or proven to have acute promyelocytic leukemia (APML) based on morphology, immunophenotype, molecular assay, or karyotype.
2. Patient has AML secondary to chronic myelogenous leukemia or patient is BCR-ABL1 t(9;22)(q34;q11) positive.
3. Patient has received systemic anticancer therapy or radiotherapy < 28 days prior to the start of study treatment. Note that hydroxyurea is allowed prior to the start of study treatment for the control of leukocytosis.
4. Patient has received therapy with hypomethylating agent (HMA) for AML or greater than 2 cycles of HMA for MDS
5. Patient has received investigational agents < 28 days or 5 half-lives, whichever is longer, prior to the start of study treatment
6. Patient has undergone hematopoietic stem cell transplant (HSCT) within 90 days prior to the start of study treatment, or on immunosuppressive therapy post HSCT at the time of screening, or with graft-versus-host disease (GVHD). The use of a stable dose of oral steroid post-HSCT, or topical steroids for ongoing skin GVHD is permitted.
7. Patient has persistent, clinically significant non-hematologic toxicities from prior therapies which have not recovered to < Grade 2
8. Patient has or is suspected of having central nervous system (CNS) leukemia. Evaluation of cerebrospinal fluid is only required if CNS involvement by leukemia is suspected during screening.
9. Patient has an active, uncontrolled systemic fungal, bacterial, or viral infection (defined as ongoing signs/symptoms related to the infection without improvement despite appropriate antibiotics, antiviral therapy, and/or other treatment). The patient should be afebrile for at least 72 hours.
10. Patient has immediate life-threatening, severe complications of leukemia such as uncontrolled bleeding, pneumonia with hypoxia or shock, and/or disseminated intravascular coagulation
11. Patient has prior history of malignancy, other than MDS, MPN or AML, unless the patient has been free of the disease for ≥ 1 year prior to the start of study treatment. However, patients with the following history/concurrent conditions are allowed:
   - Basal or squamous cell carcinoma of the skin
   - Carcinoma in situ of the cervix
   - Carcinoma in situ of the breast
   - Incidental histologic finding of prostate cancer (T1a or T1b using the tumor, nodes, metastasis clinical staging system)
12. Patient is known seropositive or active infection with human immunodeficiency virus (HIV), or active infection with hepatitis B virus (HBV) or hepatitis C virus (HCV)
13. Patient is known to have dysphagia, short-gut syndrome, gastroparesis, or other conditions that limit the ingestion or gastrointestinal absorption of drugs administered orally
14. Patients has uncontrolled hypertension (systolic blood pressure [BP] > 180 mmHg or diastolic BP > 100 mmHg) or has not been stable for at least 1 month prior to treatment
15. Significant active cardiac disease within the previous 6 months prior to signing the ICF, including:
   - New York Heart Association (NYHA) Class III or IV congestive heart failure (see Appendix J)
   - Unstable angina or angina requiring surgical or medical intervention;
   - Significant cardiac arrhythmia; and/or
   - Myocardial infarction
16. Patient is a pregnant or lactating female
17. Patient has known or suspected to have hypersensitivity to any of the components of the assigned study treatments
18. Patient has any significant medical condition, laboratory abnormality, or psychiatric illness that would prevent the patient from participating in the study
19. Patient has any condition including the presence of laboratory abnormalities, which places the patient at unacceptable risk if he/she were to participate in the study
20. Any investigational therapy within 28 days prior to initiation study treatment
21. Patient has any condition that confounds the ability to interpret data from the study
22. Additional exclusion criteria for patients based on planned Combination Arm are:
   - Combination Arm A (AML all mutations) and Arm C (HR-MDS):
      - Received strong CYP3A inhibitors, moderate CYP3A inhibitors, strong CYP3A inducers, moderate CYP3A inducers, strong CYP2C8 inhibitors, CYP2C8 substrates, or OATP1B1/3 substrates within 7 days prior to initiation of study treatment.
      - Received strong CYP2C8 inducers within 14 days prior to initiation of study treatment
      - Received live attenuated vaccines prior to initiation of study treatment.
   - Combination Arm B (non-specific FLT3):
      - Patient has heart rate corrected QT interval (QTc) interval (ie, Fridericia's correction [QTcF]) ≥ 450 ms or other factors that increase the risk of QT prolongation or arrhythmic events (eg, heart failure, hypokalemia, family history of long QT interval syndrome) at screening
      - Patient is taking those medications (list of prohibited medications will be provided in the protocol) that are known to prolong QT interval unless the patient can be transferred to other medications at least 5 half-lives prior to the start of study treatment
      - Patient has a history or presence of sustained ventricular tachycardia; any history of ventricular fibrillation or torsades de pointes; bradycardia defined as heart rate (HR) < 50 bpm; right bundle branch block + left anterior hemiblock (bifascicular block); an ejection fraction ≤ 45% assessed by MUGA or ECHO within 14 days of cycle 1 day 1
      - Patient is taking medications which are P-glycoprotein (P-gp) substrates and strong CYP3A4 inhibitors, and inducers within the previous 7 days.
      - Patients with hypokalemia and hypomagnesemia at Screening (values below lower limit of normal [LLN]).
      - Patients with Long QT Syndrome at Screening.
      - Patient requires treatment with concomitant drugs that are strong inhibitors or inducers of P-gp with the exception of drugs that are considered absolutely essential for the care of the patient.
      - Patient requires treatment with concomitant drugs that target serotonin 5-hydroxytryptamine receptor 1 (5HT1R) or 5-hydroxytryptamine receptor 2B (5HT2BR) or sigma nonspecific receptor with the exception of drugs that are considered absolutely essential for the care of the patient.
23. Patient has any significant medical condition, laboratory abnormality, or psychiatric illness that would prevent the patient from participating in the study.
24. Patient has any condition including the presence of laboratory abnormalities, which places the patient at unacceptable risk if he/she were to participate in the study.
25. Patient has any condition that confounds the ability to interpret data from the study.

**Treatment Period** The patient may be enrolled once all inclusion/exclusion criteria are verified and the patient is deemed to be eligible. The patient must start treatment within 3 days of enrolment. For all subsequent visits, an administrative window of ± 3 days is permitted.

Prior to first venetoclax dose (ie, before ramp-up), patients will receive prophylactic measures including adequate hydration and anti-hyperuricemic agents which will be continued during the ramp-up phase (ie, before ramp-up and continue until after final dose is reached or longer as clinically indicated). Treatment cycles are 28 days in duration.

### Investigational Products

The study will use oral 5-azacytidine as 200 mg and 300 mg tablets for oral administration. Each tablet is formulated using excipients that are generally regarded as safe and are used in marketed drug products.

The marketed form of venetoclax (VENCLEXTA^{®}) will be used in 10 mg, 50 mg, and 100 mg tablets for oral administration.

The marketed form of gilteritinib (XOSPATA^{®}) will be used in 40 mg tablets for oral administration.

**Treatment Administration and Schedule** For dose-finding (Phase 1b), following screening, eligible patients will be assigned to receive 5-azacytidine 300 mg orally (PO) once daily on Days 1-14 (or 21) with venetoclax or gilteritinib in a 28-day treatment cycle as described in this example.

In order to allow for the best opportunity to benefit from the treatment, and given the mechanism of action of oral 5-azacytidine and the median time to response for the venetoclax or gilteritinib combination, patients should be treated for at least 3 cycles with venetoclax or at least 6 cycles with gilteritinib, although patients will be discontinued from the treatment sooner if they demonstrate documented relapse from CR or PR, disease progression, unacceptable AEs, intercurrent illness that prevents further administration of treatment, Investigator's decision to withdraw the patient, withdrawal of consent, noncompliance with trial treatment or procedure requirements, death, or administrative reasons.

Oral 5-azacytidine will be given PO once daily for 14 (or 21) days in a 28-day cycle on Days 1-14 (or 21). Oral 5-azacytidine will be administered about the same time each day with approximately 240 mL (8 ounces) of room temperature water, with or without food.

In Phase 1b dose-finding part of the study, starting dose level of oral 5-azacytidine will be 300 mg x 14 days PO QD per 28-day cycle on Days 1-14 (or 21). Depending on the number of DLTs, the dose may decrease dose to 200 mg x 14 days; or if tolerated, dose may stay the same or increase treatment duration in days to 300 mg x 21 days..

When oral 5-azacytidine, venetoclax or gilteritinib will be administered on the same day, oral 5-azacytidine will be administered first followed by venetoclax or gilteritinib.

Venetoclax will be administered according to the approved label (Venclexta^{®}, 2019) orally QD on Days 1 to 28 per 28-day cycle for AML patients. Venetoclax dosing for HR-MDS is on Days 1 to 14 per 28-day cycle A brief dose ramp-up occurs for Cycle 1 with a starting dosing of 100 mg on Day 1, 200 mg on Day 2, and 400 mg on Day 3 and beyond. Venetoclax will be administered at 400 mg on subsequent days.

In Phase 1b dose-finding part of the study, venetoclax starting dose level will be 400 mg PO QD x 28 days for AML patients (Arm A) and 400 mg PO QD x 14 days for MDS patients (Arm C) in a 28-day cycle. Depending on the number of DLTs experienced and if related to venetoclax, the venetoclax dose may de-escalate by decreasing treatment duration to 400 mg PO QD x 21 days (Arm A), or by decreasing dosage to 200 mg PO QD x 14 days (Arm C) in a 28-day cycle. In Phase 2 dose expansion, the RP2D will be given to patients. In Phase 2 dose expansion part, the RP2D of venetoclax will continue the same schedule and timing in a 28-day cycle (ie, Cycle 3 is 28 days after Cycle 2, etc).

Patients will be hospitalized during the venetoclax dose ramp-up in Cycle 1 for a minimum of 8 days from Day 1 to Day 8. This hospitalization is required per protocol and does not constitute a serious adverse event. Patients should be instructed to take their daily dose at approximately the same time each day + 8 hours.

Gilteritinib (XOSPATA^{®}) will be administered at the recommended starting dose of 120 mg administered orally once a day (QD) on Days 1-28 of each 28-day cycle. Patients should be instructed to take their daily dose at approximately the same time each day.

**Safety Follow-up** All patients will be followed for 28 days after the last dose of investigational product (IP) or EOT Visit, whichever occurs later, for AE reporting and concomitant medication information as well as new disease therapies. The 28-day (± 3 days) safety follow-up contact may be by telephone for non-FCBP. For FCBP, the 28-day safety follow-up will be completed in the clinic for pregnancy testing requirements. In addition, any SAEs made known to the Investigator at any time thereafter that are suspected of being related to IP.

**Survival Follow-up** After the end of treatment visit, all patients will be followed every 4 weeks for survival follow-up for up to 1 year post last dose or until death, lost to follow-up, or the End of Trial whichever occurs first. New disease therapies for AML or MDS should be collected at the same time schedule.

**Efficacy Assessment** Serial blood and bone marrow BM sampling will be used to determine response to study drug therapy starting at Cycle 2 Day 1.

At baseline, a bone marrow aspirate (BMA) sample is required. A biopsy must be collected if the aspirate is not available and may be collected in addition to the aspirate per institutional practice. Cytogenetic and molecular profiling from the BMA are also required at Screening (unless they are available to enter from the patient's medical records from the past 90 days). Complete blood counts, peripheral blood smear (PBS), and BMAs will be used to determine response to therapy at specific timepoints. Samples may be obtained up to 4 days prior to the end of the cycle, ie, Days 25 to 28.

Response to treatment will be assessed by the Investigator using local laboratory results according to the following guidelines per disease criteria based on reported hematology laboratory parameters, peripheral blood smear (PBS), bone marrow aspirates (BMAs), and/or biopsies:
- AML treatment will be assessed according to modified European Leukemia Net (ELN) AML Response Criteria, ie, modified to include Complete remission with partial hematologic recovery (CRh) and hematologic response (Döhner, H et al., Blood, 2017 Jan 26, 129(4): 424-47); and IWG for AML Response Criteria (Cheson B.D. et al., J Clin Oncol. 2003, 21(24): 4642-92003). Hematologic improvement (HI) will also be assessed according to the IWG MDS HI criteria (Cheson B.D. et al., Blood 2006; 108(2): 419-425).
- MDS treatment will be assessed according to IWG for MDS Response Criteria (Cheson B.D. et al., Blood 2006; 108(2): 419-425).

Hematologic response will be evaluated as patients with antecedent hematologic disorders may be enrolled onto study. Transfusion dependence is defined as having received ≥ 2 units of RBCs and/or platelets within 8 weeks prior to study treatment. Transfusion independence is defined as a period of 8 weeks with no transfusions.

For AML, progressive disease will be defined as one of the following changes from baseline:
- For patients with 5 to 70% bone marrow (BM) blasts at baseline: a > 50% increase of BM blast count percentage from baseline to ≥ 20%; or
- For patients with > 70% BM blasts at baseline: a doubling of absolute blast count in peripheral blood from baseline to ≥ 10 x 10⁹/L (10,000/µL); or
- New extramedullary disease since last response assessment
- Progression/relapse after a hematological improvement with at least one of the following:
   - At least 50% decrement from maximum response levels in granulocytes or platelets
   - Reduction in Hgb by ≥ 1.5 g/dL
   - Transfusion dependence

For HR MDS, progressive disease will be defined as one of the following changes from baseline:
- Less than 5% blasts: ≥ 50% increase in blasts to > 5% blasts
- 5% - 10% blasts: ≥ 50% increase in blasts to > 10% blasts
- 10% - 20% blasts: ≥ 50% increase in blasts to > 20% blasts
- 20% - 30% blasts: ≥ 50% increase in blasts to > 30% blasts

Any of the following:
- At least 50% decrement from maximum remission/response levels in granulocytes or platelets
- Reduction in Hgb concentration by ≥ 2 g/dL
- Transfusion dependence

Progressive disease is to be confirmed by 2 consecutive response assessments separated by at least 1 month. The date of progressive disease is defined as the first date that one of the conditions above was met. In the absence of progressive disease (as defined above) or unacceptable toxicity, patients may continue treatment if they are deriving benefit, as judged by the Investigator.

The marrow aspiration and core sampling (biopsy) should be performed according to the standard of care and analyzed at the local site's laboratory in accordance with the International Council for Standardization in Hematology (ICSH) Guidelines (Lee, J. H. et al., J Clin Oncol. 2017, 35 (24): 2754-2763).

Patients are to undergo end-of treatment evaluations when study treatment is discontinued. The reason for treatment discontinuation will be recorded in the eCRF pages and in the source document. Patients who discontinue study treatment prior to relapse or progression will complete site visits. For patients who have discontinued study treatment due to relapse or progression, Survival Follow-up can be performed by site visits or phone calls. Patients will be followed for up to 1 year post last dose, or until they have died, are lost to follow up, or withdrawal of consent for further data collection within 1 year from the date of last dose.

For acute myeloid leukemia, response criteria will be summarized by best overall response categories:
- CR/CRh rate, and overall response rate ORR. The ORR includes all responses of complete remission (CR) (ie, CR, CRi CRp), morphologic leukemia-free state (MLFS), and partial remission (PR). CRh = Complete remission with partial hematologic recovery. CRi = CR with incomplete hematologic recovery. CRp = C-reactive protein.
- Minimal residual disease (MRD) response rate and MRD conversion rate will also be assessed as efficacy variables. Minimal residual disease will be assessed by MFC and/or next generation sequencing (NGS) at the same time as efficacy assessments and will be assessed centrally. The site will ensure peripheral blood and BMA/bone marrow biopsy (BMB) samples are collected and stored for exploratory testing at the time of each BM collection.
- Other measures of clinical activity including overall survival (OS), event-free survival (EFS), RFS, time to response (CR, CRi, CRp, PR, MLFS), duration of responses (CR, CRh and ORR), hematologic improvement rate, and a 1-year survival rate will be summarized.

For myeloid dysplastic syndrome, response criteria will be summarized by best overall response categories:
- CR, PR, mCR
- Other measures of clinical activity including OS, EFS, RFS, time to response and duration of responses for CR, PR, and mCR, hematologic improvement rate, and a 1-year survival rate will be summarized.

The results of the clinicaly study will show that oral 5-azacytidine in combination with venetoclax or gilteritinib at the maximum tolerated dose (MTD), maximum administered dose (MAD) and/or the recommended Phase 2 dose (RP2D) is safe and tolerated in patients with AML or higher-risk MDS, which include: (1) patients with newly diagnosed AML with all mutations treated with oral 5-azacytidine in combination with venetoclax; (2) patients with newly diagnosed or relapsed and/or refractory AML with a FLT3 ITD or TKD mutation treated with oral 5-azacytidine in combination with gilteritinib; and (3) patients with newly diagnosed high and very high risk MDS treated with oral 5-azacytidine in combination with venetoclax.

Further, the results of the clinical study will also show that oral 5-azacytidine when given in combination with venetoclax at the RP2D is efficacious in newly diagnosed patients with AML or higher-risk MDS, which include: (1) patients with newly diagnosed AML with all mutations treated with oral 5-azacytidine in combination with venetoclax; and (2) patients with newly diagnosed newly diagnosed HR-MDS per Revised International Prognostic Scoring System (IPSS-R) treated with oral 5-azacytidine in combination with venetoclax.

The present disclosure has been described in connection with certain embodiments and examples; however, the claimed invention is defined by the claims.

While certain embodiments have been illustrated and described, it should be understood that changes and modifications can be made therein in accordance with the knowledge of one of ordinary skill in the art without departing from the technology in its broader aspects as defined in the following claims.

The embodiments illustratively described herein may suitably be practiced in the absence of any element or elements, limitation or limitations, not specifically disclosed herein. Thus, for example, the terms "comprising," "including," "containing," etc., shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the claimed technology. Additionally, the phrase "consisting essentially of" will be understood to include those elements specifically recited and those additional elements that do not materially affect the basic and novel characteristics of the claimed technology. The phrase "consisting of" excludes any element not specified.

In addition, where features or aspects of the disclosure are described in terms of Markush groups, those skilled in the art will recognize that the disclosure is also thereby described in terms of any individual member or subgroup of members of the Markush group.

## Claims

1. A (i) pharmaceutical composition comprising 5-azacytidine, and (ii) at least one additional therapeutic agent, for use in a method of treating acute myeloid leukemia and/or myelodysplastic syndrome in a human, wherein the method comprises administering to the human (i) the pharmaceutical composition comprising 5-azacytidine orally, and (ii) the at least one additional therapeutic agent, wherein the at least one additional therapeutic agent is venetoclax.

2. The pharmaceutical composition and at least one additional therapeutic agent for use as claimed in claim 1, wherein:
(a) the 5-azacytidine and the at least one additional therapeutic agent are administered concomitantly ; or
(b) the 5-azacytidine and the at least one additional therapeutic agent are administered sequentially wherein the 5-azacytidine is administered first.

3. The pharmaceutical composition and at least one additional therapeutic agent for use as claimed in claim 1, wherein:
(a) the 5-azacytidine and the at least one additional therapeutic agent are co-formulated as a single unit dosage form; or
(b) the 5-azacytidine and the at least one additional therapeutic agent are formulated as separate dosage forms.

4. The pharmaceutical composition and at least one additional therapeutic agent for use as claimed in any one of claims 1-3, wherein:
(a) the at least one additional therapeutic agent is administered parenterally; or
(b) the at least one additional therapeutic agent is administered orally.

5. The pharmaceutical composition and at least one additional therapeutic agent for use as claimed in any one of claims 1-4, wherein the 5-azacytidine is administered:
(a) at a dose of about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg , about 590 mg, or about 600 mg orally; and/or
(b) at a dose of about 200 mg; and/or
(c) at a dose of about 300 mg; and/or
(d) for the first seven, fourteen, or twenty-one days of a 28-day cycle; and/or
(e) to the human one or two times per day; and/or;
(f) in the form of a capsule or a tablet.

6. The pharmaceutical composition and at least one additional therapeutic agent for use as claimed in claim 5, wherein the 5-azacytidine is administered in the form of:
(a) a non-enteric-coated tablet, or
(b) an immediate release oral composition.

7. The pharmaceutical composition and at least one additional therapeutic agent for use as claimed in any one of claims 1-6, wherein the 5-azacytidine is administered:
(a) at a dose of about 200 mg per day for 14 days in a 28-day cycle;
(b) at a dose of about 300 mg per day for 14 days in a 28-day cycle;
(c) at a dose of about 200 mg per day for 21 days in a 28-day cycle;
(d) at a dose of about 300 mg per day for 21 days in a 28-day cycle;
(e) at a dose of about 200 mg per day for 7 days in a 28-day cycle; or
(f) at a dose of about 300 mg per day for 7 days in a 28-day cycle.

8. The pharmaceutical composition and at least one additional therapeutic agent for use as claimed in any one of claims 1-6, wherein the 5-azacytidine is administered:
(a) daily for 1, 2, 3, 4, 5, 6, 7, 8, 9, 10, 11, 12, 13, 14, or greater than 14 days, optionally followed by a treatment dosing holiday of 1, 2, 3, 4, 5, 6, 7, 8, 9, 10, 11, 12, 13, 14, or greater than 14 days;
(b) daily for 14 or more days, optionally followed by a treatment dosing holiday of 7 or more days;
(c) for 21 or more days, optionally followed by a treatment dosing holiday of 7 or more days;
(d) for 14 days, optionally followed by a treatment dosing holiday of 14 days;
(e) for 21 or more days, followed by a treatment dosing holiday of 7 or more days; or
(f) for 14 days, followed by a treatment dosing holiday of 14 days.

9. The pharmaceutical composition and at least one additional therapeutic agent for use as claimed in claim 8, wherein at least one of steps (a), (b), (c), (d), (e), or (f) are repeated.

10. The pharmaceutical composition and at least one additional therapeutic agent for use as claimed in any one of claims 1-4, wherein the 5-azacytidine is administered:
(a) at a dose of about 300 mg daily for 14 days, followed by a treatment dosing holiday of 14 days;
(b) at a dose of about 200 mg daily for 14 days, followed by a treatment dosing holiday of 14 days;
(c) at a dose of about 300 mg daily for 21 days, followed by a treatment dosing holiday of 7 days;
(d) at a dose of about 200 mg daily, followed by a treatment dosing holiday of 7 days;
(e) at a dose of 300 mg daily for 14 days, followed by a treatment dosing holiday of 14 days;
(f) at a dose of 200 mg daily for 14 days, followed by a treatment dosing holiday of 14 days;
(g) at a dose of 300 mg daily for 21 days, followed by a treatment dosing holiday of 7 days; or
(h) at a dose of 200 mg daily, followed by a treatment dosing holiday of 7 days.

11. The pharmaceutical composition and at least one additional therapeutic agent for use as claimed in claim 10, wherein at least one of steps (a), (b), (c), (d), (e), (f), (g), or (h) are repeated.

12. The pharmaceutical composition and at least one additional therapeutic agent for use as claimed in any one of claims 1-6, wherein the 5-azacytidine is administered:
(a) using a treatment cycle comprising administration of 5-azacytidine per day for 7 days in a 28-day cycle;
(b) using a treatment cycle comprising administration of 5-azacytidine per day for 14 days in a 28-day cycle; or
(c) using a treatment cycle comprising administration of 5-azacytidine per day for 21 days in a 28-day cycle.

13. The pharmaceutical composition and at least one additional therapeutic agent for use as claimed in claim 1, wherein the 5-azacytidine and the at least one additional therapeutic agent provides a synergistic effect to treat the acute myeloid leukemia or myelodysplastic syndrome.

14. The pharmaceutical composition and at least one additional therapeutic agent for use as claimed in claim 1, wherein:
(a) the acute myeloid leukemia is characterized as caused by a FLT3-ITD mutation; and/or
(b) the acute myeloid leukemia is resistant to treatment with the at least one additional therapeutic agent alone; and/or
(c) the 5-azacytidine is administered before the at least one additional therapeutic agent; and/or
(d) the acute myeloid leukemia is responsive to treatment with a FMS-like tyrosine kinase-3 (FLT3 inhibitor); and/or
(e) the acute myeloid leukemia is characterized as having an overexpression of MCL-1; and/or
(f) the 5-azacytidine primes the cancer cells for apoptosis mediated by the at least one additional therapeutic agent by downregulating the expression of MCL-1; and/or
(g) the 5-azacytidine and at least one additional therapeutic agent augments MCL-1 degradation; and/or
(h) the 5-azacytidine alters cell metabolism; and/or
(i) the 5-azacytidine causes cell cycle arrest; and/or
(j) the 5-azacytidine suppresses oxidative phosphorylation; and/or
(k) the 5-azacytidine increases expression of activating transcription factor 3 (ATF3); and/or
(l) the 5-azacytidine decreases expression of stearoyl-CoA desaturase (SCD); and/or
(m) the 5-azacytidine and at least one additional therapeutic agent increases median survival as compared to 5-azacytidine administered intravenously or subcutaneously and at least one additional therapeutic agent; and/or
(n) the 5-azacytidine and at least one additional therapeutic agent increases median survival as compared to 5-azacytidine administered intravenously or subcutaneously and at least one additional therapeutic agent by about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%.

15. The pharmaceutical composition and at least one additional therapeutic agent for use as claimed in any one of claims 1-6, wherein:
(a) the 5-azacytidine is administered daily to the human for 1, 2, 3, 4, 5, 6, 7, 8, 9, 10, 11, 12, 13, or 14 days;
(b) the at least one additional therapeutic agent is administered to the human for one or more days; and
(c) optionally repeating steps (a) and (b).

16. The pharmaceutical composition and at least one additional therapeutic agent for use as claimed in any one of claims 1-6, wherein:
(a) the 5-azacytidine is administered daily to the human for 1, 2, 3, 4, 5, 6, 7, 8, 9, 10, 11, 12, 13, or 14 days of a 28-day cycle;
(b) the at least one therapeutic agent is administered concurrently daily to the human for 1, 2, 3, 4, 5, 6, 7, 8, 9, 10, 11, 12, 13, 14, 15, 16, 17, 18, 19, 20, 21, 22, 23, 24, 25, 26, 27, or 28 days of a 28-day cycle; and
(c) optionally repeating steps (a) and (b).

17. The pharmaceutical composition and at least one additional therapeutic agent for use as claimed in any one of claims 1-16, wherein said method is a method of treating acute myeloid leukemia in a human.

18. The pharmaceutical composition and at least one additional therapeutic agent for use as claimed in any one of claims 1-16, wherein said method is a method of treating myelodysplastic syndrome in a human, optionally wherein the myelodysplastic syndrome is high and very high risk myelodysplastic syndrome as defined by the Revised International Prognostic Scoring System (IPSS-R).

## Patentansprüche

1. (i) Pharmazeutische Zusammensetzung, umfassend 5-Azacytidin, und (ii) mindestens ein zusätzliches therapeutisches Mittel zur Verwendung in einem Verfahren zur Behandlung von akuter myeloischer Leukämie und/oder myelodysplastischem Syndrom bei einem Menschen, wobei das Verfahren Verabreichen (i) der pharmazeutischen Zusammensetzung, umfassend 5-Azacytidin, auf oralem Wege und (ii) des mindestens einen zusätzlichen therapeutischen Mittels, wobei das mindestens eine zusätzliche therapeutische Mittel Venetoclax ist, an den Menschen umfasst.

2. Pharmazeutische Zusammensetzung und mindestens ein zusätzliches therapeutisches Mittel zur Verwendung nach Anspruch 1, wobei:
(a) das 5-Azacytidin und das mindestens eine zusätzliche therapeutische Mittel gleichzeitig verabreicht werden; oder
(b) das 5-Azacytidin und das mindestens eine zusätzliche therapeutische Mittel nacheinander verabreicht werden, wobei das 5-Azacytidin zuerst verabreicht wird.

3. Pharmazeutische Zusammensetzung und mindestens ein zusätzliches therapeutisches Mittel zur Verwendung nach Anspruch 1, wobei:
(a) das 5-Azacytidin und das mindestens eine zusätzliche therapeutische Mittel als eine Einzeldosiseinheitsform miteinander formuliert sind; oder
(b) das 5-Azacytidin und das mindestens eine zusätzliche therapeutische Mittel als separate Dosisformen formuliert sind.

4. Pharmazeutische Zusammensetzung und mindestens ein zusätzliches therapeutisches Mittel zur Verwendung nach einem der Ansprüche 1-3, wobei:
(a) das mindestens eine zusätzliche therapeutische Mittel parenteral verabreicht wird; oder
(b) das mindestens eine zusätzliche therapeutische Mittel oral verabreicht wird.

5. Pharmazeutische Zusammensetzung und mindestens ein zusätzliches therapeutisches Mittel zur Verwendung nach einem der Ansprüche 1-4, wobei das 5-Azacytidin verabreicht wird:
(a) in einer Dosis von etwa 50 mg, etwa 60 mg, etwa 70 mg, etwa 80 mg, etwa 90 mg, etwa 100 mg, etwa 110 mg, etwa 120 mg, etwa 130 mg, etwa 140 mg, etwa 150 mg, etwa 160 mg, etwa 170 mg, etwa 180 mg, etwa 190 mg, etwa 200 mg, etwa 210 mg, etwa 220 mg, etwa 230 mg, etwa 240 mg, etwa 250 mg, etwa 260 mg, etwa 270 mg, etwa 280 mg, etwa 290 mg, etwa 300 mg, etwa 310 mg, etwa 320 mg, etwa 330 mg, etwa 340 mg, etwa 350 mg, etwa 360 mg, etwa 370 mg, etwa 380 mg, etwa 390 mg, etwa 400 mg, etwa 410 mg, etwa 420 mg, etwa 430 mg, etwa 440 mg, etwa 450 mg, etwa 460 mg, etwa 470 mg, etwa 480 mg, etwa 490 mg, etwa 500 mg, etwa 510 mg, etwa 520 mg, etwa 530 mg, etwa 540 mg, etwa 550 mg, etwa 560 mg, etwa 570 mg, etwa 580 mg, etwa 590 mg oder etwa 600 mg oral; und/oder
(b) in einer Dosis von etwa 200 mg; und/oder
(c) in einer Dosis von etwa 300 mg; und/oder
(d) für die ersten sieben, vierzehn oder einundzwanzigsten Tage eines 28-Tage-Zyklus; und/oder
(e) ein- oder zweimal pro Tag an den Menschen; und/oder
(f) in Form einer Kapsel oder einer Tablette.

6. Pharmazeutische Zusammensetzung und mindestens ein zusätzliches therapeutisches Mittel zur Verwendung nach Anspruch 5, wobei das 5-Azacytidin in der folgenden Form verabreicht wird:
(a) eine nicht magensaftresistent überzogene Tablette oder
(b) eine orale Zusammensetzung mit sofortiger Freisetzung.

7. Pharmazeutische Zusammensetzung und mindestens ein zusätzliches therapeutisches Mittel zur Verwendung nach einem der Ansprüche 1-6, wobei das 5-Azacytidin verabreicht wird:
(a) in einer Dosis von etwa 200 mg pro Tag für 14 Tage in einem 28-Tage-Zyklus;
(b) in einer Dosis von etwa 300 mg pro Tag für 14 Tage in einem 28-Tage-Zyklus;
(c) in einer Dosis von etwa 200 mg pro Tag für 21 Tage in einem 28-Tage-Zyklus;
(d) in einer Dosis von etwa 300 mg pro Tag für 21 Tage in einem 28-Tage-Zyklus;
(e) in einer Dosis von etwa 200 mg pro Tag für 7 Tage in einem 28-Tage-Zyklus; oder
(f) in einer Dosis von etwa 300 mg pro Tag für 7 Tage in einem 28-Tage-Zyklus.

8. Pharmazeutische Zusammensetzung und mindestens ein zusätzliches therapeutisches Mittel zur Verwendung nach einem der Ansprüche 1-6, wobei das 5-Azacytidin verabreicht wird:
(a) täglich für 1, 2, 3, 4, 5, 6, 7, 8, 9, 10, 11, 12, 13, 14 oder mehr als 14 Tage, gegebenenfalls gefolgt von einer Behandlungspause ohne Dosisgabe von 1, 2, 3, 4, 5, 6, 7, 8, 9, 10, 11, 12, 13, 14 oder mehr als 14 Tage;
(b) täglich für 14 oder mehr Tage, gegebenenfalls gefolgt von einer Behandlungspause ohne Dosisgabe von 7 oder mehr Tagen;
(c) für 21 oder mehr Tage, gegebenenfalls gefolgt von einer Behandlungspause ohne Dosisgabe von 7 oder mehr Tagen;
(d) für 14 Tage, gegebenenfalls gefolgt von einer Behandlungspause ohne Dosisgabe von 14 Tagen;
(e) für 21 oder mehr Tage, gefolgt von einer Behandlungspause ohne Dosisgabe von 7 oder mehr Tagen oder
(f) für 14 Tage, gefolgt von einer Behandlungspause ohne Dosisgabe für 14 Tage.

9. Pharmazeutische Zusammensetzung und mindestens ein zusätzliches therapeutisches Mittel zur Verwendung nach Anspruch 8, wobei mindestens einer der Schritte (a), (b), (c), (d), (e) oder (f) wiederholt wird.

10. Pharmazeutische Zusammensetzung und mindestens ein zusätzliches therapeutisches Mittel zur Verwendung nach einem der Ansprüche 1-4, wobei das 5-Azacytidin verabreicht wird:
(a) in einer Dosis von etwa 300 mg täglich für 14 Tage, gefolgt von einer Behandlungspause ohne Dosisgabe von 14 Tagen;
(b) in einer Dosis von etwa 200 mg täglich für 14 Tage, gefolgt von einer Behandlungspause ohne Dosisgabe von 14 Tagen;
(c) in einer Dosis von etwa 300 mg täglich für 21 Tage, gefolgt von einer Behandlungspause ohne Dosisgabe von 7 Tagen;
(d) in einer Dosis von etwa 200 mg täglich, gefolgt von einer Behandlungspause ohne Dosisgabe von 7 Tagen;
(e) in einer Dosis von etwa 300 mg täglich für 14 Tage, gefolgt von einer Behandlungspause ohne Dosisgabe von 14 Tagen;
(f) in einer Dosis von 200 mg täglich für 14 Tage, gefolgt von einer Behandlungspause ohne Dosisgabe von 14 Tagen;
(g) in einer Dosis von 300 mg täglich für 21 Tage, gefolgt von einer Behandlungspause ohne Dosisgabe von 7 Tagen; oder
(h) in einer Dosis von 200 mg täglich, gefolgt von einer Behandlungspause ohne Dosisgabe von 7 Tagen.

11. Pharmazeutische Zusammensetzung und mindestens ein zusätzliches therapeutisches Mittel zur Verwendung nach Anspruch 10, wobei mindestens einer der Schritte (a), (b), (c), (d), (e), (f), (g) oder (h) wiederholt wird.

12. Pharmazeutische Zusammensetzung und mindestens ein zusätzliches therapeutisches Mittel zur Verwendung nach einem der Ansprüche 1-6, wobei das 5-Azacytidin verabreicht wird:
(a) unter Verwendung eines Behandlungszyklus, der Verabreichung von 5-Azacytidin pro Tag für 7 Tage in einem 28-Tage-Zyklus umfasst;
(b) unter Verwendung eines Behandlungszyklus, der Verabreichung von 5-Azacytidin pro Tag für 14 Tage in einem 28-Tage-Zyklus umfasst; oder
(c) unter Verwendung eines Behandlungszyklus, der Verabreichung von 5-Azacytidin pro Tag für 21 Tage in einem 28-Tage-Zyklus umfasst.

13. Pharmazeutische Zusammensetzung und mindestens ein zusätzliches therapeutisches Mittel zur Verwendung nach Anspruch 1, wobei das 5-Azacytidin und das mindestens eine zusätzliche therapeutische Mittel eine synergistische Wirkung zur Behandlung der akuten myeloischen Leukämie oder des myelodysplastischen Syndroms bereitstellen.

14. Pharmazeutische Zusammensetzung und mindestens ein zusätzliches therapeutisches Mittel zur Verwendung nach Anspruch 1, wobei:
(a) die akute myeloische Leukämie als durch eine FLT3-ITD-Mutation verursacht charakterisiert wird; und/oder
(b) die akute myeloische Leukämie resistent gegen Behandlung mit dem mindestens einen zusätzlichen therapeutischen Mittel allein ist; und/oder
(c) das 5-Azacytidin vor dem mindestens einen zusätzlichen therapeutischen Mittel verabreicht wird; und/oder
(d) die akute myeloische Leukämie auf Behandlung mit einem FMS-ähnlichen Tyrosinkinase-3-Inhibitor (FLT3-Inhibitor) anspricht; und/oder
(e) die akute myeloische Leukämie als eine Überexpression von MCL-1 aufweisend charakterisiert wird; und/oder
(f) das 5-Azacytidin die Krebszellen Priming auf Apoptose unterzieht, die durch das mindestens eine zusätzliche therapeutische Mittel vermittelt wird, indem die Expression von MCL-1 herunterreguliert wird; und/oder
(g) das 5-Azacytidin und mindestens ein zusätzliches therapeutisches Mittel den MCL-1-Abbau steigern; und/oder
(h) das 5-Azacytidin den Zellmetabolismus ändert; und/oder
(i) das 5-Azacytidin einen Zellzyklusarrest bewirkt; und/oder
(j) das 5-Azacytidin oxidative Phosphorylierung unterdrückt; und/oder
(k) das 5-Azacytidin Expression des aktivierenden Transkriptionsfaktors 3 (ATF3) erhöht; und/oder
(l) das 5-Azacytidin Expression von Stearoyl-CoA-Desaturase (SCD) verringert; und/oder
(m) das 5-Azacytidin und mindestens ein zusätzliches therapeutisches Mittel das mediane Überleben im Vergleich zu intravenös oder subkutan verabreichtem 5-Azacytidin und mindestens einem zusätzlichen therapeutischen Mittel erhöhen; und/oder
(n) das 5-Azacytidin und mindestens ein zusätzliches therapeutisches Mittel das mediane Überleben im Vergleich zu intravenös oder subkutan verabreichtem 5-Azacytidin und mindestens einem zusätzlichen therapeutischen Mittel um etwa 10 %, etwa 15 %, etwa 20 %, etwa 25 %, etwa 30 %, etwa 35 %, etwa 40 %, etwa 45 %, etwa 50 %, etwa 55 %, etwa 60 %, etwa 65 %, etwa 70 %, etwa 75 %, etwa 80 %, etwa 85 %, etwa 90 %, etwa 95 %, oder etwa 100 % erhöhen.

15. Pharmazeutische Zusammensetzung und mindestens ein zusätzliches therapeutisches Mittel zur Verwendung nach einem der Ansprüche 1-6, wobei:
(a) das 5-Azacytidin dem Menschen täglich für 1, 2, 3, 4, 5, 6, 7, 8, 9, 10, 11, 12, 13 oder 14 Tage verabreicht wird;
(b) das mindestens eine zusätzliche therapeutische Mittel dem Menschen für einen oder mehrere Tage verabreicht wird; und
(c) gegebenenfalls die Schritte (a) und (b) wiederholt werden.

16. Pharmazeutische Zusammensetzung und mindestens ein zusätzliches therapeutisches Mittel zur Verwendung nach einem der Ansprüche 1-6, wobei:
(a) das 5-Azacytidin dem Menschen täglich für 1, 2, 3, 4, 5, 6, 7, 8, 9, 10, 11, 12, 13 oder 14 Tage eines 28-Tage-Zyklus verabreicht wird;
(b) das mindestens eine therapeutische Mittel dem Menschen gleichzeitig täglich für 1, 2, 3, 4, 5, 6, 7, 8, 9, 10, 11, 12, 13, 14, 15, 16, 17, 18, 19, 20, 21, 22, 23, 24, 25, 26, 27 oder 28 Tage eines 28-Tage-Zyklus verabreicht wird; und
(c) gegebenenfalls die Schritte (a) und (b) wiederholt werden.

17. Pharmazeutische Zusammensetzung und mindestens ein zusätzliches therapeutisches Mittel zur Verwendung nach einem der Ansprüche 1-16, wobei das Verfahren ein Verfahren zur Behandlung von akuter myeloischer Leukämie bei einem Menschen ist.

18. Pharmazeutische Zusammensetzung und mindestens ein zusätzliches therapeutisches Mittel zur Verwendung nach einem der Ansprüche 1-16, wobei das Verfahren ein Verfahren zur Behandlung des myelodysplastischen Syndroms bei einem Menschen ist, wobei das myelodysplastische Syndrom gegebenenfalls ein myelodysplastisches Syndrom mit hohem und sehr hohem Risiko ist, wie durch das überarbeitete International Prognostic Scoring System (IPSS-R) definiert.

## Revendications

1. (i) Une composition pharmaceutique comprenant de la 5-azacytidine, et (ii) au moins un agent thérapeutique supplémentaire, pour une utilisation dans un procédé de traitement de la leucémie myéloïde aiguë et/ou du syndrome myélodysplasique chez un humain, le procédé comprenant l'administration à l'humain de (i) la composition pharmaceutique comprenant de la 5-azacytidine par voie orale, et (ii) l'au moins un agent thérapeutique supplémentaire, l'au moins un agent thérapeutique supplémentaire étant le vénétoclax.

2. La composition pharmaceutique et au moins un agent thérapeutique supplémentaire pour une utilisation selon la revendication 1, dans laquelle :
(a) la 5-azacytidine et l'au moins un agent thérapeutique supplémentaire sont administrés simultanément ; ou
(b) la 5-azacytidine et l'au moins un agent thérapeutique supplémentaire sont administrés successivement, la 5-azacytidine étant administrée en premier.

3. La composition pharmaceutique et au moins un agent thérapeutique supplémentaire pour une utilisation selon la revendication 1, dans laquelle :
(a) la 5-azacytidine et l'au moins un agent thérapeutique supplémentaire sont coformulés sous la forme d'une seule forme posologique unitaire ; ou
(b) la 5-azacytidine et l'au moins un agent thérapeutique supplémentaire sont formulés sous forme de formes posologiques distinctes.

4. La composition pharmaceutique et au moins un agent thérapeutique supplémentaire pour une utilisation selon l'une quelconque des revendications 1 à 3, dans laquelle :
(a) l'au moins un agent thérapeutique supplémentaire est administré par voie parentérale ; ou
(b) l'au moins un agent thérapeutique supplémentaire est administré par voie orale.

5. La composition pharmaceutique et au moins un agent thérapeutique supplémentaire pour une utilisation selon l'une quelconque des revendications 1 à 4, dans laquelle la 5-azacytidine est administrée :
(a) à une dose d'environ 50 mg, environ 60 mg, environ 70 mg, environ 80 mg, environ 90 mg, environ 100 mg, environ 110 mg, environ 120 mg, environ 130 mg, environ 140 mg, environ 150 mg, environ 160 mg, environ 170 mg, environ 180 mg, environ 190 mg, environ 200 mg, environ 210 mg, environ 220 mg, environ 230 mg, environ 240 mg, environ 250 mg, environ 260 mg, environ 270 mg, environ 280 mg, environ 290 mg, environ 300 mg, environ 310 mg, environ 320 mg, environ 330 mg, environ 340 mg, environ 350 mg, environ 360 mg, environ 370 mg, environ 380 mg, environ 390 mg, environ 400 mg, environ 410 mg, environ 420 mg, environ 430 mg, environ 440 mg, environ 450 mg, environ 460 mg, environ 470 mg, environ 480 mg, environ 490 mg, environ 500 mg, environ 510 mg, environ 520 mg, environ 530 mg, environ 540 mg, environ 550 mg, environ 560 mg, environ 570 mg, environ 580 mg, environ 590 mg ou environ 600 mg par voie orale ; et/ou
(b) à une dose d'environ 200 mg ; et/ou
(c) à une dose d'environ 300 mg ; et/ou
(d) pendant les sept, quatorze ou vingt-et-un premiers jours d'un cycle de 28 jours ; et/ou
(e) à l'être humain une ou deux fois par jour ; et/ou
(f) sous la forme d'une capsule ou d'un comprimé.

6. La composition pharmaceutique et au moins un agent thérapeutique supplémentaire pour une utilisation selon la revendication 5, dans laquelle la 5-azacytidine est administrée sous la forme :
(a) d'un comprimé non gastro-résistant, ou
(b) d'une composition orale à libération immédiate.

7. La composition pharmaceutique et au moins un agent thérapeutique supplémentaire pour une utilisation selon l'une quelconque des revendications 1 à 6, dans laquelle la 5-azacytidine est administrée :
(a) à une dose d'environ 200 mg par jour pendant 14 jours sur un cycle de 28 jours ;
(b) à une dose d'environ 300 mg par jour pendant 14 jours sur un cycle de 28 jours ;
(c) à une dose d'environ 200 mg par jour pendant 21 jours sur un cycle de 28 jours ;
(d) à une dose d'environ 300 mg par jour pendant 21 jours sur un cycle de 28 jours ;
(e) à une dose d'environ 200 mg par jour pendant 7 jours sur un cycle de 28 jours ; ou
(f) à une dose d'environ 300 mg par jour pendant 7 jours sur un cycle de 28 jours.

8. La composition pharmaceutique et au moins un agent thérapeutique supplémentaire pour une utilisation selon l'une quelconque des revendications 1 à 6, dans laquelle la 5-azacytidine est administrée :
(a) quotidiennement pendant 1, 2, 3, 4, 5, 6, 7, 8, 9, 10, 11, 12, 13, 14 ou plus de 14 jours, éventuellement suivis d'une parenthèse thérapeutique de 1, 2, 3, 4, 5, 6, 7, 8, 9, 10, 11, 12, 13, 14 ou plus de 14 jours ;
(b) quotidiennement pendant au moins 14 jours, éventuellement suivis d'une parenthèse thérapeutique d'au moins 7 jours ;
(c) pendant au moins 21 jours, éventuellement suivis d'une parenthèse thérapeutique d'au moins 7 jours ;
(d) pendant 14 jours, éventuellement suivis d'une parenthèse thérapeutique de 14 jours ;
(e) pendant au moins 21 jours, suivis d'une parenthèse thérapeutique d'au moins 7 jours ; ou
(f) pendant 14 jours, suivis d'une parenthèse thérapeutique de 14 jours.

9. La composition pharmaceutique et au moins un agent thérapeutique supplémentaire pour une utilisation selon la revendication 8, dans laquelle au moins une des étapes (a), (b), (c), (d), (e) et (f) est répétée.

10. La composition pharmaceutique et au moins un agent thérapeutique supplémentaire pour une utilisation selon l'une quelconque des revendications 1 à 4, dans laquelle la 5-azacytidine est administrée :
(a) à une dose d'environ 300 mg quotidiennement pendant 14 jours, suivis d'une parenthèse thérapeutique de 14 jours ;
(b) à une dose d'environ 200 mg quotidiennement pendant 14 jours, suivis d'une parenthèse thérapeutique de 14 jours ;
(c) à une dose d'environ 300 mg quotidiennement pendant 21 jours, suivis d'une parenthèse thérapeutique de 7 jours ;
(d) à une dose d'environ 200 mg quotidiennement, suivie d'une parenthèse thérapeutique de 7 jours ;
(e) à une dose de 300 mg quotidiennement pendant 14 jours, suivis d'une parenthèse thérapeutique de 14 jours ;
(f) à une dose de 200 mg quotidiennement pendant 14 jours, suivis d'une parenthèse thérapeutique de 14 jours ;
(g) à une dose de 300 mg quotidiennement pendant 21 jours, suivis d'une parenthèse thérapeutique de 7 jours ; ou
(h) à une dose de 200 mg quotidiennement, suivie d'une parenthèse thérapeutique de 7 jours.

11. La composition pharmaceutique et au moins un agent thérapeutique supplémentaire pour une utilisation selon la revendication 10, dans laquelle au moins une des étapes (a), (b), (c), (d), (e), (f), (g) et (h) est répétée.

12. La composition pharmaceutique et au moins un agent thérapeutique supplémentaire pour une utilisation selon l'une quelconque des revendications 1 à 6, dans laquelle la 5-azacytidine est administrée :
(a) au moyen d'un cycle de traitement comprenant l'administration de 5-azacytidine tous les jours pendant 7 jours sur un cycle de 28 jours ;
(b) au moyen d'un cycle de traitement comprenant l'administration de 5-azacytidine tous les jours pendant 14 jours sur un cycle de 28 jours ; ou
(c) au moyen d'un cycle de traitement comprenant l'administration de 5-azacytidine tous les jours pendant 21 jours sur un cycle de 28 jours.

13. La composition pharmaceutique et au moins un agent thérapeutique supplémentaire pour une utilisation selon la revendication 1, dans laquelle la 5-azacytidine et l'au moins un agent thérapeutique supplémentaire apportent un effet synergique pour traiter la leucémie myéloïde aiguë ou le syndrome myélodysplasique.

14. La composition pharmaceutique et au moins un agent thérapeutique supplémentaire pour une utilisation selon la revendication 1, dans laquelle :
(a) la leucémie myéloïde aiguë se caractérise comme étant induite par une mutation FLT3-ITD ; et/ou
(b) la leucémie myéloïde aiguë est résistante à un traitement avec l'au moins un agent thérapeutique supplémentaire seul ; et/ou
(c) la 5-azacytidine est administrée avant l'au moins un agent thérapeutique supplémentaire ; et/ou
(d) la leucémie myéloïde aiguë est sensible à un traitement avec une tyrosine kinase 3 de type FMS (inhibiteur de la FLT3) ; et/ou
(e) la leucémie myéloïde aiguë se caractérise comme présentant une surexpression de MCL-1 ; et/ou
(f) la 5-azacytidine déclenche l'apoptose des cellules cancéreuses médiée par l'au moins un agent thérapeutique supplémentaire en régulant à la baisse l'expression de la MCL-1 ; et/ou
(g) la 5-azacytidine et au moins un agent thérapeutique supplémentaire augmentent la dégradation de la MCL-1 ; et/ou
(h) la 5-azacytidine modifie le métabolisme cellulaire ; et/ou
(i) la 5-azacytidine provoque un arrêt du cycle cellulaire ; et/ou
(j) la 5-azacytidine supprime la phosphorylation oxydative ; et/ou
(k) la 5-azacytidine augmente l'expression du facteur de transcription activateur 3 (ATF3) ; et/ou
(l) la 5-azacytidine diminue l'expression de la stéaroyl-CoA désaturase (SCD) ; et/ou
(m) la 5-azacytidine et au moins un agent thérapeutique supplémentaire augmentent la survie médiane par rapport à la 5-azacytidine administrée par voie intraveineuse ou sous-cutanée et au moins un agent thérapeutique supplémentaire ; et/ou
(n) la 5-azacytidine et au moins un agent thérapeutique supplémentaire augmentent la survie médiane par rapport à la 5-azacytidine administrée par voie intraveineuse ou sous-cutanée et au moins un agent thérapeutique supplémentaire d'environ 10 %, environ 15 %, environ 20 %, environ 25 %, environ 30 %, environ 35 %, environ 40 %, environ 45 %, environ 50 %, environ 55 %, environ 60 %, environ 65 %, environ 70 %, environ 75 %, environ 80 %, environ 85 %, environ 90 %, environ 95 % ou environ 100 %.

15. La composition pharmaceutique et au moins un agent thérapeutique supplémentaire pour une utilisation selon l'une quelconque des revendications 1 à 6, dans laquelle :
(a) la 5-azacytidine est administrée quotidiennement à l'humain pendant 1, 2, 3, 4, 5, 6, 7, 8, 9, 10, 11, 12, 13 ou 14 jours ;
(b) l'au moins un agent thérapeutique supplémentaire est administré à l'humain pendant un ou plusieurs jours ; et
(c) éventuellement, les étapes (a) et (b) sont répétées.

16. La composition pharmaceutique et au moins un agent thérapeutique supplémentaire pour une utilisation selon l'une quelconque des revendications 1 à 6, dans laquelle :
(a) la 5-azacytidine est administrée quotidiennement à l'humain pendant 1, 2, 3, 4, 5, 6, 7, 8, 9, 10, 11, 12, 13 ou 14 jours d'un cycle de 28 jours ;
(b) l'au moins un agent thérapeutique est administré simultanément et quotidiennement à l'humain pendant 1, 2, 3, 4, 5, 6, 7, 8, 9, 10, 11, 12, 13, 14, 15, 16, 17, 18, 19, 20, 21, 22, 23, 24, 25, 26, 27 ou 28 jours d'un cycle de 28 jours ; et
(c) éventuellement, les étapes (a) et (b) sont répétées.

17. La composition pharmaceutique et au moins un agent thérapeutique supplémentaire pour une utilisation selon l'une quelconque des revendications 1 à 16, ledit procédé étant un procédé de traitement de la leucémie myéloïde aiguë chez un humain.

18. La composition pharmaceutique et au moins un agent thérapeutique supplémentaire pour une utilisation selon l'une quelconque des revendications 1 à 16, ledit procédé étant un procédé de traitement du syndrome myélodysplasique chez un humain, le syndrome myélodysplasique étant éventuellement un syndrome myélodysplasique à haut et très haut risque tel que défini par le système international de notation pronostique révisé (IPSS-R).
